# EUROPÄISCHE PATENTSCHRIFT

(11) **EP 1 838 315 B1**
(45) Veröffentlichungstag und Bekanntmachung des Hinweises auf die Patenterteilung: **12.03.2014**
(21) Anmeldenummer: 05823573.0
(22) Anmeldetag: 30.12.2005
(51) Int. Cl.: A61K 31/47, A61P 9/12, C07D 217/14

(54) **SUBSTITUIERTE 4-PHENYLTETRAHYDROISOCHINOLINE, VERFAHREN ZU IHRER HERSTELLUNG, IHRE VERWENDUNG ALS MEDIKAMENT, SOWIE SIE ENTHALTENDES MEDIKAMENT**
SUBSTITUTED 4-PHENYLTETRAHYDROISOQUINOLINES, METHODS FOR PRODUCING THEM, THEIR USE AS DRUG, AND DRUG CONTAINING THEM
4-PHENYLTÉTRAHYDROISOCHINOLINES SUBSTITUÉES, PROCÉDÉ POUR LES PRÉPARER, LEUR UTILISATION EN TANT QUE MÉDICAMENT ET MÉDICAMENT LES CONTENANT

(30) Priorität: 12.01.2005 DE 102005001411
(43) Veröffentlichungstag der Anmeldung: 03.10.2007
(73) Patentinhaber: SANOFI, 75008 Paris (FR)
(72) Erfinder: HEINELT, Uwe, 65926 Frankfurt am Main (DE); LANG, Hans-Jochen, 65719 Hofheim (DE); WIRTH, Klaus, 65830 Kriftel (DE); LICHER, Thomas, 65812 Bad Soden (DE); HOFMEISTER, Armin, 55278 Dexheim (DE)
(74) Vertreter: Michalski Hüttermann & Partner Patentanwälte
(86) Internationale Anmeldenummer: PCT/EP2005/014127
(87) Internationale Veröffentlichungsnummer: WO 2006/074813

(56) Entgegenhaltungen:
- WO-A-03/048129
- WO-A-2004/085404

## Beschreibung

Die Erfindung betrifft substituierte 4-Phenyltetrahydroisochinoline. Medikamente, die Verbindungen dieses Typs enthalten, sind nützlich bei der Prävention oder Behandlung diverser Erkrankungen. So lassen sich die Verbindungen unter anderem bei Nierenerkrankungen wie akutem oder chronischem Nierenversagen, bei Störungen der Gallenfunktion und bei Atemstörungen wie Schnarchen oder Schlafapnoen einsetzen.

Die Erfindung betrifft Verbindungen der Formel I 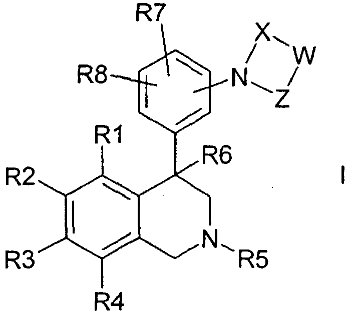 worin bedeuten:
- R1, R2, R3 und R4: unabhängig voneinander Wasserstoff, F, Cl, Br, I, CN, NO₂ oder R11-(CₘH₂ₘ)-Aₙ-;
m Null, 1, 2, 3 oder 4;
n Null oder 1;
R11 Wasserstoff, Methyl oder CₚF₂ₚ₊₁;
A Sauerstoff, NH, N(CH₃) oder S(O)_{q};
p 1, 2 oder 3;
q Null, 1 oder 2;
- R5: Wasserstoff, Alkyl mit 1, 2, 3, 4, 5 oder 6 C-Atomen oder Cycloalkyl mit 3, 4, 5 oder 6 C Atomen;
- R6: Wasserstoff, OH, F, CF₃, Alkyl mit 1, 2, 3 oder 4 C-Atomen oder Cycloalkyl mit 3, 4, 5 oder 6 C Atomen;
- R7 und R8: unabhängig voneinander Wasserstoff. F, Cl, Br, CN, CO₂R12, NR13R14 oder R16-(CₘₘH₂ₘₘ)-Bₙₙ-;
R12 Wasserstoff, Alkyl mit 1, 2, 3 oder 4 C-Atomen oder Cycloalkyl mit 3, 4, 5 oder 6 C-Atomen;
R13 und R14 unabhängig voneinander Wasserstoff, Alkyl mit 1, 2, 3 oder 4 C-Atomen oder Cycloalkyl mit 3, 4, 5 oder 6 C-Atomen;
oder
R13 und R14 bilden mit dem Stickstoffatom, an das sie gebunden sind, einen 4, 5, 6 oder 7 gliedrigen Ring, in dem eine CH₂-Gruppe durch NR15, S oder Sauerstoff ersetzt sein kann;
R15 Wasserstoff, Alkyl mit 1, 2, 3 oder 4 C-Atomen oder Cycloalkyl mit 3, 4, 5 oder 6 C-Atomen;
mm Null, 1, 2, 3 oder 4;
nn Null oder 1;
R16 Wasserstoff, Methyl oder CₚₚF₂ₚₚ₊₁;
B Sauerstoff oder S(O)_{qq};
pp 1, 2 oder 3;
qq Null, 1 oder 2;
- W: CᵣH₂ᵣ oder CₛH_{2s-2;}
wobei eine oder mehrere CH₂-Gruppen in CᵣH₂ᵣ und CₛH₂ₛ₋₂ durch NR17, Sauerstoff oder S ersetzt sein können;
R17 Wasserstoff, Alkyl mit 1, 2, 3 oder 4 C-Atomen oder Cycloalkyl mit 3, 4, 5 oder 6 C-Atomen;
r 1, 2, 3, 4, 5, 6, 7 oder 8;
s 2, 3, 4, 5, 6, 7 oder 8;
- X: -C(O)- oder -S(O)₂-;
- Z: -C(O)- oder eine Bindung;
sowie deren pharmazeutisch verträgliche Salze und Trifluoracetate.

In einer Ausführungsform sind Verbindungen der Formel I bevorzugt, worin bedeuten
- R1, R2, R3 und R4: unabhängig voneinander Wasserstoff, F, Cl, Br, CN oder R11-(CₘH₂ₘ)-Aₙ-;
m Null oder 1;
n Null oder 1;
R11 Wasserstoff, Methyl oder CₚF₂ₚ₊₁;
A Sauerstoff, NCH₃ oder S(O)_{q};
p 1 oder 2;
q Null, 1 oder 2;
- R5: Wasserstoff, Methyl, Ethyl oder Cyclopropyl;
- R6: Wasserstoff oder Methyl;
- R7 und R8: unabhängig voneinander Wasserstoff, F, Cl, CN, CO₂R12, NR13R14 oder R16-(CₘₘH₂ₘₘ)-Bₙₙ-;
R12 Wasserstoff, Methyl oder Ethyl;
R13 und R14 unabhängig voneinander Wasserstoff, Alkyl mit 1, 2, 3 oder 4 C-Atomen oder Cycloalkyl mit 3, 4, 5 oder 6 C-Atomen;
oder
R13 und R14 bilden mit dem Stickstoffatom, an das sie gebunden sind, einen 5, 6 oder 7 gliedrigen Ring, in dem eine CH₂-Gruppe durch NR15, S oder Sauerstoff ersetzt sein kann;
R15 Wasserstoff, Alkyl mit 1, 2, 3 oder 4 C-Atomen oder Cycloalkyl mit 3, 4, 5 oder 6 C-Atomen;
mm Null, 1 oder 2;
nn Null oder 1;
R16 Wasserstoff, Methyl oder CₚₚF₂ₚₚ₊₁;
B Sauerstoff oder S(O)qq;
pp 1 oder 2;
qq Null, 1 oder 2;
- W: CᵣH₂ᵣ oder CₛH_{2s-2;}
wobei eine oder mehrere CH₂-Gruppen in CᵣH₂ᵣ und CₛH₂ₛ₋₂ durch NR17, Sauerstoff oder S ersetzt sein können;
R17 Wasserstoff, Alkyl mit 1, 2, 3 oder 4 C-Atomen oder Cycloalkyl mit 3, 4, 5 oder 6 C-Atomen;
r 2, 3, 4, 5, 6, 7 oder 8;
s 2, 3, 4, 5, 6, 7 oder 8;
- X: -C(O)- oder -S(O)₂-;
- Z: -C(O)-;
sowie deren pharmazeutisch verträglichen Salze und Trifluoracetate.

Besonders bevorzugt sind Verbindungen der Formel I, worin bedeuten
- R1 und R3: Wasserstoff;
- R2 und R4: unabhängig voneinander Wasserstoff, F, Cl, Br, NH₂, NHCH₃ oder N(CH₃)₂;
- R5: Wasserstoff, Methyl, Ethyl oder Cyclopropyl;
- R6: Wasserstoff oder Methyl;
- R7 und R8: Wasserstoff;
- W: CᵣH₂ᵣ oder CₛH₂ₛ₋₂;
wobei eine oder mehrere CH₂-Gruppen in CᵣH₂ᵣ und CₛH₂ₛ₋₂ durch NR17, Sauerstoff oder S ersetzt sein können;
R17 Wasserstoff oder Methyl;
r 2, 3, 4, 5 oder 6;
s 2, 3, 4, 5 oder 6;
- X: -C(O)- oder -S(O)₂-;
- Z: -C(O)-;
sowie deren pharmazeutisch verträglichen Salze und Trifluoracetate.

In einer weiteren Ausführungsform sind Verbindungen der Formel I bevorzugt, worin bedeuten
- R1 und R3: Wasserstoff;
- R2 und R4: unabhängig voneinander Wasserstoff, F, Cl, NH₂, NHCH₃ oder N(CH₃)₂;
- R5: Wasserstoff, Methyl, Ethyl oder Cyclopropyl;
- R6: Wasserstoff oder Methyl;
- R7 und R8: Wasserstoff;
- W: CᵣH₂ᵣ oder CₛH₂ₛ₋₂;
wobei eine oder mehrere CH₂-Gruppen in CᵣH₂ᵣ und CₛH₂ₛ₋₂ durch NR17, Sauerstoff oder S ersetzt sein können;
R17 Wasserstoff oder Methyl;
r 2, 3, 4, 5 oder 6;
s 2, 3, 4, 5 oder 6;
- X: -C(O)- oder -S(O)₂-;
- Z: -C(O)-;
sowie deren pharmazeutisch verträglichen Salze und Trifluoracetate.

Ganz besonders bevorzugt sind Verbindungen der Formel I ausgewählt aus der Gruppe:
1-[2-(8-Brom-6-chlor-2-methyl-1,2,3,4-tetrahydro-isoquinolin-4-yl)-phenyl]-pyrrolidin-2,5-dion,
3-[2-((R)-6,8-Dichlor-2-cyclopropyl-1,2,3,4-tetrahydro-isochinolin-4-yl)-phenyl]-1-methyl-imidazolidin-2,4-dion,
3-[2-((R)-6,8-Dichlor-2-methyl-1,2,3,4-tetrahydro-isochinolin-4-yl)-phenyl]-1-methyl-imidazolidin-2,4-dion
3-[2-((R)-6,8-Dichlor-2-cyclopropyl-1,2,3,4-tetrahydro-isochinolin-4-yl)-phenyl]-imidazolidin-2,4-dion
(S)-1-[2-((R)-6,8-Dichlor-2-cyclopropyl-1,2,3,4-tetrahydro-isochinolin-4-yl)-phenyl]-3-hydroxy-pyrrolidin-2,5-dion,
(R)-1-[2-((R)-6,8-Dichlor-2-cyclopropyl-1,2,3,4-tetrahydro-isochinolin-4-yl)-phenyl]-3-hydroxy-pyrrolidin-2,5-dion,
(R)-1-[2-((R)-6,8-Dichlor-2-methyl-1,2,3,4-tetrahydro-isochinolin-4-yl)-phenyl]-3-hydroxy-pyrrolidin-2,5-dion,
(S)-1-[2-((R)-6,8-Dichlor-2-methyl-1,2,3,4-tetrahydro-isochinolin-4-yl)-phenyl]-3-hydroxy-pyrrolidin-2,5-dion,
4-[2-((R)-6,8-Dichlor-2-methyl-1,2,3,4-tetrahydro-isochinolin-4-yl)-phenyl]-morpholin-3,5-dion,
3-[2-((R)-6,8-Dichlor-2-methyl-1,2,3,4-tetrahydro-isochinolin-4-yl)-phenyl]-oxazolidin-2,4-dion,
3-[2-((R)-6,8-Dichlor-2-methyl-1,2,3,4-tetrahydro-isochinolin-4-yl)-phenyl]-thiazolidin-2,4-dion,
1-[2-((R)-6,8-Dichlor-2-cyclopropyl-1,2,3,4-tetrahydro-isochinolin-4-yl)-phenyl]-pyrrolidin-2,5-dion,
(S)-1-[2-((R)-6,8-Dichlor-2-methyl-1,2,3,4-tetrahydro-isochinolin-4-yl)-phenyl]-3-methyl-piperidin-2,6-dion,
(R)-1-[2-((R)-6,8-Dichlor-2-methyl-1,2,3,4-tetrahydro-isochinolin-4-yl)-phenyl]-3-methyl-piperidin-2,6-dion,
1-[2-((R)-6,8-Dichlor-2-methyl-1,2,3,4-tetrahydro-isochinolin-4-yl)-phenyl]-3,5-dimethyl-piperidin-2,6-dion,
1-[2-((R)-6,8-Dichlor-2-methyl-1,2,3,4-tetrahydro-isochinolin-4-yl)-phenyl]-3-methyl-pyrrolidin-2,5-dion,
1-[2-((R)-6,8-Dichlor-2-methyl-1,2,3,4-tetrahydro-isochinolin-4-yl)-phenyl]-3,3-dimethyl-pyrrolidin-2,5-dion,
1-[2-(6,8-Dichlor-2-cyclopropyl-1,2,3,4-tetrahydro-isochinolin-4-yl)-phenyl]-pyrrolidin-2,5-dion,
1-[4-((S)-6,8-Dichlor-2-methyl-1,2,3,4-tetrahydro-isochinolin-4-yl)-phenyl]-piperidin-2,6-dion,
1-[4-((S)-6,8-Dichlor-2-methyl-1,2,3,4-tetrahydro-isochinolin-4-yl)-phenyl]-pyrrolidin-2,5-dion,
1-[4-(6,8-Dichlor-2-methyl-1,2,3,4-tetrahydro-isochinolin-4-yl)-phenyl]-pyrrolidin-2,5-dion,
1-[2-((R)-6,8-Dichlor-2-methyl-1,2,3,4-tetrahydro-isochinolin-4-yl)-phenyl]-piperidin-2,6-dion,
1-[2-(6,8-Dichlor-2-methyl-1,2,3,4-tetrahydro-isochinolin-4-yl)-phenyl]-piperidin-2,6-dion,
1-[3-((S)-6,8-Dichlor-2-methyl-1,2,3,4-tetrahydro-isochinolin-4-yl)-phenyl]-pyrrol-2,5-dion,
1-[2-(6,8-Dichlor-2-methyl-1,2,3,4-tetrahydro-isochinolin-4-yl)-phenyl]-pyrrolidin-2,5-dion,
1-[2-((R)-6,8-Dichlor-2-methyl-1,2,3,4-tetrahydro-isochinolin-4-yl)-phenyl]-pyrrolidin-2,5-dion,
3-[2-((R)-6,8-Dichlor-2-methyl-1,2,3,4-tetrahydro-isochinolin-4-yl)-phenyl]-imidazolidin-2,4-dion,
3-[2-(6,8-Dichlor-2-methyl-1,2,3,4-tetrahydro-isochinolin-4-yl)-phenyl]-imidazolidin-2,4-dion,
3-[3-((S)-6,8-Dichlor-2-methyl-1,2,3,4-tetrahydro-isochinolin-4-yl)-phenyl]-imidazolidin-2,4-dion,
3-[4-((S)-6,8-Dichlor-2-methyl-1,2,3,4-tetrahydro-isochinolin-4-yl)-phenyl]-imidazolidin-2,4-dion,
3-[4-(6,8-Dichlor-2-methyl-1,2,3,4-tetrahydro-isochinolin-4-yl)-phenyl]-imidazolidin-2,4-dion,
3-[2-((R)-6,8-Dichlor-2-cyclopropyl-1,2,3,4-tetrahydro-isochinolin-4-yl)-phenyl]-oxazolidin-2,4-dion,
3-[2-((R)-6,8-Dichlor-2-methyl-1,2,3,4-tetrahydro-isochinolin-4-yl)-phenyl]-5-methyl-imidazolidin-2,4-dion,
(3R,4S)-1-[2-((R)-6,8-Dichlor-2-methyl-1,2,3,4-tetrahydro-isochinolin-4-yl)-phenyl]-3,4-dimethyl-pyrrolidin-2,5-dion,
1-[4-((S)-6,8-Dichlor-2-methyl-1,2,3,4-tetrahydro-isochinolin-4-yl)-phenyl]-pyrrol-2,5-dion,
1-[3-((S)-6,8-Dichlor-2-methyl-1,2,3,4-tetrahydro-isochinolin-4-yl)-phenyl]-piperidin-2,6-dion,
1-[3-((S)-6,8-Dichlor-2-methyl-1,2,3,4-tetrahydro-isochinolin-4-yl)-phenyl]-pyrrolidin-2,5-dion,
1-[4-(6,8-Dichlor-2-methyl-1,2,3,4-tetrahydro-isochinolin-4-yl)-phenyl]-piperidin-2,6-dion,
3-[3-(6,8-Dichlor-2-methyl-1,2,3,4-tetrahydro-isochinolin-4-yl)-phenyl]-imidazolidin-2,4-dion,
1-[2-((R)-2-Methyl-1,2,3,4-tetrahydro-isochinolin-4-yl)-phenyl]-pyrrolidin-2,5-dion,
3-[2-((R)-6,8-Dichlor-2-methyl-1,2,3,4-tetrahydro-isoquinolin-4-yl)-phenyl]-5-isopropyl-imidazolidin-2,4-dion,
3-[2-((R)-6,8-Dichlor-2-methyl-1,2,3,4-tetrahydro-isoquinolin-4-yl)-phenyl]-5-isobutyl-imidazolidin-2,4-dion,
(R und S)-3-[2-((R)-6,8-Dichlor-2-methyl-1,2,3,4-tetrahydro-isochinolin-4-yl)-phenyl]-5-(2-methylsulfanyl-ethyl)-imidazolidin-2,4-dion,
3-[2-((R)-6,8-Dichlor-2-methyl-1,2,3,4-tetrahydro-isochinolin-4-yl)-phenyl]-5,5-dimethyl-imidazolidin-2,4-dion,
1-[2-((R)-6,8-Dichlor-1,2,3,4-tetrahydro-isochinolin-4-yl)-phenyl]-pyrrolidin-2,5-dion,
3-[2-((R)-6,8-Dichlor-2-cyclopropyl-1,2,3,4-tetrahydro-isochinolin-4-yl)-phenyl]-thiazolidin-2,4-dion,
1-[2-((S)-6,8-Dichlor-2-methyl-1,2,3,4-tetrahydro-isochinolin-4-yl)-phenyl]-pyrrolidin-2,5-dion,
1-[2-((S)-6,8-Dichlor-2-cyclopropyl-1,2,3,4-tetrahydro-isochinolin-4-yl)-phenyl]-pyrrolidin-2,5-dion,
1-[2-((R)-6,8-Dichlor-2-methyl-1,2,3,4-tetrahydro-isochinolin-4-yl)-phenyl]-3,3,4,4-tetramethyl-pyrrolidin-2,5-dion,
(S)-1-[2-((R)-6,8-Dichlor-2-cyclopropyl-1,2,3,4-tetrahydro-isochinolin-4-yl)-phenyl]-3-methyl-piperidin-2,6-dion,
1-[4-(6,8-Dichlor-2-methyl-1,2,3,4-tetrahydro-isochinolin-4-yl)-phenyl]-4,4-dimethyl-piperidin-2,6-dion
und 1-[2-(6,8-Dichlor-2-methyl-1,2,3,4-tetrahydro-isochinolin-4-yl)-phenyl]-pyrrol-2,5-dion,
   sowie deren pharmazeutisch verträgliche Salze und Trifluoracetate.

Speziell bevorzugt sind Verbindungen der Formel I ausgewählt aus der Gruppe: 1-[2-(8-Brom-6-chlor-2-methyl-1,2,3,4-tetrahydro-isoquinolin-4-yl)-phenyl]-pyrrolidin-2,5-dion,
3-[2-((R)-6,8-Dichlor-2-cyclopropyl-1,2,3,4-tetrahydro-isochinolin-4-yl)-phenyl]-1-methyl-imidazolidin-2,4-dion,
3-[2-((R)-6,8-Dichlor-2-methyl-1,2,3,4-tetrahydro-isochinolin-4-yl)-phenyl]-1-methyl-imidazolidin-2,4-dion
3-[2-((R)-6,8-Dichlor-2-cyclopropyl-1,2,3,4-tetrahydro-isochinolin-4-yl)-phenyl]-imidazolidin-2,4-dion
(S)-1-[2-((R)-6,8-Dichlor-2-cyclopropyl-1,2,3,4-tetrahydro-isochinolin-4-yl)-phenyl]-3-hydroxy-pyrrolidin-2,5-dion,
(R)-1-[2-((R)-6,8-Dichlor-2-cyclopropyl-1,2,3,4-tetrahydro-isochinolin-4-yl)-phenyl]-3-hydroxy-pyrrolidin-2,5-dion,
(R)-1-[2-((R)-6,8-Dichlor-2-methyl-1,2,3,4-tetrahydro-isochinolin-4-yl)-phenyl]-3-hydroxy-pyrrolidin-2,5-dion,
(S)-1-[2-((R)-6,8-Dichlor-2-methyl-1,2,3,4-tetrahydro-isochinolin-4-yl)-phenyl]-3-hydroxy-pyrrolidin-2,5-dion,
4-[2-((R)-6,8-Dichlor-2-methyl-1,2,3,4-tetrahydro-isochinolin-4-yl)-phenyl]-morpholin-3,5-dion,
3-[2-((R)-6,8-Dichlor-2-methyl-1,2,3,4-tetrahydro-isochinolin-4-yl)-phenyl]-oxazolidin-2,4-dion,
3-[2-((R)-6,8-Dichlor-2-methyl-1,2,3,4-tetrahydro-isochinolin-4-yl)-phenyl]-thiazolidin-2,4-dion,
1-[2-((R)-6,8-Dichlor-2-cyclopropyl-1,2,3,4-tetrahydro-isochinolin-4-yl)-phenyl]-pyrrolidin-2,5-dion,
(S)-1-[2-((R)-6,8-Dichlor-2-methyl-1,2,3,4-tetrahydro-isochinolin-4-yl)-phenyl]-3-methyl-piperidin-2,6-dion,
(R)-1-[2-((R)-6,8-Dichlor-2-methyl-1,2,3,4-tetrahydro-isochinolin-4-yl)-phenyl]-3-methyl-piperidin-2,6-dion,
1-[2-((R)-6,8-Dichlor-2-methyl-1,2,3,4-tetrahydro-isochinolin-4-yl)-phenyl]-3,5-dimethyl-piperidin-2,6-dion,
1-[2-((R)-6,8-Dichlor-2-methyl-1,2,3,4-tetrahydro-isochinolin-4-yl)-phenyl]-3-methyl-pyrrolidin-2,5-dion,
1-[2-((R)-6,8-Dichlor-2-methyl-1,2,3,4-tetrahydro-isochinolin-4-yl)-phenyl]-3,3-dimethyl-pyrrolidin-2,5-dion,
1-[2-(6,8-Dichlor-2-cyclopropyl-1,2,3,4-tetrahydro-isochinolin-4-yl)-phenyl]-pyrrolidin-2,5-dion,
1-[4-((S)-6,8-Dichlor-2-methyl-1,2,3,4-tetrahydro-isochinolin-4-yl)-phenyl]-piperidin-2,6-dion,
1-[4-((S)-6,8-Dichlor-2-methyl-1,2,3,4-tetrahydro-isochinolin-4-yl)-phenyl]-pyrrolidin-2,5-dion,
1-[4-(6,8-Dichlor-2-methyl-1,2,3,4-tetrahydro-isochinolin-4-yl)-phenyl]-pyrrolidin-2,5-dion,
1-[2-((R)-6,8-Dichlor-2-methyl-1,2,3,4-tetrahydro-isochinolin-4-yl)-phenyl]-piperidin-2,6-dion,
1-[2-(6,8-Dichlor-2-methyl-1,2,3,4-tetrahydro-isochinolin-4-yl)-phenyl]-piperidin-2,6-dion,
1-[3-((S)-6,8-Dichlor-2-methyl-1,2,3,4-tetrahydro-isochinolin-4-yl)-phenyl]-pyrrol-2,5-dion,
1-[2-(6,8-Dichlor-2-methyl-1,2,3,4-tetrahydro-isochinolin-4-yl)-phenyl]-pyrrolidin-2,5-dion,
1-[2-((R)-6,8-Dichlor-2-methyl-1,2,3,4-tetrahydro-isochinolin-4-yl)-phenyl]-pyrrolidin-2,5-dion,
3-[2-((R)-6,8-Dichlor-2-methyl-1,2,3,4-tetrahydro-isochinolin-4-yl)-phenyl]-imidazolidin-2,4-dion,
3-[2-(6,8-Dichlor-2-methyl-1,2,3,4-tetrahydro-isochinolin-4-yl)-phenyl]-imidazolidin-2,4-dion,
3-[3-((S)-6,8-Dichlor-2-methyl-1,2,3,4-tetrahydro-isochinolin-4-yl)-phenyl]-imidazolidin-2,4-dion,
3-[4-((S)-6,8-Dichlor-2-methyl-1,2,3,4-tetrahydro-isochinolin-4-yl)-phenyl]-imidazolidin-2,4-dion,
3-[4-(6,8-Dichlor-2-methyl-1,2,3,4-tetrahydro-isochinolin-4-yl)-phenyl]-imidazolidin-2,4-dion,
3-[2-((R)-6,8-Dichlor-2-cyclopropyl-1,2,3,4-tetrahydro-isochinolin-4-yl)-phenyl]-oxazolidin-2,4-dion,
3-[2-((R)-6,8-Dichlor-2-methyl-1,2,3,4-tetrahydro-isochinolin-4-yl)-phenyl]-5-methyl-imidazolidin-2,4-dion,
(3R,4S)-1-[2-((R)-6,8-Dichlor-2-methyl-1,2,3,4-tetrahydro-isochinolin-4-yl)-phenyl]-3,4-dimethyl-pyrrolidin-2,5-dion,
1-[4-((S)-6,8-Dichlor-2-methyl-1,2,3,4-tetrahydro-isochinolin-4-yl)-phenyl]-pyrrol-2,5-dion,
1-[3-((S)-6,8-Dichlor-2-methyl-1,2,3,4-tetrahydro-isochinolin-4-yl)-phenyl]-piperidin-2,6-dion,
1-[3-((S)-6,8-Dichlor-2-methyl-1,2,3,4-tetrahydro-isochinolin-4-yl)-phenyl]-pyrrolidin-2,5-dion,
1-[4-(6,8-Dichlor-2-methyl-1,2,3,4-tetrahydro-isochinolin-4-yl)-phenyl]-piperidin-2,6-dion
und
3-[3-(6,8-Dichlor-2-methyl-1,2,3,4-tetrahydro-isochinolin-4-yl)-phenyl]-imidazolidin-2,4-dion,
sowie deren pharmazeutisch verträgliche Salze und Trifluoracetate.

Insbesondere bevorzugt sind Verbindungen der Formel I ausgewählt aus der Gruppe: 1-[2-(8-Brom-6-chlor-2-methyl-1,2,3,4-tetrahydro-isoquinolin-4-yl)-phenyl]-pyrrolidin-2,5-dion,
3-[2-((R)-6,8-Dichlor-2-cyclopropyl-1,2,3,4-tetrahydro-isochinolin-4-yl)-phenyl]-1-methyl-imidazolidin-2,4-dion,
3-[2-((R)-6,8-Dichlor-2-methyl-1,2,3,4-tetrahydro-isochinolin-4-yl)-phenyl]-1-methyl-imidazolidin-2,4-dion
3-[2-((R)-6,8-Dichlor-2-cyclopropyl-1,2,3,4-tetrahydro-isochinolin-4-yl)-phenyl]-imidazolidin-2,4-dion
(S)-1-[2-((R)-6,8-Dichlor-2-cyclopropyl-1,2,3,4-tetrahydro-isochinolin-4-yl)-phenyl]-3-hydroxy-pyrrolidin-2,5-dion,
(R)-1-[2-((R)-6,8-Dichlor-2-cyclopropyl-1,2,3,4-tetrahydro-isochinolin-4-yl)-phenyl]-3-hydroxy-pyrrolidin-2,5-dion,
(R)-1-[2-((R)-6,8-Dichlor-2-methyl-1,2,3,4-tetrahydro-isochinolin-4-yl)-phenyl]-3-hydroxy-pyrrolidin-2,5-dion,
(S)-1-[2-((R)-6,8-Dichlor-2-methyl-1,2,3,4-tetrahydro-isochinolin-4-yl)-phenyl]-3-hydroxy-pyrrolidin-2,5-dion,
4-[2-((R)-6,8-Dichlor-2-methyl-1,2,3,4-tetrahydro-isochinolin-4-yl)-phenyl]-morpholin-3,5-dion,
3-[2-((R)-6,8-Dichlor-2-methyl-1,2,3,4-tetrahydro-isochinolin-4-yl)-phenyl]-oxazolidin-2,4-dion,
3-[2-((R)-6,8-Dichlor-2-methyl-1,2,3,4-tetrahydro-isochinolin-4-yl)-phenyl]-thiazolidin-2,4-dion,
1-[2-((R)-6,8-Dichlor-2-cyclopropyl-1,2,3,4-tetrahydro-isochinolin-4-yl)-phenyl]-pyrrolidin-2,5-dion,
(S)-1-[2-((R)-6,8-Dichlor-2-methyl-1,2,3,4-tetrahydro-isochinolin-4-yl)-phenyl]-3-methyl-piperidin-2,6-dion,
(R)-1-[2-((R)-6,8-Dichlor-2-methyl-1,2,3,4-tetrahydro-isochinolin-4-yl)-phenyl]-3-methyl-piperidin-2,6-dion,
1-[2-((R)-6,8-Dichlor-2-methyl-1,2,3,4-tetrahydro-isochinolin-4-yl)-phenyl]-3,5-dimethyl-piperidin-2,6-dion,
1-[2-((R)-6,8-Dichlor-2-methyl-1,2,3,4-tetrahydro-isochinolin-4-yl)-phenyl]-3-methyl-pyrrolidin-2,5-dion,
1-[2-((R)-6,8-Dichlor-2-methyl-1,2,3,4-tetrahydro-isochinolin-4-yl)-phenyl]-3,3-dimethyl-pyrrolidin-2,5-dion,
1-[2-(6,8-Dichlor-2-cyclopropyl-1,2,3,4-tetrahydro-isochinolin-4-yl)-phenyl]-pyrrolidin-2,5-dion,
1-[4-((S)-6,8-Dichlor-2-methyl-1,2,3,4-tetrahydro-isochinolin-4-yl)-phenyl]-piperidin-2,6-dion,
1-[4-((S)-6,8-Dichlor-2-methyl-1,2,3,4-tetrahydro-isochinolin-4-yl)-phenyl]-pyrrolidin-2,5-dion,
1-[4-(6,8-Dichlor-2-methyl-1,2,3,4-tetrahydro-isochinolin-4-yl)-phenyl]-pyrrolidin-2,5-dion,
1-[2-((R)-6,8-Dichlor-2-methyl-1,2,3,4-tetrahydro-isochinolin-4-yl)-phenyl]-piperidin-2,6-dion,
1-[2-(6,8-Dichlor-2-methyl-1,2,3,4-tetrahydro-isochinolin-4-yl)-phenyl]-piperidin-2,6-dion,
1-[3-((S)-6,8-Dichlor-2-methyl-1,2,3,4-tetrahydro-isochinolin-4-yl)-phenyl]-pyrrol-2,5-dion,
1-[2-(6,8-Dichlor-2-methyl-1,2,3,4-tetrahydro-isochinolin-4-yl)-phenyl]-pyrrolidin-2,5-dion,
1-[2-((R)-6,8-Dichlor-2-methyl-1,2,3,4-tetrahydro-isochinolin-4-yl)-phenyl]-pyrrolidin-2,5-dion,
3-[2-((R)-6,8-Dichlor-2-methyl-1,2,3,4-tetrahydro-isochinolin-4-yl)-phenyl]-imidazolidin-2,4-dion,
3-[2-(6,8-Dichlor-2-methyl-1,2,3,4-tetrahydro-isochinolin-4-yl)-phenyl]-imidazolidin-2,4-dion,
3-[3-((S)-6,8-Dichlor-2-methyl-1,2,3,4-tetrahydro-isochinolin-4-yl)-phenyl]-imidazolidin-2,4-dion,
3-[4-((S)-6,8-Dichlor-2-methyl-1,2,3,4-tetrahydro-isochinolin-4-yl)-phenyl]-imidazolidin-2,4-dion,
3-[4-(6,8-Dichlor-2-methyl-1,2,3,4-tetrahydro-isochinolin-4-yl)-phenyl]-imidazolidin-2,4-dion
und
3-[2-((R)-6,8-Dichlor-2-cyclopropyl-1,2,3,4-tetrahydro-isochinolin-4-yl)-phenyl]-oxazolidin-2,4-dion,
sowie deren pharmazeutisch verträgliche Salze und Trifluoracetate.

Eine weitere Ausführungsform umfasst Verbindungen der Formel I, worin bedeuten
- R1, R2, R3 und R4: unabhängig voneinander Wasserstoff, F, Cl, Br, CN oder R11-(CₘH₂ₘ)-Aₙ-;
m Null oder 1;
n Null oder 1;
R11 Wasserstoff, Methyl oder CₚF₂ₚ₊₁;
A Sauerstoff, NCH₃ oder S(O)q;
p 1 oder 2;
q Null, 1 oder 2;
- R5: Wasserstoff, Methyl, Ethyl oder Cyclopropyl;
- R6: Wasserstoff oder Methyl;
- R7 und R8: unabhängig voneinander Wasserstoff, F, Cl, CN, CO₂R12, NR13R14 oder R16-(CₘₘH₂ₘₘ)-Bₙₙ-;
R12 Wasserstoff, Methyl oder Ethyl;
R13, R14 unabhängig voneinander Wasserstoff, Alkyl mit 1, 2, 3 oder 4 C-Atomen oder Cycloalkyl mit 3, 4, 5 oder 6 C-Atomen;
oder
R13 und R14 bilden mit dem Stickstoffatom, an das sie gebunden sind, einen 5, 6 oder 7 gliedrigen Ring, in dem eine CH₂-Gruppe durch NR15, S oder Sauerstoff ersetzt sein kann;
R15 Wasserstoff, Alkyl mit 1, 2, 3 oder 4 C-Atomen oder Cycloalkyl mit 3, 4, 5 oder 6 C-Atomen;
mm Null, 1 oder 2;
nn Null oder 1;
R16 Wasserstoff, Methyl oder CₚₚF₂ₚₚ₊₁;
B Sauerstoff oder S(O)_{qq};
pp 1 oder 2;
qq Null, 1 oder 2;
- W: CᵣH₂ᵣ oder CₛH₂ₛ₋₂;
wobei eine oder mehrere CH₂-Gruppen in CᵣH₂ᵣ und CₛH₂ₛ₋₂ durch NR17, Sauerstoff oder S ersetzt sein können;
R17 Wasserstoff, Alkyl mit 1, 2, 3 oder 4 C-Atomen oder Cycloalkyl mit 3, 4, 5 oder 6 C-Atomen;
r 1, 2, 3, 4, 5, 6, 7 oder 8;
s 2, 3, 4, 5, 6, 7 oder 8;
- X: -C(O)- oder -S(O)₂-;
- Z: eine Bindung;
sowie deren pharmazeutisch verträglichen Salze und Trifluoracetate.

Bevorzugt sind Verbindungen der Formel I, worin bedeuten
- R1 und R3: Wasserstoff
- R2 und R4: unabhängig voneinander Wasserstoff, F, Cl, Br, NH₂, NHCH₃ oder N(CH₃)₂;
- R5: Wasserstoff, Methyl, Ethyl oder Cyclopropyl;
- R6: Wasserstoff oder Methyl;
- R7 und R8: Wasserstoff;
- W: CᵣH₂ᵣ oder CₛH₂ₛ₋₂;
wobei eine oder mehrere CH₂-Gruppen in CᵣH₂ᵣ und CₛH₂ₛ₋₂ durch NR17, Sauerstoff oder S ersetzt sein können;
R17 Wasserstoff oder Methyl
r 1, 2, 3, 4, 5 oder 6;
s 2, 3, 4, 5 oder 6;
- X: -C(O)- oder -S(O)₂-;
- Z: eine Bindung;
sowie deren pharmazeutisch verträglichen Salze und Trifluoracetate.

Besonders bevorzugt sind Verbindungen der Formel I, worin bedeuten
- R1 und R3: Wasserstoff
- R2 und R4: unabhängig voneinander Wasserstoff, F, Cl, NH₂, NHCH₃ oder N(CH₃)₂;
- R5: Wasserstoff, Methyl, Ethyl oder Cyclopropyl;
- R6: Wasserstoff oder Methyl;
- R7 und R8: Wasserstoff;
- W: CᵣH₂ᵣ oder CₛH₂ₛ₋₂; wobei eine oder mehrere CH₂-Gruppen in CᵣH₂ᵣ und CₛH₂ₛ₋₂ durch NR17, Sauerstoff oder S ersetzt sein können;
R17 Wasserstoff, Methyl
r 1, 2, 3, 4, 5 oder 6;
s 2, 3, 4, 5 oder 6;
- X: -C(O)- oder -S(O)₂-;
- Z: eine Bindung;
sowie deren pharmazeutisch verträglichen Salze und Trifluoracetate.

Ganz besonders bevorzugt sind Verbindungen der Formel I ausgewählt aus der Gruppe:
1-[2-(8-Brom-6-chlor-2-methyl-1,2,3,4-tetrahydro-isochinolin-4-yl)-phenyl]-pyrrolidin-2-on,
1-[2-((R)-6,8-Dichlor-2-methyl-1,2,3,4-tetrahydro-isochinolin-4-yl)-phenyl]-3-methyl-1,3-dihydro-imidazol-2-on,
(R)-6,8-Dichlor-4-[2-(1,1-dioxo-1-λ⁶-isothiazolidin-2-yl)-phenyl]-2-methyl-1,2,3,4-tetrahydro-isochinolin,
3-[2-((R)-6,8-Dichlor-2-methyl-1,2,3,4-tetrahydro-isochinolin-4-yl)-phenyl]-oxazolidin-2-on,
1-[2-((R)-6,8-Dichlor-2-methyl-1,2,3,4-tetrahydro-isochinolin-4-yl)-phenyl]-1,3-dihydroimidazol-2-on,
1-[2-((R)-6,8-Dichlor-2-methyl-1,2,3,4-tetrahydro-isochinolin-4-yl)-phenyl]-imidazolidin-2-on,
1-[2-((R)-6,8-Dichlor-2-methyl-1,2,3,4-tetrahydro-isoch inolin-4-yl)-phenyl]-pyrrolid in-2-on,
1-[2-((R)-6,8-Dichlor-2-methyl-1,2,3,4-tetrahydro-isochinolin-4-yl)-phenyl]-pyrrolidin-2-on,
1-[3-((S)-6,8-Dichlor-2-methyl-1,2,3,4-tetrahydro-isochinolin-4-yl)-phenyl]-piperidin-2-on,
1-((2-((R)-6,8-Dichlor-2-cyclopropyl-1,2,3,4-tetrahydro-isochinolin-4-yl)-phenyl]-pyrrolidin-2-on,
3-[2-((R)-6,8-Dichlor-2-cyclopropyl-1,2,3,4-tetrahydro-isochinolin-4-yl)-phenyl]-oxazolidin-2-on
   und
   6,8-Dichlor-4-[4-(1,1-dioxo-1-λ⁶-[1,2,5]thiadiazolidin-2-yl)-phenyl]-2-methyl-1,2,3,4-tetrahydro-isochinoline,
   sowie deren pharmazeutisch verträglichen Salze und Trifluoracetate.

Speziell bevorzugt sind Verbindungen der Formel I ausgewählt aus der Gruppe: 1-[2-(8-Brom-6-chlor-2-methyl-1,2,3,4-tetrahydro-isochinolin-4-yl)-phenyl]-pyrrolidin-2-on,
1-[2-((R)-6,8-Dichlor-2-methyl-1,2,3,4-tetrahydro-isochinolin-4-yl)-phenyl]-3-methyl-1,3-dihydro-imidazol-2-on,
(R)-6,8-Dichlor-4-[2-(1,1-dioxo-1-λ⁶-isothiazolidin-2-yl)-phenyl]-2-methyl-1,2,3,4-tetrahydro-isochinolin,
3-[2-((R)-6,8-Dichlor-2-methyl-1,2,3,4-tetrahydro-isochinolin-4-yl)-phenyl]-oxazolidin-2-on,
1-[2-((R)-6,8-Dichlor-2-methyl-1,2,3,4-tetrahydro-isochinoün-4-yl)-phenyl]-1,3-dihydro-imidazol-2-on,
1-[2-((R)-6,8-Dichlor-2-methyl-1,2,3,4-tetrahydro-isochinolin-4-yl)-phenyl]-imidazolidin-2-on,
1-[2-((R)-6,8-Dichlor-2-methyl-1,2,3,4-tetrahydro-isochinolin-4-yl)-phenyl]-pyrrolidin-2-on,
1-[2-((R)-6,8-Dichlor-2-methyl-1,2,3,4-tetrahydro-isochinolin-4-yl)-phenyl]-pyrrolidin-2-on,
1-[3-((S)-6,8-Dichlor-2-methyl-1,2,3,4-tetrahydro-isochinolin-4-yl)-phenyl]-piperidin-2-on,
1-[2-((R)-6,8-Dichlor-2-cyclopropyl-1,2,3,4-tetrahydro-isochinolin-4-yl)-phenyl]-pyrrolidin-2-on
und
3-[2-((R)-6,8-Dichlor-2-cyclopropyl-1,2,3,4-tetrahydro-isochinolin-4-yl)-phenyl]-oxazolidin-2-on,
sowie deren pharmazeutisch verträglichen Salze und Trifluoracetate.

Insbesondere bevorzugt sind Verbindungen der Formel I ausgewählt aus der Gruppe: 1-[2-(8-Brom-6-chlor-2-methyl-1,2,3,4-tetrahydro-isochinolin-4-yl)-phenyl]-pyrrolidin-2-on,
1-[2-((R)-6,8-Dichlor-2-methyl-1,2,3,4-tetrahydro-isochinolin-4-yl)-phenyl]-3-methyl-1,3-dihydro-imidazol-2-on,
(R)-6,8-Dichlor-4-[2-(1,1-dioxo-1-λ⁶-isothiazolidin-2-yl)-phenyl]-2-methyl-1,2,3,4-tetrahydro-isochinolin,
3-[2-((R)-6,8-Dichlor-2-methyl-1,2,3,4-tetrahydro-isochinolin-4-yl)-phenyl]-oxazolidin-2-on,
1-[2-((R)-6,8-Dichlor-2-methyl-1,2,3,4-tetrahydro-isochinolin-4-yl)-phenyl]-1,3-dihydroimidazol-2-on,
1-[2-((R)-6,8-Dichlor-2-methyl-1,2,3,4-tetrahydro-isochinolin-4-yl)-phenyl]-imidazolidin-2-on,
1-[2-((R)-6,8-Dichlor-2-methyl-1,2,3,4-tetrahydro-isochinolin-4-yl)-phenyl]-pyrrolidin-2-on
und
1-[2-((R)-6,8-Dichlor-2-methyl-1,2,3,4-tetrahydro-isochinolin-4-yl)-phenyl]-pyrrolidin-2-on,
sowie deren pharmazeutisch verträglichen Salze und Trifluoracetate.

In einer weiteren Ausführungsform sind Verbindungen der Formel I bevorzugt, in denen die Reste R1, R2, R3 und R4 unabhängig voneinander beschrieben werden durch Wasserstoff, F, Cl, Br, CN oder R11-(CₘH₂ₘ)-Aₙ-, wobei m und n unabhängig voneinander Null oder 1 sind, R11 Wasserstoff, Methyl oder C_{P}F₂ₚ₊₁ ist und A Sauerstoff, NCH₃ oder S(O)q ist, wobei p 1 oder 2 und q Null, 1 oder 2 sind; besonders bevorzugt sind Verbindungen der Formel I, in denen R1 und R3 Wasserstoff sind und R2 und R4 unabhängig voneinander Wasserstoff, F, Cl, Br, NH₂, NHCH₃ oder N(CH₃)₂, beispielsweise Cl oder Br, sind. In einer Ausführungsform sind Verbindungen der Formel bevorzugt, in denen R1 und R3 Wasserstoff sind und R2 und R4 unabhängig voneinander F, Cl, NH₂, NHCH₃ oder N(CH₃)₂, beispielsweise Cl, sind.

In einer weiteren Ausführungsform sind Verbindungen der Formel I bevorzugt, in denen R5 durch Wasserstoff, Methyl, Ethyl oder Cyclopropyl beschrieben wird; besonders bevorzugt sind Verbindungen der Formel I, in denen R5 Wasserstoff, Methyl oder Cyclopropyl, beispielweise Methyl, ist.

In einer weiteren Ausführungsform sind Verbindungen der Formel bevorzugt, in denen R6 durch Wasserstoff oder Methyl, beispielsweise Wasserstoff, beschrieben wird,

In einer weiteren Ausführungsform sind Verbindungen der Formel I bevorzugt, in denen die Reste R7 und R8 unabhängig voneinander beschrieben werden durch Wasserstoff, F, Cl, CN, CO₂R12, NR13R14 oder R16-(CₘₘH₂ₘₘ)-Bₙₙ-, wobei R12 Wasserstoff, Methyl oder Ethyl ist, R13 und R14 unabhängig voneinander Wasserstoff, Alkyl mit 1, 2, 3 oder 4 C-Atomen oder Cycloalkyl mit 3, 4, 5 oder 6 C-Atomen sind oder R13 und R14 zusammen mit dem Stickstoffatom, an das sie gebunden sind, einen 5-, 6- oder 7- gliedrigen Ring bilden, in dem eine CH₂-Gruppe durch NR15, S oder Sauerstoff ersetzt sein kann und wobei R15 Wasserstoff, Alkyl mit 1, 2, 3 oder 4 C-Atomen oder Cycloalkyl mit 3, 4, 5 oder 6 C-Atomen ist, und wobei mm Null, 1 oder 2 ist, nn Null oder 1 ist und R16 Wasserstoff, Methyl oder CₚₚF₂ₚₚ₊₁ sind, wobei B Sauerstoff oder S(O)qq ist, wobei pp 1 oder 2 und qq Null, 1 oder 2 ist; besonders bevorzugt sind Verbindungen der Formel I, in denen R7 und R8 Wasserstoff sind.

In einer weiteren Ausführungsform sind Verbindungen der Formel I bevorzugt, in denen W beschrieben wird durch CᵣH₂ᵣ oder CₛH₂ₛ₋₂, wobei eine oder mehrere CH₂-Gruppen in CᵣH₂ᵣ und CₛH₂ₛ₋₂ durch NR17, Sauerstoff oder S ersetzt sein können, wobei R17 Wasserstoff, Alkyl mit 1, 2, 3 oder 4 C-Atomen oder Cycloalkyl mit 3, 4, 5 oder 6 C-Atomen, insbesondere Wasserstoff oder Methyl ,beispielsweise Wasserstoff, ist, und wobei r 2, 3, 4, 5, 6, 7 oder 8, insbesondere 2, 3, 4 ,5 oder 6, ist und s 2, 3, 4, 5, 6, 7 oder 8, insbesondere 2, 3, 4, 5 oder 6, ist.

In einer weiteren Ausführungsform sind Verbindungen der Formel I bevorzugt, in denen X -C(O)- oder -S(O)₂- ist.

In einer weiteren Ausführungsform sind Verbindungen der Formel I bevorzugt, in denen
Z -C(O)- ist.
In einer anderen Ausführungsform sind Verbindungen der Formel I bevorzugt, in denen Z eine Bindung ist.

Enthalten die Verbindungen der Formel I ein oder mehrere Asymmetriezentren, so können diese unabhängig voneinander sowohl S als auch R konfiguriert sein. Die Verbindungen können als optische Isomere, als Diastereomere, als Racemate oder als Gemische in allen Verhältnissen derselben vorliegen. Die Verbindungen der Formel I können weiterhin in Form von Rotationsisomeren vorliegen.

Die vorliegende Erfindung umfasst alle möglichen tautomeren Formen der Verbindungen der Formeln I.

Die vorliegende Erfindung umfasst weiterhin Derivate der Verbindungen der Formel I, zum Beispiel Solvate, wie Hydrate und Alkoholaddukte, Ester, Prodrugs und andere physiologisch akzeptablen Derivate der Verbindungen der Formel I, sowie aktive Metaboliten der Verbindungen der Formel I. Die Erfindung umfasst ebenfalls alle Kristallmodifikationen der Verbindungen der Formel I.

Alkylreste können geradkettig oder verzweigt sein. Dies gilt auch, wenn sie Substituenten tragen oder als Substituenten anderer Reste auftreten, beispielsweise in Fluoralkylresten oder Alkoxyresten. Beispiele für Alkylreste sind Methyl, Ethyl, n-Propyl, Isopropyl (= 1-Methylethyl), n-Butyl, Isobutyl (= 2-Methylpropyl), sec-Butyl (= 1-Methylpropyl), tert-Butyl (= 1,1-Dimethylethyl), n-Pentyl, Isopentyl, tert-Pentyl, Neopentyl und Hexyl. Bevorzugte Alkylreste sind Methyl, Ethyl, n-Propyl, Isopropyl und n-Butyl. In Alkylresten können ein oder mehrere, zum Beispiel 1, 2, 3, 4, 5, 6, 7, 8, 9, 10, 11, 12, 13 oder 14, Wasserstoffatome durch Fluoratome substituiert sein. Beispiele für solche Fluoralkylreste sind Trifluormethyl, 2,2,2-Trifluorethyl, Pentafluorethyl, Heptafluorisopropyl. Substituierte Alkylreste können in beliebigen Positionen substituiert sein.

Alkylenreste, wie beispielsweise CₘH₂ₘ, CₘₘH₂ₘₘ oder CᵣH₂ᵣ, können geradkettig oder verzweigt sein. Dies gilt auch, wenn sie Substituenten tragen oder als Substituenten anderer Reste auftreten, beispielsweise in Fluoralkylenresten, wie beispielweise in CₚF₂ₚ und CₚₚF₂ₚₚ. Beispiele für Alkylenreste sind Methylen, Ethylen, 1-Methylmethylen, Propylen, 1-Methylethylen, Butylen, 1-Propylmethylen, 1-Ethyl-1-methylmethylen, 1,2-Dimethylethylen, 1,1-Dimethylmethylen, 1-Ethylethylen, 1-Methylpropylen, 2-Methylpropylen, Pentylen, 1-Butylmethylen, 1-Propylethylen, 1-Methyl-2-ethylethylen, 1,2-Dimethylpropylen, 1,3-Dimethylpropylen, 2,2-Dimethylpropylen, Hexylen und 1-Methylpentylen. In Alkylenresten können ein oder mehrere, zum Beispiel 1, 2, 3, 4, 5, 6, 7, 8, 9, 10, 11 oder 12 Wasserstoffatome durch Fluoratome substituiert sein. Substituierte Alkylenreste können in beliebigen Positionen substituiert sein. In den Alkylenresten können eine oder mehrere CH₂-Gruppen durch Sauerstoff, S, NH, N-Alkyl oder N-Cycloalkyl ersetzt sein. Dabei können sowohl in geradkettigen als auch in verzweigten Alkylenketten CH₂-Gruppen durch Sauerstoff, S, NH, N-Alkyl oder N-Cycloalkyl ersetzt sein, beispielsweise als 1-Hydroxyethylen-Rest.

Alkenylenreste, wie zum Beispiel CₛH₂ₛ₋₂, können geradkettig oder verzweigt sein. Dies gilt auch, wenn sie Substituenten tragen beispielsweise in Fluoralkenylenresten. Die Alkenylenreste können in unterschiedlichen Positionen ungesättigt sein. Beispiele für Alkenylenreste sind Ethenylen, 1-Methylethenylen, Propenylen, But-1-enylen, But-2-enylen, 1-Methylprop-1-enylen, 1,2-Dimethylethylen, Pentenylen oder Hexenylen. In Alkenylenresten können ein oder mehrere, zum Beispiel 1, 2, 3, 4, 5, 6, 7, 8, 9 oder 10 Wasserstoffatome durch Fluoratome substituiert sein. Substituierte Alkenylenreste können in beliebigen Positionen substituiert sein. In den Alkenylenresten können eine oder mehrere CH₂-Gruppen durch Sauerstoff, S, NH, N-Alkyl oder N-Cycloalkyl ersetzt sein. Dabei können sowohl in geradkettigen als auch in verzweigten Alkenylenketten CH₂-Gruppen durch Sauerstoff, S, NH, N-Alkyl oder N-Cycloalkyl ersetzt sein.

Beispiele für Cycloalkylreste sind Cyclopropyl, Cyclobutyl, Cyclopentyl oder Cyclohexyl. In Cycloalkylresten können ein oder mehrere, zum Beispiel 1, 2, 3, 4, 5, 6, 7, 8, 9, 10, 11 oder 12 Wasserstoffatome durch Fluoratome substituiert sein. Substituierte Cycloalkylreste können in beliebigen Positionen substituiert sein. Cycloalkylreste können auch verzeigt als Alkyl-cycloalkyl oder Cycloalkyl-alkyl vorliegen, zum Beispiel Methyl-cyclohexyl oder Cyclohexyl-methyl.

Beispiele für Ringe aus NR13R14, wobei R13 und R14 mit dem Stickstoffatom, an das sie gebunden sind, einen 4, 5, 6 oder 7 gliedrigen Ring bilden, sind Morpholin, Pyrrolidin, Piperidin, Piperazin und N-Methylpiperazin.

Als CH₂-Einheiten gelten auch die in einem Alkylrest terminalen CH₃-Gruppen, die in diesem Zusammenhang als CH₂-H Gruppierungen aufgefasst werden. Dies gilt auch in verzweigten Alkylenresten, wie beispielsweise CₘH₂ₘ, CₘₘH₂ₘₘ oder CᵣH₂ᵣ.

Wenn eine Variable, beispielsweise cycloalkyl oder R1, mehr als einmal als Komponente vorkommt, sind die Definitionen der Variable bei jedem Auftreten unabhängig voneinander.

Enthalten die Verbindungen der Formel I eine oder mehrere saure oder basische Gruppen bzw. einen oder mehrere basische Heterocyclen, so gehören auch die entsprechenden physiologisch oder toxikologisch verträglichen Salze zur Erfindung, insbesondere die pharmazeutisch verwendbaren Salze. So können die Verbindungen der Formel I an einer sauren Gruppe deprotoniert werden und beispielsweise als Alkalimetallsalze, vorzugsweise Natrium- oder Kaliumsalze, oder als Ammoniumsalze, zum Beispiel als Salze mit Ammoniak oder organischen Aminen oder Aminosäuren, verwendet werden. Da Verbindungen der Formel I sets wenigstens eine basische Gruppe enthalten, können sie auch in Form ihrer physiologisch verträglichen Säureadditionssalze z. B. mit folgenden Säuren hergestellt werden: aus anorganischen Säuren wie Salzsäure, Schwefelsäure oder Phosphorsäure oder aus organischen Säuren wie Essigsäure, Zitronensäure, Weinsäure, Milchsäure, Malonsäure, Methansulfonsäure, Fumarsäure. Als Säureadditionssalze kommen dabei Salze aller pharmakologisch verträglichen Säuren in Frage, beispielsweise Halogenide, insbesondere Hydrochloride, Lactate, Sulfate, Citrate, Tartrate, Acetate, Phosphate, Methylsulfonate, p-Toluolsulfonate, Adipinate, Fumarate, Gluconate, Glutamate, Glycerolphosphate, Maleinate und Pamoate (diese Gruppe entspricht auch den physiologisch akzeptablen Anionen); aber auch Trifluoracetate.

Gegenstand der Erfindung sind auch die im folgenden beschriebenen Verfahren zur Herstellung von Verbindungen der Formel I.

Die hier beschriebenen Verbindungen der Formel I lassen sich beispielsweise nach oder analog zu literaturbekannten Methoden ausgehend von Anilin-Derivaten der Formel VIII herstellen.

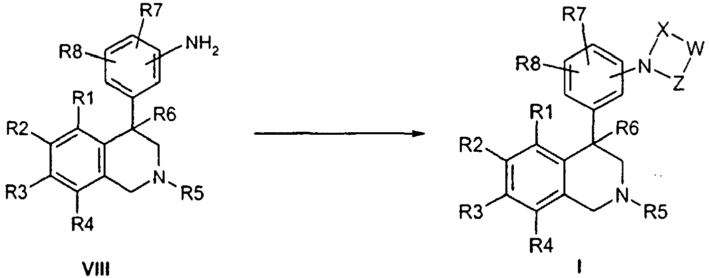

Das Anilin der Formel VIII lässt sich beispielsweise durch Erhitzen mit den entsprechenden Säuren der Formel XXII in Polyphosphorsäure (PPA) in die Verbindungen der Formel Ia überführen (Tetrahedron Letters 2003, 44, 2133), wobei R1 bis R8 und W wie oben definiert sind
und
X und Z -C(O)- entsprechen.

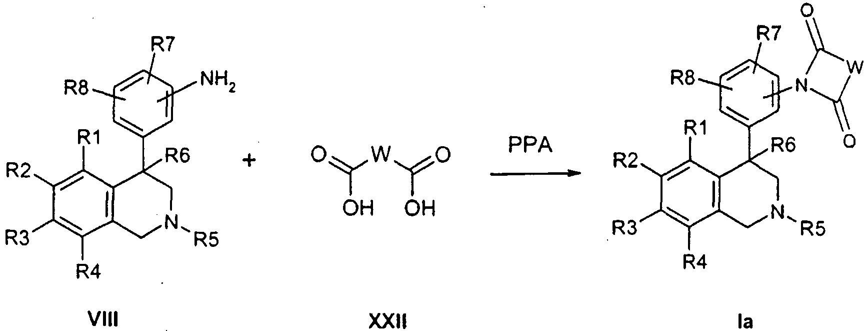

Alternativ können Verbindungen der Formel Ia erhalten werden, indem man Aniline der Formel VIII in aprotischen Lösungsmitteln wie Dichlormethan mit Anhydriden vom Typ XXIX zu intermediären Amid-Säuren umsetzt. Die gewünschten Imide der Formel Ia werden dann erhalten, indem man die Intermediate in aprotischen Lösungsmitteln wie Dichlormethan mit geeigneten Cyclisierungsreagenzien wie EDC in Gegenwart von Basen wie Hünig-Base ringschließt. Alternativ können die Intermediate auch in hoch siedenden Lösungsmitteln wie Diphenylether oder ganz ohne Lösungsmittel erhitzt werden, so dass es unter Wasserverlust zur Ringschlussbildung kommt, wobei die Reste R1-R8 und W wie oben definiert sind
und
X und Z -C(O)- entsprechen.

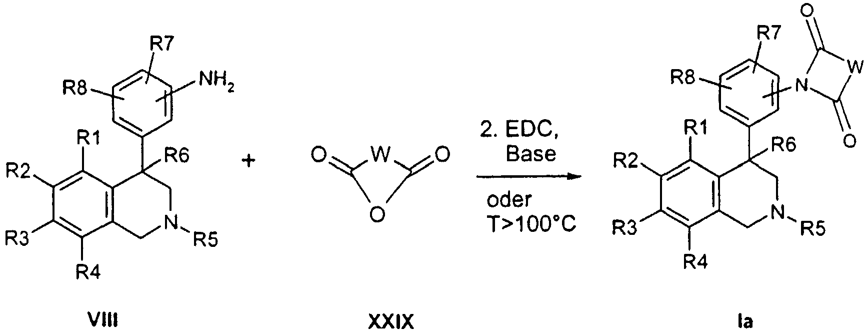

Verbindungen der Formel Ib können beispielsweise in einer zweistufigen Reaktion ausgehend von Isocyanatcarbonsäureestern der Formel XII aufgebaut werden. Dabei wird intermediär zunächst durch Reaktion des Anilinstickstoffs mit der Isocyanatgruppe der Harnstoff aufgebaut, bevor anschließend säurekatalysiert der Ringschluss erfolgt, wobei R1 bis R8 wie oben definiert sind,
X und Z -C(O)- entsprechen,
R22 Alkyl mit 1, 2, 3 oder 4C-Atomen ist, beispielsweise Methyl oder Ethyl, -V_{N}-NH- für einen Rest W steht, in dem eine terminale CH₂-Gruppe durch NH ersetzt ist
und
-V'-NCO für einen Rest W steht, in dem eine terminale CH₂-Gruppe durch eine Isocyanat-Gruppe ersetzt ist.

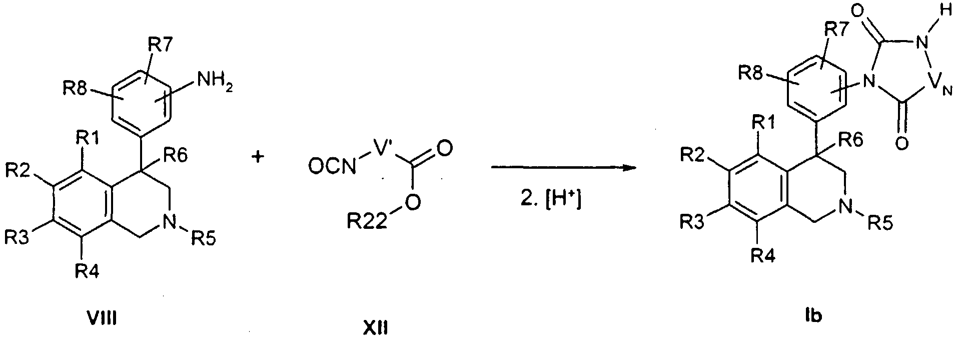

Eine Variation des Restes R17 lässt sich anschließend durch Alkylierung der Verbindung der Formel Ib in Gegenwart einer Base wie beispielsweise Lithiumdiisopropylamid oder Lithium- bzw. Natriumhexamethyldisilazid mit einem Alkylierungsmittel der Formel XIII erreichen,
wobei R1 bis R8 und R17 wie oben definiert sind,
X und Z -C(O)- entsprechen,
-V_{N}-NH- für einen Rest W steht, in dem eine terminale CH₂-Gruppe durch NH ersetzt ist,
-V_{N}-NR17- für einen Rest W steht, in dem eine terminale CH₂-Gruppe durch NR17 ersetzt ist
und
LG einer bei Alkylierungen gängigen Fluchtgruppe wie beispielsweise Bromid, Chlorid, Tosylat oder Mesylat entspricht.

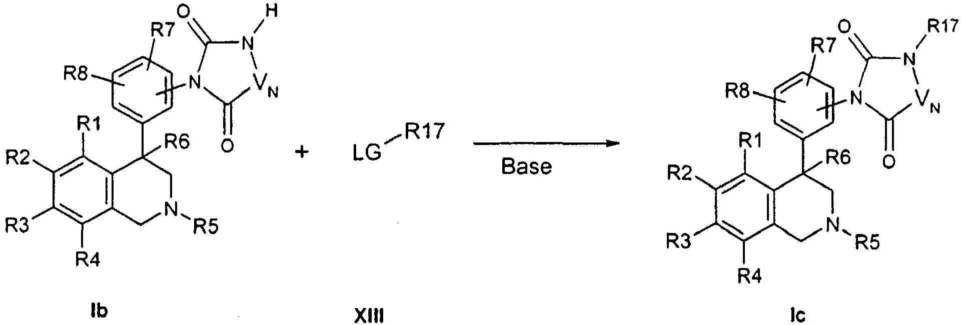

Alternativ kann man den Ring auch in einer dreistufigen Sequenz aufbauen. Dazu wird zunächst auf den anilinischen Stickstoff eine Carbonylgruppe übertragen. Dies geschieht beispielsweise mit Chlorameisensäurederivaten der Formel XIV oder Carbonyldiimidazol. Nachfolgende Umsetzung mit Aminoestern der Formel XV, gefolgt von säure- oder basenkatalysierter Cyclisierung, bevorzugt mit Salzsäure oder Natriumhexamethyldisilazid, führt zu Verbindungen der Formel Ic,
wobei R1 bis R8, R17 und -V_{N}-NR17- wie oben definiert sind,
R23 Alkyl mit 1, 2, 3 oder 4C-Atomen, beispielsweise Methyl oder Ethyl, ist und
R24 ein optional substituiert Phenylrest ist, beispielsweise phenyl oder 4-nitrophenyl.

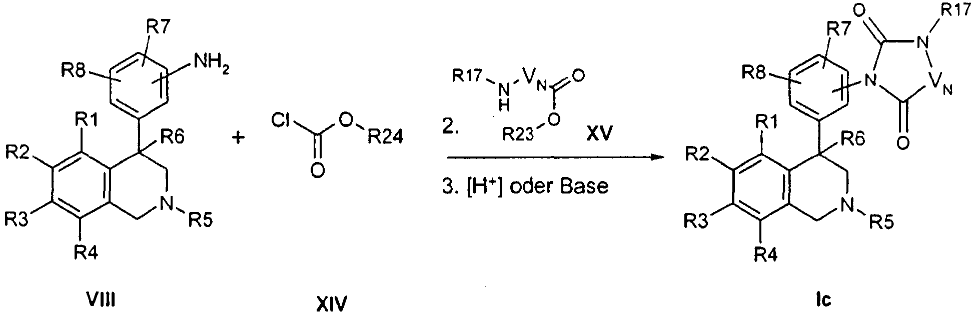

Cyclische Harnstoffe der Formel Id lassen sich aus dem Produkt vom Anilin der Formel VIII mit dem Chlorameisensäurederivat der Formel XIV erhalten, indem man das gebildete Carbamat mit Aminen der Formel XVI umsetzt, gefolgt von einem Cyclisierungsschritt in Gegenwart einer Base wie beispielsweise Natriumhydrid, Kaliumcarbonat oder Natriumhexamethyldisilazid, wobei
wobei R1 bis R8, R17, R24, -V_{N}-NR17- und -V_{N}-NHR17 wie oben definiert sind, und
Y eine Fluchtgruppe, beispielsweise Chlor, oder aber eine Vorstufe zu einer Fluchtgruppe, beispielsweise Hydroxy, das dann zum Beispiel mit Mesylchlorid in eine Fluchtgruppe überführt wird, ist.

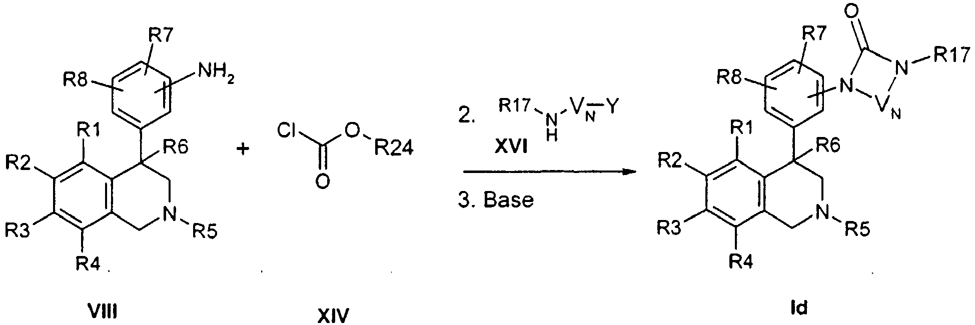

Lactame der Formel le können durch Umsetzung eines Anilins der Formel VIII mit Lactonen der Formel XVII hergestellt werden (Synlett 2001, 1485), indem in den intermediär gebildeten Hydroxyamiden aus der Hydroxygruppe durch Umsetzung mit beispielsweise Sulfonsäurechloriden, Anydriden oder starken Säuren eine gute Fluchtgruppe gemacht wird, die dann anschließend durch den anilinischen Stickstoff bevorzugt in Gegenwart von Base wie beispielsweise Natriumhydrid, Kaliumcarbonat oder Natriumhexamethyldisilazid substituiert wird,
wobei R1 bis R8 und W wie oben definiert sind,
X -C(O)- entspricht
und
Z eine Bindung ist.

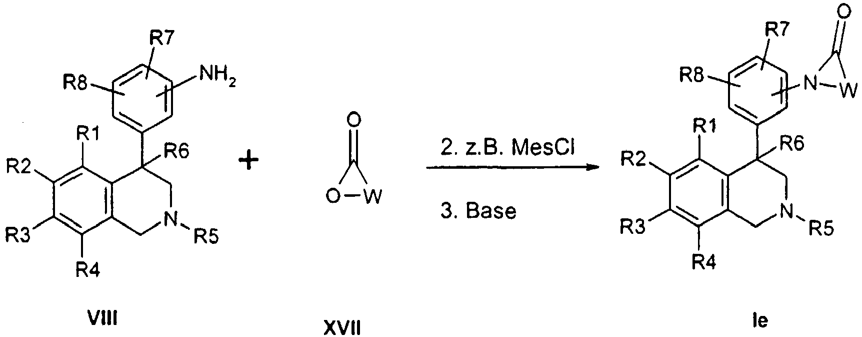

Alternativ kann auch die direkte Umsetzung von Halogenaromaten der Formel XVIII mit Lactamen der Formel XIX in Gegenwart von Katalysatoren wie beispielsweise Kupferiodid zu Verbindungen der Formel le erfolgen (J. Am. Chem. Soc. 2001, 7727, ibd. 2002, 7421),
wobei R1-R8 und W wie oben definiert sind,
X -C(O)- entspricht,
Z eine Bindung ist
und
Hal Cl, Br, I oder -O-Triflat ist.

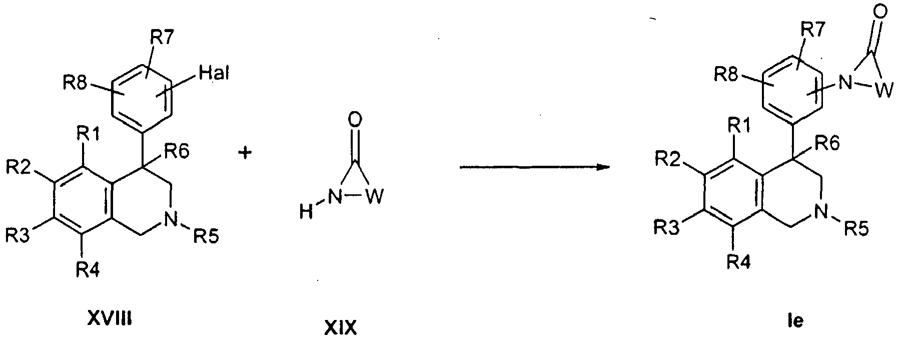

Die Darstellung der Halogenaromaten der Formel XVIII erfolgt dabei ausgehend von den Carbonylderivaten der Formel VI analog zu dem Aufbau der Anilinderivate der Formel VIII, der weiter unten beschrieben wird.

Eine weitere Alternative stellt die Umsetzung von Anilinen der Formel VIII mit Säurechloriden der Formel XXV zu Verbindungen der Formel le dar. Die intermediär erhaltenen Amide werden dann in Gegenwart von Basen wie Natriumhydrid, Kaliumcarbonat oder Natriumhexamethyldisilazid in Lösungsmitteln wie THF, DMSO und DMF cyclisiert. Dabei sind die Reste R1 bis R8, LG und W wie oben definiert, Z entspricht einer Bindung und
X -C(O)-

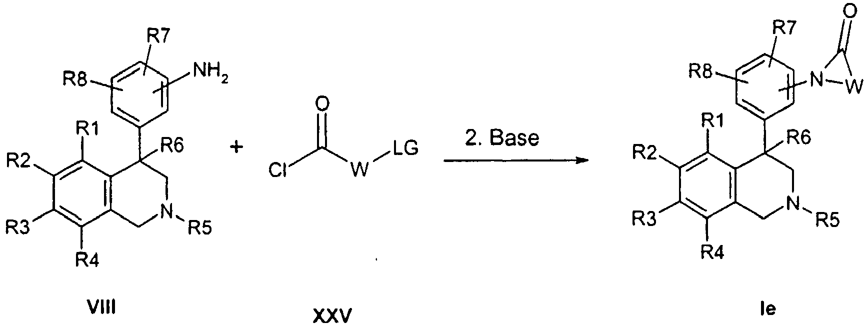

Sulfonamide der Formel If lassen sich ausgehend von den Anilinen der Formel VIII durch Umsetzung mit Chloralkylsulfamylchloriden der Formel XX darstellen (Tetrahedron Letters 44, 5483 (2003)),
wobei R1 bis R8, R17 und -V_{N}-NR17- wie oben definiert sind,
T Cl oder Br ist,
X -SO₂- entspricht
und
Z eine Bindung ist.

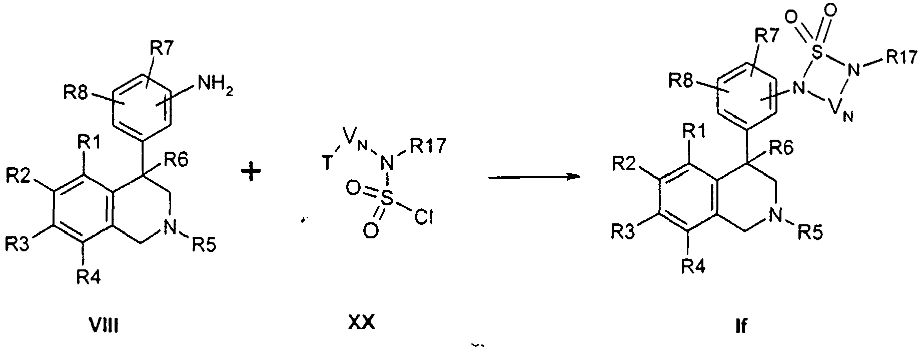

Verbindungen der Formel Ig können über eine säurekatalysierte Cyclisierungsreaktion aufbaut werden. Ausgehend von den Harnstoffen der Formel XXI, die nach einer der oben genannten Methoden hergestellt werden können, werden in Gegenwart von Säure wie beispielsweise Salzsäure oder Ameisensäure unter Spaltung des Ketals bzw. Acetals Verbindungen der Formel Ig hergestellt, wobei R1 bis R8 und R17 wie oben definiert sind,
X -C(O)- entspricht,
Z eine Bindung ist,
-NR17-V"-CV*HCV"'(ORa)₂ einem Rest W mit der Bedeutung CᵣH₂ᵣ entspricht, in dem eine terminale CH₂-Gruppe durch NR17, eine weitere CH₂-Gruppe durch eine Acetal/Ketalgruppierung ersetzt ist und V* und V"' mögliche Verzweigungen des Alkylenrestes angeben, wobei Ra Alkyl mit 1, 2, 3 oder 4 C-Atomen ist, beispielsweise Methyl oder Ethyl, oder die beiden Reste Ra gemeinsam einen Ethylenrest bilden und
-NR17-V"-CV*=CV"'- steht für einen Rest W mit der Bedeutung CₛH₂ₛ₋₂, in dem eine terminale CH₂-Gruppe durch NR17 ersetzt ist und V* und V"' mögliche Verzweigungen des Alkenylrestes angeben.

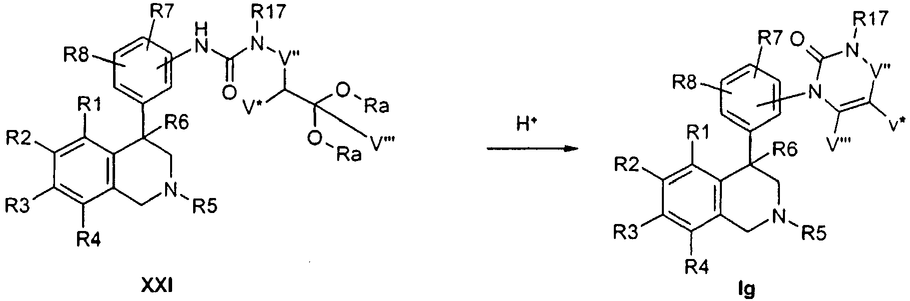

Verbindungen der Formel Ih können gewonnen werden, indem man zunächst das Anilin VIII mit Chlorthioformiaten der Formel XXVI wie Phenylchlorthioformiat umsetzt und anschließend das erhaltene intermediäre Thiocarbamat nach Behandlung mit Natriummethylat-Lösung mit einem Säurechlorid vom Typ XXVII reagieren lässt. Dabei sind die Reste R1 bis R8, R24 und LG wie oben definiert.

V_{K} steht für eine Gruppe W, die um eine CH₂-Gruppe verkürzt ist,

V_{S}-S- steht für eine Gruppe W, in der eine CH₂-Gruppe durch Schwefel ersetzt ist und
X und Z entsprechen -C(O)-.

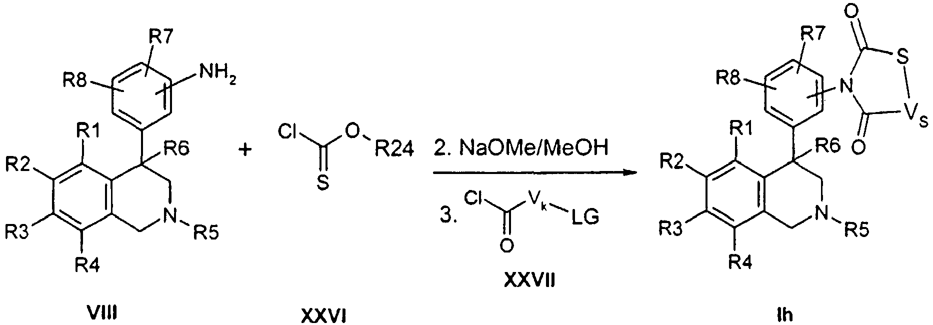

Verbindungen der Formel li lassen sich in einer dreistufigen Sequenz wie folgt synthetisieren. Zunächst werden Aniline vom Typ VIII in aprotische Lösungsmitteln wie THF in Gegenwart von Basen wie Natriumhexamethyldisilazid mit Säurechloriden vom Typ XXVIII zu intermediären Amiden umgesetzt. Anschließend wird in Gegenwart von Nukleophilen und Basen wie Kaliumcarbonat in Methanol die Schutzgruppe P vom Sauerstoff entfernt. Abschließend wird der erhaltene Alkohol in einem aprotischen Lösungsmittel wie THF mit einem Carbonyläquivalent wie 1,1-Carbonyldiimidazol oder Phosgen in Gegenwart einer Base wie Natriumhexamethyldisilazid umgesetzt.

Die Reste R1-R8 sind wie oben definiert,

V_{O}-O- entspricht einer Gruppe W, in der eine CH₂-Gruppe durch Sauerstoff ersetzt ist,

P entspricht einer Schutzgruppe wie Acetyl, Benzoyl, Benzoxycarbonyl oder Trityl und X und Z entsprechen -C(O)-.

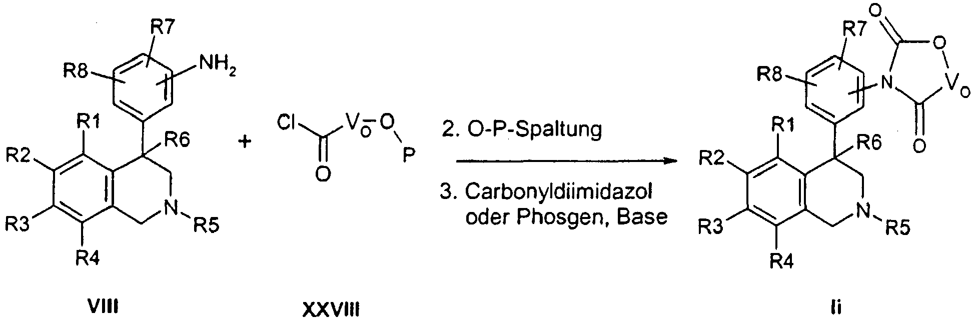

Verbindungen der Formel Ij lassen sich darstellen, indem man Aniline der Formel VIII mit Chloroformaten der Formel XXX in aprotischen Lösungsmitteln wie THF umsetzt und das gebildete intermediäre Carbamat anschließend in aprotischen Lösungsmitteln wie THF in Gegenwart einer Base wie Natriumhydrid ringschließt. Dabei sind die Reste R1-R8. LG und V_{O}-O wie oben definiert und
Z entspricht einer Bindung und
X -C(O)-.

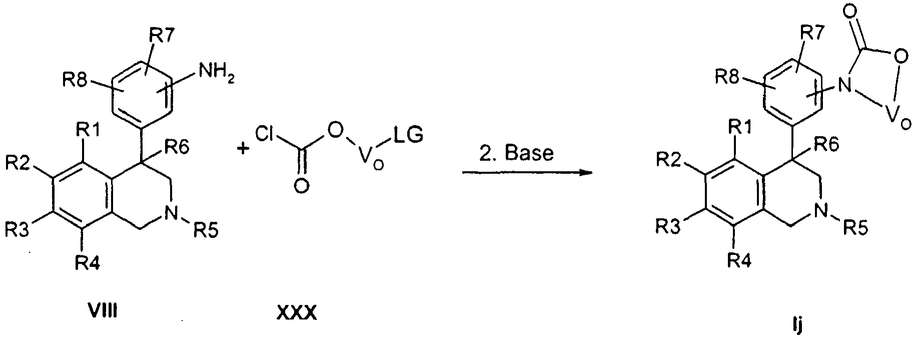

Verbindungen der Formel Ik lassen sich erhalten, indem man Aniline der Formel VIII mit Sulfonsäurechloriden der Formel XXXI in aprotischen Lösungsmitteln wie THF in Gegenwart von Basen wie Natriumhexamethyldisilazid umsetzt. Dabei sind die Reste R1-R2, T und W wie oben definiert und
Z entspricht einer Bindung und
X -SO₂-.

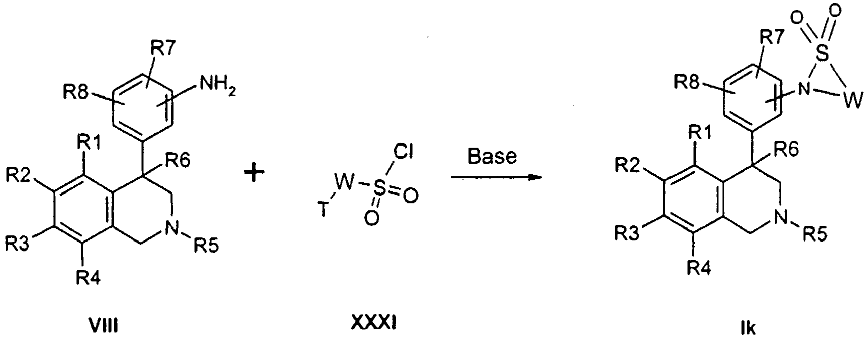

Die Verbindungen der Formeln XII, XIII, XIV, XV, XVI, XVII, XIX, XX,XXII, XXV, XXVI, XXVII, XXVIII, XXIX, XXX und XXXI sind käuflich erhältlich oder können nach oder analog zu in der Literatur beschriebenen, dem Fachmann bekannten Verfahren hergestellt werden.

Die Ausgangsverbindungen der Formel VIII können wie folgt hergestellt werden:

Durch Reduktion der Carbonylgruppierung in Verbindungen der Formel VI und anschließender säurekatalysierter Cyclisierung der entsprechenden Alkohole der Formel VII (vgl. Tetrahedron Lett. 1989, 30, 5837; Org. Prep. Proced. Int. 1995, 27, 513) können Tetrahydroisochinoline der Formel VIIIa, nach bekannten Verfahren gewonnen werden, wobei R1 bis R8 wie oben definiert sind
und
R20 für eine dem Fachmann geläufige Stickstoff-Schutzgruppe steht, beispielsweise ein Acetylrest.

Zu Verbindungen der Formel VIII gelangt man anschließend in dem Fachmann bekannter Weise ausgehend von Verbindungen vom Typ der Formel VIIIa, indem man die Schutzgruppe R20 entfernt. Dies geschieht in protischen Solvenzien wie Wasser oder niederen Alkoholen bevorzugt unter Säurekatalyse beispielsweise mit Salzsäure oder Trifluoressigsäure oder unter Basenkatalyse beispielsweise in Gegenwart von Natriummethylat oder -ethylat.

Alternativ kann nach erfolgter Reduktion die Schutzgruppe R20 vor der Cyclisierung abgespalten werden, was vorteilhaft in Gegenwart starker Basen wie beispielsweise Natriummethlylat oder -ethylat in Methanol oder Ethanol gelingt. Aber auch mit Säuren wie Salzsäure vorteilhaft in Gegenwart von Alkoholen wie Methanol oder Ethanol kann R20 entfernt werden. Vorheriges Entfernen von R20 ist besonders im Falle der ortho-Aminoderivate empfehlenswert. Man gelangt so direkt zu den ungeschützten Anilinen der Formel VIII.

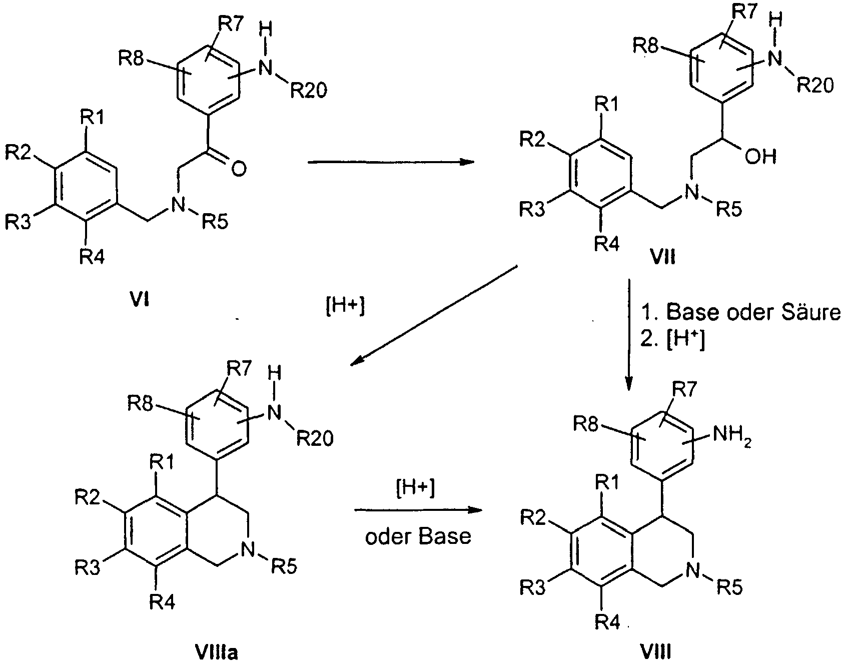

Zur Herstellung alkylverzweigter Verbindungen der Formel I, in denen R6 nicht Wasserstoff ist, können die entsprechenden Diphenyl-essigsäureester der Formel IX in alpha-Stellung mit R6 nach bekannten Methoden alkyliert werden. Vorteilhafterweise wird dabei das anilinische Wasserstoffatom durch die Schutzgruppe R21, beispielsweise Allyl oder Benzyl, ersetzt. Die Verbindungen der Formel X können durch Standardverfahren in die entsprechenden Amide der Formel XI überführt werden, welche in einer Pictet-Spengler-analogen Reaktion in die gewünschten Tetrahydroisochinoline der Formel VIIIb überführt werden (vgl. Tetrahedron 1987, 43, 439; Chem. Pharm. Bull. 1985, 33, 340),
wobei R1 bis R8 und R20 wie oben definiert sind,
R21 eine Schutzgruppe, beispielsweise Allyl oder Benzyl ist
und
LG einer bei Alkylierungen gängigen Fluchtgruppe wie beispielsweise Bromid, Chlorid, Tosylat oder Mesylat entspricht.

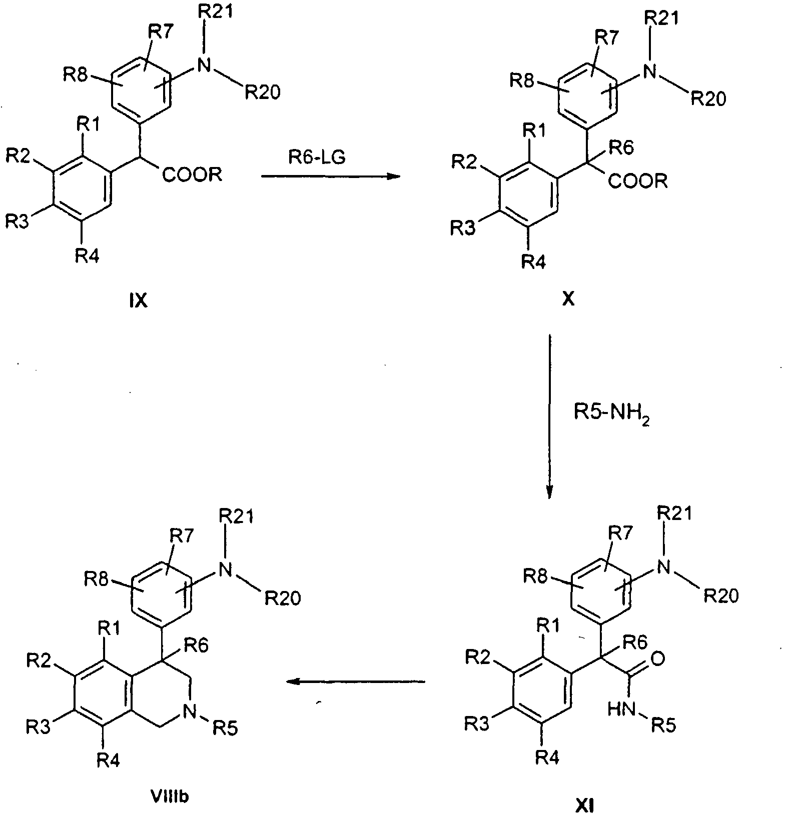

Entschützen der Verbindung der Formel VIIIb mittels dem Fachmann bekannter Verfahren liefert das freie Anilin der Formel VIII. Je nach Art der Schutzgruppe werden dabei Carbonylschutzgruppen wie Acetyl vorteilhaft säuren- (z. B. wässrige HCl) oder basenkatalysiert (z. B. mittels Natriummethlylat oder -ethylat) entfernt, während Benzyl- oder Allyl-Schutzgruppen am besten durch Hydrierung abgespalten werden. Verbindungen der Formel IX sind käuflich erhältlich oder können nach oder analog zu in der Literatur beschriebenen, dem Fachmann bekannten Verfahren hergestellt werden.

Die oben eingesetzten Verbindungen der Formel VI werden vorzugsweise aus Benzylaminen der Formel IV in dem Fachmann bekannter Weise und den entsprechenden Amino-substituierten alpha-Bromacetophenon-Verbindungen der Formel V hergestellt,
wobei R1 bis R8 und R20 wie oben definiert sind,

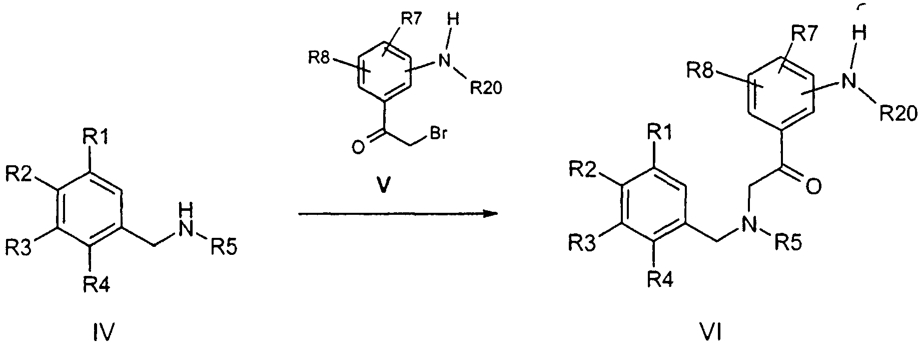

Die alpha-Bromacetophenon-Verbindungen der Formel V lassen sich in literatur-bekannten Verfahren aus den entsprechenden Acetophenon-Vorläufern durch Bromierung gewinnen.

Die Benzylamin-Vorläufer der Formel IV können, falls nicht käuflich erhältlich, nach dem Fachmann bekannten Standardverfahren aus den entsprechenden Benzylchloriden oder -bromiden der Formel III und den entsprechenden Aminen synthetisiert werden,
wobei R1 bis R5 wie oben definiert sind
und
X F, Cl, Br oder I, insbesondere Cl oder Br ist.

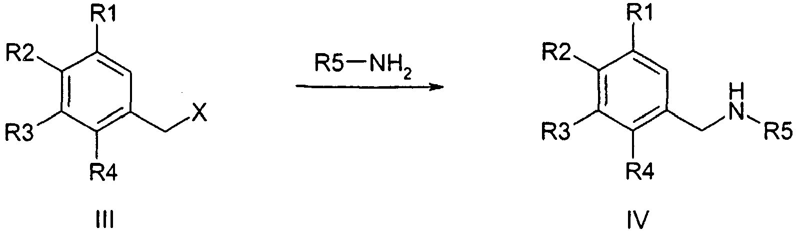

Alternativ sind Verbindungen der Formel IV auch durch reduktive Aminierung eines Aldehyds der Formel IIIa nach dem Fachmann bekannten Standardverfahren zugänglich,
wobei R1 bis R5 wie oben definiert sind.

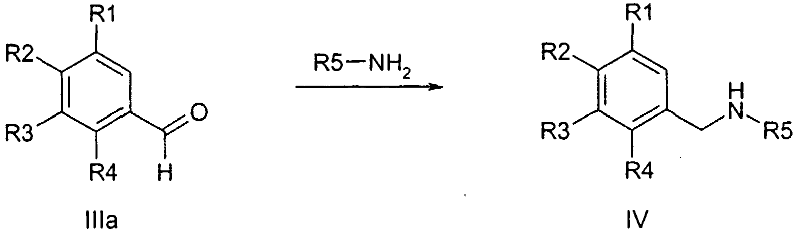

Die Verbindungen der Formeln III und IIIa und R5-NH₂ sind käuflich erhältlich oder können nach oder analog zu in der Literatur beschriebenen, dem Fachmann bekannten Verfahren hergestellt werden.

Die Aufarbeitung und gewünschtenfalls die Reinigung der Produkte und/oder Zwischenprodukte erfolgt nach den üblichen Methoden wie Extraktion, Chromatographie oder Kristallisation und den üblichen Trocknungen.

Es konnte gezeigt werden, dass Verbindungen der Formel I hervorragende Inhibitoren des Natrium-Wasserstoffaustauschers (NHE), insbesondere des Natrium-Wasserstoffaustauschers vom Subtyp 3 (NHE3), darstellen.

Die bisher bekannten NHE3-Inhibitoren leiten sich beispielsweise von Verbindungen des Acylguanidin-Typs (EP825178), Norbornylamin-Typs (WO0144164), 2-Guanidinochinazolin-Typs (WO0179186) oder Benzamidin-Typs (WO0121582, WO0172742) ab. Das ebenfalls als NHE3-Inhibitor beschriebene Squalamin (M. Donowitz et al. Am. J. Physiol. 276 (Cell Physiol. 45): C136 - C144) wirkt nach derzeitigem Wissensstand nicht unmittelbar wie die Verbindungen nach Formel I, sondern über einen indirekten Mechanismus und erreicht so seine maximale Wirkstärke erst nach einer Stunde.

Tetrahydroisochinoline als Inhibitoren des Natrium-Wasserstoffaustauschers vom Subtyp 3 (NHE3) werden bereits in den Patentanmeldung WO03048129, WO2004085404 und deutschen Anmeldung Nr. 102004046492.8 beschrieben. In der Patentanmeldung WO03055880 ist die verwandte Verbindungsklasse der Tetrahydroisochinolinium-Salze als NHE3-Inhibitoren beschrieben. Überraschend wurde nun gefunden, dass die hier beschriebenen Verbindungen der Formel I ebenfalls potente Inhibitoren des NHE3 darstellen und dabei vorteilhafte pharmakologische und pharmakokinetische Eigenschaften besitzen.

Der NHE3 wird im Körper verschiedener Spezies bevorzugt in der Galle, dem Darm und in der Niere gefunden (Larry Fliegel et al, Biochem. Cell. Biol. 76: 735 - 741, 1998), ließ sich aber auch im Gehirn nachweisen (E. Ma et al. Neuroscience 79: 591 - 603).

Aufgrund ihrer NHE-inhibitorischen Eigenschaften eignen sich die Verbindungen der Formel I Zur Prävention und Behandlung von Krankheiten, die durch eine Aktivierung bzw. durch einen aktivierten NHE verursacht werden, sowie von Krankheiten, die sekundär durch die NHE-bedingten Schädigungen verursacht werden.

Die Verbindungen der Formel I können auch zur Behandlung und Prävention von Krankheiten eingesetzt werden, wobei der NHE nur partiell gehemmt wird, beispielsweise durch Verwendung einer niedrigeren Dosierung.

Die Verwendung der erfindungsgemäßen Verbindungen betrifft die Prävention und die Behandlung akuter und chronischer Krankheiten in der Veterinär- und in der Humanmedizin.

Infolge Ihrer pharmakologische Wirkungen sind die Verbindungen der Formel I insbesondere dazu geeignet, zu einer Verbesserung des Atemantriebes führen. Sie können deshalb zur Behandlung von gestörten Atmungszuständen herangezogen werden, wie sie beispielsweise bei folgenden klinischen Zuständen und Krankheiten auftreten können: Gestörter zentraler Atemantrieb (z. B. zentrale Schlafapnoen, plötzlicher Kindstod, postoperative Hypoxie), muskulär-bedingte Atemstörungen, Atemstörungen nach Langzeitbeatmung, Atemstörungen bei Adaptation im Hochgebirge, obstruktive und gemischte Form der Schlafapnoen, akute und chronische Lungenkrankheiten mit Hypoxie und Hyperkapnie.

Zusätzlich erhöhen die Verbindungen den Muskeltonus der oberen Atemwege, so dass das Schnarchen unterdrückt wird. Die genannten Verbindungen finden deshalb vorteilhaft Verwendung zur Herstellung eines Medikaments zur Prävention und Behandlung von Schlafapnoen und muskulär bedingter Atemstörungen und zur Herstellung eines Medikaments zur Prävention und Behandlung des Schnarchens.

Eine Kombination eines NHE-Inhibitors der Formel I mit einem Carboanhydrase-Hemmer (z. B. Acetazolamid) kann sich als vorteilhaft erweisen, wobei letzterer eine metabolische Azidose herbeiführt und dadurch bereits die Atmungstätigkeit steigert, so dass eine verstärkte Wirkung und verminderter Wirkstoffeinsatz erzielt werden können.

Die erfindungsgemäßen Verbindungen schonen infolge ihrer NHE3-inhibitorischen Wirkung die zellulären Energiereserven, die sich unter toxischen und pathogenen Ereignissen rasch erschöpfen und so zur Zellschädigung oder zum Zelluntergang führen. Dabei wird die energieaufwendige ATP-verbrauchende Natriumresorption im proximalen Tubulus unter dem Einfluß von NHE3-Inhibitoren vorübergehend still gelegt und die Zelle kann so eine akute pathogene, ischämische oder toxische Situation überleben. Die Verbindungen eignen sich deshalb beispielsweise als Arzneimittel zur Behandlung ischämischer Noxen, zum Beispiel des akuten Nierenversagens. Weiterhin eignen sich die Verbindungen auch zur Behandlung aller chronischen Nierenerkrankungen und Nephritisformen, die infolge vermehrter Proteinausscheidung zum chronischen Nierenversagen führen. Dementsprechend eignen sich die Verbindungen der Formel I zur Herstellung eines Medikaments zur Behandlung diabetischer Spätschäden, der diabetischen Nephropathie und chronischer Nierenerkrankungen, insbesondere von allen Nierenentzündungen (Nephritiden), die mit einer vermehrten Protein-/ Albuminausscheidung verbunden sind.

Es hat sich gezeigt, dass die erfindungsgemäß verwendeten Verbindungen eine milde abführende Wirkung besitzen und demzufolge auch vorteilhaft als Abführmittel oder bei drohender Darmverstopfung verwendet werden können.

Weiterhin können die erfindungsgemäßen Verbindungen vorteilhaft zur Prävention und Therapie von akuten und chronischen Erkrankungen des Darmtrakts verwendet werden, die beispielweise durch ischämische Zustände im Intestinalbereich und / oder durch nachfolgende Reperfusion oder durch entzündliche Zustände und Ereignisse ausgelöst werden. Solche Komplikationen können beispielweise durch mangelnde Darmperistaltik auftreten, wie sie z.B. häufig nach chirurgischen Eingriffen, bei Darmverstopfung oder stark verminderter Darmtätigkeit zu beobachten sind.

Mit den erfindungsgemäßen Verbindungen besteht die Möglichkeit, der Gallenstein-Bildung vorzubeugen.

Die erfindungsgemäßen NHE-Inhibitoren eignen sich generell zur Behandlung von Krankheiten, die durch Ischämie und durch Reperfusion hervorgerufen werden.

Die erfindungsgemäßen Verbindungen sind infolge ihrer pharmakologischen Eigenschaften als antiarrhythmische Arzneimittel geeignet.

Durch ihre cardioprotektive Komponente eignen sich die NHE-Inhibitoren hervorragend zur Infarktprophylaxe und der Infarktbehandlung sowie zur Behandlung der angina pectoris, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäß verwendeten Verbindungen der Formel I infolge Inhibition des zellulären Na⁺/H⁺-Austauschmechanismus als Arzneimittel zur Behandlung aller akuten oder chronischen durch Ischämie ausgelösten Schäden oder dadurch primär oder sekundär induzierten Krankheiten verwendet werden.

Dies betrifft auch ihre Verwendung als Arzneimittel für chirurgische Eingriffe. So können die erfindungsgemäßen Verbindungen bei Organ-Transplantationen verwendet werden, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den mit Verbindungen der Formel I vorbehandelten Empfängerorganismus verwendet werden können.

Die Verbindungen sind ebenfalls wertvolle, protektiv wirkende Arzneimittel bei der Durchführung angioplastischer operativer Eingriffe beispielsweise am Herzen wie auch an peripheren Organen und Gefäßen.

Weiterhin können die erfindungsgemäßen Verbindungen bei der Durchführung von Bypassoperationen verwendet werden, beispielsweise bei Bypassoperationen an Koronargefäßen und bei Coronary Artery Bypass Graft (CABG).

Entsprechend ihrer Wirkung gegen ischämisch induzierte Schäden können die erfindungsgemäßen Verbindungen der Formel I auch bei Wiederbelebung nach einem Herzstillstand eingesetzt werden.

Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z.B. zur Behandlung des Schlaganfalls oder des Hirnödems geeignet sind.

Da NHE-Inhibitoren menschliches Gewebe und Organe nicht nur effektiv gegen Schäden schützen, die durch Ischämie und Reperfusion verursacht werden, sondern auch gegen die cytotoxische Wirkung von Arzneimitteln, wie sie insbesondere in der Krebstherapie und der Therapie von Autoimmunerkrankungen Anwendung finden, ist deren kombinierte Verabreichung mit Verbindungen der Formel geeignet, die cytotoxischen Effekte einer Therapie zu vermindern oder zu unterdrücken. Durch die Verminderung der cytotoxischen Effekte, insbesondere der Cardiotoxizität, infolge Ko-Medikation mit NHE-Inhibitoren kann außerdem die Dosis der cytotoxischen Therapeutika erhöht und / oder die Medikation mit solchen Arzneimitteln verlängert werden. Der therapeutische Nutzen einer solchen cytotoxischen Therapie kann durch die Kombination mit NHE-Inhibitoren erheblich gesteigert werden.

Die Verbindungen der Formel I eignen sich insbesondere zur Verbesserung der Therapie mit Arzneimitteln, die eine unerwünschte cardiotoxische Komponente besitzen.

Generell können die hier beschriebenen NHE Inhibitoren in günstiger Weise mit anderen, den intrazellulären pH-Wert ebenfalls regulierenden Verbindungen kombiniert werden, wobei Inhibitoren der Enzymgruppe der Carboanhydratasen, Inhibitoren der Bicarbonationen transportierenden Systeme wie des Natrium-Bicarbonat-Cotransporters (NBC) oder des Natrium-abhängigen Chlorid-Bicarbonat-Austauschers (NCBE), sowie mit anderen NHE-Inhibitoren mit inhibitorischer Wirkung auf andere NHE-Subtypen, als Kombinationspartner infrage kommen, weil durch sie die pharmakologisch relevanten pH-regulierenden Effekte der hier beschriebenen NHE-Inhibitoren verstärkt oder moduliert werden können.

Entsprechend ihrer protektiven Wirkung gegen ischämisch induzierte Schäden sind die erfindungsgemäßen Verbindungen auch als Arzneimittel zur Behandlung von Ischämien des Nervensystems, insbesondere des Zentralnervensystems, geeignet, wobei sie z. B. zur Behandlung des Schlaganfalls oder des Hirnödems geeignet sind.

Die Verbindungen der Formel I eignen sich auch zur Therapie und Prophylaxe von Erkrankungen und Störungen, die durch Übererregbarkeit des Zentralnervensystems ausgelöst werden, insbesondere zur Behandlung von Erkrankungen des epileptischen Formenkreises, zentral ausgelöster klonischer und tonischer Spasmen, psychischer Depressionszustände, Angsterkrankungen und Psychosen. Dabei können die erfindungsgemäßen NHE-Inhibitoren allein angewandt werden oder in Kombination mit anderen antiepileptisch wirkenden Substanzen oder antipsychotischen Wirkstoffen, oder Carboanhydratasehemmern, beispielweise mit Acetazolamid, sowie mit weiteren Inhibitoren des NHE oder des natrium-abhängigen Chlorid-Bicarbonat-Austauschers (NCBE).

Darüber hinaus eignen sich die erfindungsgemäßen Verbindungen der Formel I ebenfalls zur Behandlung von Formen des Schocks, wie beispielweise des allergischen, cardiogenen, hypovolämischen und des bakteriellen Schocks.

Die Verbindungen der Formel I können ebenfalls zur Prävention und zur Behandlung thrombotischer Erkrankungen verwendet werden, da sie als NHE-Inhibitoren sowohl die Plättchenaggregation selbst inhibieren können. Darüberhinaus können sie die nach Ischämie und Reperfusion stattfindende überschießende Freisetzung von Entzündungs- und Gerinnungsmediatoren, insbesondere des von Willebrand Faktors und thrombogener Selectin-Proteine, hemmen bzw. verhindern. Damit kann die pathogene Wirkung thrombogener und entzündungsrelevanter Faktoren vermindert und ausgeschaltet werden. Deshalb sind die NHE-Inhibitoren der vorliegenden Erfindung kombinierbar mit weiteren antikoagulativen und/oder thrombolytischen Wirkstoffen wie beispielsweise recombinantem oder natürlichen tissue plasminogen activator, Streptokinase, Urokinase, Acetylsalizylsäure, Thrombinantagonisten, Faktor Xa-Antagonisten, fibrinolytisch wirkenden Arzneistoffen, Thromboxan-RezeptorAntagonisten, Phosphodiesterase-Inhibitoren, Faktor-VIIa-Antagonisten, Clopidogrel, Ticlopidin usw. Eine kombinierte Anwendung der vorliegenden NHE-Inhibitoren mit NCBE-Inhibitoren und/oder mit Inhibitoren der Carboanhydratase, wie beispielsweise mit Acetazolamid, ist besonders günstig.

Darüber hinaus zeichnen sich die erfindungsgemäßen NHE-Inhibitoren durch starke inhibierende Wirkung auf die Proliferationen von Zellen, beispielsweise der Fibroblasten-Zellproliferation und der Proliferation der glatten Gefäßmuskelzellen, aus. Deshalb kommen die Verbindungen der Formel I als wertvolle Therapeutika für Krankheiten in Frage, bei denen die Zellproliferation eine primäre oder sekundäre Ursache darstellt, und können deshalb als Antiatherosklerotika, Mittel gegen das chronische Nierenversagen, Krebserkrankungen, verwendet werden. Sie können somit zur Behandlung von Organhypertrophien und -hyperplasien, beispielsweise des Herzens und der Prostata verwendet werden. Verbindungen der Formel I sind deshalb zur Prävention und zur Behandlung der Herzinsuffizienz (congestive heart failure = CHF) wie auch bei der Behandlung und Prävention der Prostatahyperplasie bzw. Prostatahypertrophie geeignet.

NHE-Inhibitoren zeichnen sich weiterhin durch eine Verzögerung oder Verhinderung von fibrotischen Erkrankungen aus. Sie eignen sich somit als ausgezeichnete Mittel zur Behandlung von Fibrosen des Herzens, sowie der Lungenfibrose, Leberfibrose, der Nierenfibrose und anderer fibrotischer Erkrankungen.

Da der NHE bei essentiellen Hypertonikern signifikant erhöht ist, eignen sich die Verbindungen der Formel I zur Prävention und zur Behandlung des Bluthochdrucks und von Herz-Kreislauferkrankungen. Dabei können sie allein oder mit einem geeigneten Kombinationspartner zur Bluthochdruckbehandlung und zur Behandlung von Herz-Kreislauferkrankungen zur Anwendung kommen. So können beispielsweise ein oder mehrere thiazid-artig wirkende Diuretika, Loop-Diuretika, Aldosteron- und Pseudoaldosteron-Antagonisten, wie Hydrochlorothiazid, Indapamid, Polythiazid, Furosemid, Piretanid, Torasemid, Bumetanid, Amilorid, Triamteren, Spironolacton oder Epleron, mit Verbindungen der Formel I kombiniert werden. Weiterhin können die NHE-Inhibitoren der vorliegenden Erfindung in Kombination mit Calcium-Antagonisten, wie Verapamil, Diltiazem, Amlodipin oder Nifedipin, sowie mit ACE-Hemmern, wie beispielsweise Ramipril, Enalapril, Lisinopril, Fosinopril oder Captopril verwendet werden. Weitere günstige Kombinationspartner sind auch ß-Blocker wie Metoprolol, Albuterol usw., Antagonisten des Angiotensin-Rezeptors und seiner Rezeptor-Subtypen wie Losartan, Irbesartan, Valsartan, Omapatrilat, Gemopatrilat, Endothelin-Antagonisten, Renin-Inhibitoren, Adenosin-Rezeptoragonisten, Inhibitoren und Aktivatoren von Kaliumkanälen wie Glibenclamid, Glimepirid, Diazoxid, Cromokalim, Minoxidil und deren Derivate, Aktivatoren des mitochondrialen ATP-sensitiven Kaliumkanals (mitoK(ATP) Kanal), Inhibitoren weiterer Kaliumkanäle, wie des Kv1.5 usw.

Infolge ihrer antiphlogistischen Wirkung können erfindungsgemäße NHE-Inhibitoren als Antiinflammatorika verwendet werden. Mechanistisch fällt dabei die Inhibition der Freisetzung von Entzündungsmediatoren auf. Die Verbindungen können somit allein oder in Kombination mit einem Antiphlogistikum bei der Prävention oder Behandlung chronischer und akuter inflammatorischer Erkrankungen verwendet werden. Als Kombinationspartner werden vorteilhaft steroidale und nicht-steroidale Antiinflammatorika verwendet.

Es wurde außerdem gefunden, dass NHE-Inhibitoren eine günstige Beeinflussung der Serumlipoproteine zeigen. Sie können daher zur Prophylaxe und zur Regression von atherosklerotischen Veränderungen herangezogen werden, indem sie einen kausalen Risikofaktor ausschalten. Hierzu zählen nicht nur die primären Hyperlipidämien, sondern auch gewisse sekundäre Hyperlipidämien, wie sie z.B. beim Diabetes vorkommen. Darüber hinaus führen NHE-Inhibitoren zu einer deutlichen Reduktion der durch Stoffwechselanomalien induzierten Infarkte und insbesondere zu einer signifikanten Verminderung der induzierten Infarktgröße und dessen Schweregrades. NHE-Inhibitoren der Formel I finden deshalb vorteilhaft Verwendung zur Herstellung eines Medikaments zur Behandlung von Hypercholesterinämie; zur Herstellung eines Medikaments zur Prävention der Atherogenese; zur Herstellung eines Medikaments zur Prävention und Behandlung der Atherosklerose, zur Herstellung eines Medikaments zur Prävention und Behandlung von Krankheiten, die durch erhöhte Cholesterinspiegel ausgelöst werden, zur Herstellung eines Medikaments zur Prävention und Behandlung von Krankheiten, die durch endotheliale Dysfunktion ausgelöst werden, zur Herstellung eines Medikaments zur Prävention und Behandlung von atherosklerose-induzierter Hypertonie, zur Herstellung eines Medikaments zur Prävention und Behandlung von atherosklerose-induzierter Thrombosen, zur Herstellung eines Medikaments zur Prävention und Behandlung von Hypercholesterinämie- und endothelialer Dysfunktion induzierter ischämischer Schäden und postischämischer Reperfusionsschäden, zur Herstellung eines Medikaments zur Prävention und zur Behandlung kardialer Hypertrophien und Cardiomyopathien und der congestiven Herzinsuffizienz (CHF), zur Herstellung eines Medikaments zur Prävention und Behandlung von Hypercholesterinämie- und endothelialer Dysfunktion induzierter koronarer Gefäßspasmen und myocardialer Infarkte, zur Herstellung eines Medikaments zur Behandlung der genannten Leiden in Kombinationen mit blutdrucksenkenden Stoffen, bevorzugt mit Angiotensin Converting Enzyme (ACE)-Hemmern und Angiotensin-Rezeptorantagonisten. Eine Kombination eines NHE-Inhibitors der Formel I mit einem blutfettspiegelsenkenden Wirkstoff, bevorzugt mit einem HMG-CoA-Reduktaseinhibitor (z.B. Lovastatin oder Pravastatin), wobei letzterer eine hypolipidämische Wirkung herbeiführt und dadurch die hypolipidämischen Eigenschaften des NHE-Inhibitors der Formel I steigert, stellt eine günstige Kombination mit verstärkter Wirkung und vermindertem Wirkstoffeinsatz dar.

So führen NHE-Inhibitoren zu einen wirksamen Schutz gegen Endothelschädigungen unterschiedlicher Genese. Mit diesem Schutz der Gefäße gegen das Syndrom der endothelialen Dysfunktion sind NHE-Inhibitoren wertvolle Arzneimittel zur Prävention und zur Behandlung koronarer Gefäßspasmen, peripherer Gefäßkrankheiten, insbesondere von claudicatio intermittens, der Atherogenese und der Atherosklerose, der linksventrikulären Hypertrophie und der dilatierten Kardiomyopathie, und thrombotischer Erkrankungen.

Außerdem eignen sich NHE-Inhibitoren zur Behandlung des nicht-insulinabhängigen Diabetes (NIDDM), wobei beispielweise die Insulinresistenz zurückgedrängt wird. Dabei kann es zur Verstärkung von antidiabetischer Wirksamkeit und Wirkqualität der erfindungsgemäßen Verbindungen günstig sein, diese mit einem Biguanid wie Metformin, mit einem antidiabetischen Sulfonylharnstoff, wie Glyburid, Glimepirid, Tolbutamid usw., einem Glucosidase-Inhibitor, einem PPAR-Agonisten, wie Rosiglitazone, Pioglitazone etc, mit einem Insulinpräparat unterschiedlicher Verabreichungsform, mit einem DB4 Inhibitor, mit einem Insulinsensitizer oder mit Meglitinide zu kombinieren.

Neben den akuten antidiabetischen Effekten wirken NHE-Inhibitoren der Entstehung diabetischer Spätkomplikation entgegen und können deshalb als Arzneimittel zur Prävention und Behandlung diabetischer Spätschäden, wie der diabetischen Nephropathie, der diabetischen Neuropathie, der diabetischen Retinopathie, der diabetischen Cardiomyopathie und anderen, als Folge des Diabetes auftretenden Erkrankungen verwendet werden. Dabei können sie mit den oben unter NIDDM-Behandlung beschriebenen antidiabetischen Arzneimitteln vorteilhaft kombiniert werden. Der Kombination mit einer günstigen Darreichungsform von Insulin kann dabei eine besondere Bedeutung zukommen.

NHE-Inhibitoren zeigen neben den protektiven Effekten gegen akute ischämische Ereignisse und die nachfolgenden ebenso akut belastenden Reperfusionsereignisse, auch noch direkte therapeutisch verwertbare Wirkungen gegen Erkrankungen und Störungen des gesamten Säugetierorganismus, die mit den Erscheinungen des chronisch ablaufenden Alterungsprozesses zusammenhängen und die auch unabhängig von akuten Mangeldurchblutungszuständen sind und auch bei normalen, nicht-ischämischen Bedingungen auftreten können. Bei diesen pathologischen, über die lange Zeit des Alterns ausgelösten altersbedingten Erscheinungen wie Krankheit, Siechtum und Tod, die neuerdings mit NHE-Inhibitoren einer Behandlung zugänglich gemacht werden können, handelt es sich um Erkrankungen und Störungen, die maßgeblich durch altersbedingte Veränderungen von lebensnotwendigen Organen und deren Funktion verursacht werden und im alternden Organismus zunehmend an Bedeutung gewinnen.

Erkrankungen, die mit einer altersbedingten Funktionsstörung, mit altersbedingten Verschleißerscheinungen von Organen zusammenhängen, sind beispielsweise die ungenügende Ansprechbarkeit und Reagibilität der Blutgefäße gegenüber Kontraktions- und Relaxationsreaktionen. Diese altersbedingte Abnahme der Gefäßreagibilität auf konstriktorische und relaxierende Reize, die ein essentieller Prozess des Herz-Kreislaufsystems und somit des Lebens und der Gesundheit sind, kann signifikant durch NHE-Inhibitoren aufgehoben bzw. verringert werden. Eine wichtige Funktion und ein Maß für die Aufrechterhaltung der Gefäßreagibilität ist die Blockade bzw. Retardierung der altersbedingt fortschreitenden endothelialen Dysfunktion, die hochsignifikant durch NHE-Inhibitoren aufgehoben werden kann. NHE-Inhibitoren eignen sich somit hervorragend zur Behandlung und Prävention der altersbedingt fortschreitenden endothelialen Dysfunktion, insbesondere von claudicatio intermittens. Außerdem eignen sich NHE-Inhibitoren somit hervorragend zur Behandlung und Prävention der Herzinsuffizienz, des congestive heart failure (CHF) sowie zur Behandlung und insbesondere zur Prävention von altersbedingten Formen von Krebs.

Dabei kommt eine Kombination mit blutdrucksenkenden Medikamenten, wie mit ACE-Hemmern, Angiotensinrezeptorantagonisten, Diuretika, Ca²⁺-Antagonisten etc. oder mit stoffwechselnormalisierenden Medikamenten, wie Cholesterinsenkern, in Betracht. Die Verbindungen der Formel I eignen sich somit zur Prävention altersbedingter Gewebsveränderungen und zur Gesunderhaltung und Lebensverlängerung unter Erhalt einer hohen Lebensqualität.

Die erfindungsgemäßen Verbindungen sind wirkungsvolle Inhibitoren des zellulären Natrium-Protonen-Antiporters (Na/H-Exchanger), der bei zahlreichen Erkrankungen (Essentielle Hypertonie; Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäß verwendeten Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes und diabetischer Spätkomplikationen, proliferativer Erkrankungen usw.

Weiterhin sind NHE-Inhibitoren zur Behandlung von durch Bakterien sowie durch Protozoen ausgelösten Erkrankungen (human und veterinär) geeignet. Bei den durch Protozoen ausgelösten Erkrankungen sind insbesondere Malariaerkrankungen des Menschen und Hühnercoccidiose zu nennen.

Außerdem eignen sich die Verbindungen als Mittel zur Bekämpfung von saugenden Parasiten in der Human- und Veterinärmedizin sowie im Pflanzenschutz. Dabei ist die Verwendung als Mittel gegen blutsaugende Parasiten in der Human- und Veterinärmedizin bevorzugt.

Die genannten Verbindungen finden daher vorteilhaft Verwendung allein oder in Kombination mit anderen Arzneimitteln oder Wirkstoffen zur Herstellung eines Medikaments zur Behandlung oder Prophylaxe von Störungen des Atemantriebs, von Atemstörungen, Schlaf-bedingten Atemstörungen, Schlafapnoen, von Schnarchen, von akuten und chronischen Nierenerkrankungen, des akuten Nierenversagens und des chronischen Nierenversagens, von Störungen der Darmfunktion, des Bluthochdrucks, der essentiellen Hypertonie, von Erkrankungen des Zentralnervensystems, von Erkrankungen, die durch ZNS-Übererregbarkeit resultieren, Epilepsie und zentral ausgelöste Krämpfe oder von Angstzuständen, Depressionen und Psychosen, von ischämischen Zuständen des peripheren oder zentralen Nervensystems oder des Schlaganfalls, von akuten und chronischen Schäden und Erkrankungen peripherer Organe oder Gliedmaßen, die durch Ischämie- oder durch Reperfusionsereignisse verursacht werden, von Atherosklerose, von Störungen des Fettstoffwechsels, von Thrombosen, von Störungen der Gallenfunktion, von Befall durch Ektoparasiten, von Erkrankungen infolge endothelialer Dysfunktion, von Protozoen-Erkrankungen, der Malaria, zur Konservierung und Lagerung von Transplantaten für chirurgische Maßnahmen, für den Einsatz bei chirurgischen Operationen und Organtransplantationen oder zur Behandlung von Schockzuständen oder der Zuckerkrankheit und diabetischer Spätschäden oder von Krankheiten, bei denen die Zellproliferation eine primäre oder sekundäre Ursache darstellt und zur Gesunderhaltung und Lebensverlängerung.

Die Erfindung betrifft weiterhin die Verwendung der Verbindungen der Formel I und deren pharmazeutisch verträgliche Salze zur Verwendung als Medikament.

Die Erfindung betrifft auch Heilmittel für die humane, veterinäre oder phytoprotektive Anwendung enthaltend eine wirksame Menge einer Verbindung der Formel I und / oder eines pharmazeutisch verträglichen Salzes davon, ebenso wie Heilmittel für die humane, veterinäre oder phytoprotektive Anwendung enthaltend eine wirksame Menge einer Verbindung der Formel I und / oder eines pharmazeutisch verträglichen Salzes davon allein oder in Kombination mit einem oder mehreren anderen pharmakologischen Wirkstoffen oder Arzneimitteln.

Arzneimittel, die eine Verbindung der Formel I oder deren pharmazeutisch verträgliche Salze enthalten, können zum Beispiel oral, parenteral, intramuskulär, intravenös, rektal, nasal, durch Inhalation, subkutan oder durch eine geeignete transkutane Darreichungsform appliziert werden, wobei die bevorzugte Applikation von dem jeweiligen Erscheinungsbild der Erkrankung abhängig ist. Die Verbindungen der Formel I können dabei allein oder zusammen mit galenischen Hilfsstoffen zur Anwendung kommen, und zwar sowohl in der Veterinär- als auch in der Humanmedizin und im Pflanzenschutz. Die Arzneimittel enthalten Wirkstoffe der Formel I und/oder deren pharmazeutisch verträgliche Salze im allgemeinen in einer Menge von 0.01 mg bis 1 g pro Dosiseinheit.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wässrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen, perkutanen oder intravenösen Applikation werden die verwendeten aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel. Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.

Im Durchschnitt beträgt die tägliche Dosis einer Verbindung der Formel I bei einem etwa 75 kg schweren Patienten mindestens 0,001 mg/kg, vorzugsweise 0,1 mg/kg, bis höchstens 30 mg/kg, vorzugsweise 1 mg/kg Körpergewicht. In akuten Situationen, etwa unmittelbar nach Erleiden apnoetischer Zustände im Hochgebirge, können auch noch höhere Dosen notwendig werden. Insbesondere bei i.v.-Anwendung, etwa bei einem Infarktpatienten auf der Intensivstation können bis zu 300 mg/kg pro Tag notwendig werden. Die Tagesdosis kann auf eine oder mehrere, zum Beispiel bis zu 4 Einzeldosen verteilt werden.

### Versuchsbeschreibungen und Beispiele

Liste der verwendeten Abkürzungen:
- TFA: Trifluoressigsäure
- HPLC: High Performance Liquid Chromatography
- LCMS: Liquid Chromatography Mass Spectrometry
- Rt: Retentionszeit
- THF: Tetrahydrofuran
- DMSO: Dimethylsulfoxid
- abs.: absolut(es)
- DMF: Dimethylformamid
- ACN: Acetonitril
- min.: Minuten
- h: Stunde(n)
- EDC: N-(3-Dimethylaminopropyl)-N'-ethyl-carbodiimid
- AiBN: 2,2'-Azobis-(2-methyl-propionitril)
- NBS: N-Bromsuccinimid
- CI: Chemische Ionisation
- ESI: Elektrospray Ionisation
- m: Multiplett
- d: Dublett
- s: Singulett

### Allgemeines:

Von den Epimeren an C-4 der Formel I zeigt sich das eine Epimer oft noch aktiver als das andere.

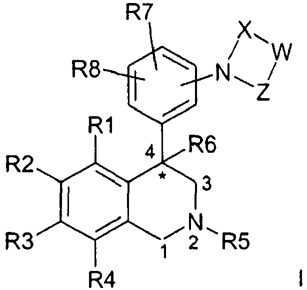

Daher wurden die verwendeten Enantiomere der ortho-, meta- und para-Amine (2-, 3-oder 4-(1,2,3,4-tetrahydro-isoquinolin-4-yl)-phenylamin, beispielsweise 2-, 3- oder 4-(6,8-Dichlor-2-methyl-1,2,3,4-tetrahydro-isochinolin-4-yl)-phenylamin), teilweise an chiraler Phase getrennt, wie in WO2004085404 beschrieben.

Racemische Amine der Formel VIII können, wie in WO2004085404 beschrieben, dargestellt werden, zum Beispiel: racemisches para-Amin: 4-(6,8-Dichloro-2-methyl-1,2,3,4-tetrahydro-isochinolin-4-yl)-phenylamin (Beispiel 1, Zwischenprodukt 6); meta-Amin: 3-(6,8-Dichloro-2-methyl-1,2,3,4-tetrahydro-isochinolin-4-yl)-phenylamin (Beispiel 2, Zwischenprodukt 1), ortho-Amin: 2-(6,8-Dichloro-2-methyl-1,2,3,4-tetrahydro-isochinolin-4-yl)-phenylamin (Beispiel 3, Zwischenprodukt 5) und nachfolgend in ihre Enantiomere, wie in WO2004085404 beschrieben, getrennt werden, zum Beispiel: enantiomerenreines para-Amin: (S)-4-(6,8-Dichloro-2-methyl-1,2,3,4-tetrahydro-isochinolin-4-yl)-phenylamin (Beispiel 42, Zwischenprodukt 1 Enantiomer B); meta-Amin: (S)-3-(6,8-Dichloro-2-methyl-1,2,3,4-tetrahydro-isochinolin-4-yl)-phenylamin (Beispiel 20, Zwischenprodukt ,Enantiomer B); ortho-Amin: (R)-2-(6,8-Dichloro-2-methyl-1,2,3,4-tetrahydro-isochinolin-4-yl)-phenylamin (Beispiel 41, Zwischenprodukt 1,Enantiomer B).

2-(6,8-Dichlor-2-cyclopropyl-1,2,3,4-tetrahydro-isoquinolin-4-yl)-phenylamin und die entsprechenden (R)- und (S)-Enantiomere davon können wie in Beispiel 41 b) beschrieben hergestellt werden.

Die beschriebenen Synthesen wurden, soweit nicht anders erwähnt, in dem Fachmann bekannter Weise unter Schutzgas wie Argon in Standardreaktionsgefäßen wie Ein-, Zwei- oder Dreihalskolben durchgeführt, die wenn erforderlich mit Rührern, Kühlern, Tropftrichtern und Ähnlichem versehen waren. Die Lösungsmittel wurden, falls nicht anders beschrieben, am Rotationsverdampfer im geeigneten Vakuum und bei geeigneter Temperatur abgezogen.

### Bedingungen:

### Präparative HPLC:

Die präparative HPLC wurde unter folgenden Bedingungen durchgeführt:

| | |
|---|---|
| stationäre Phase: | Merck Purospher RP18 (10µM) 250 x 25 mm |
| mobile Phase: | 90% H₂O (0,05% TFA)→ 90% Acetonitril in 40 min; 25 ml/min |

### Analytische HPLC/MS

### HPLC-Methoden

### Methode A:

| | |
|---|---|
| stationäre Phase: | YMC J'sphere ODS H80 20 x 2,1 mm |
| mobile Phase: | 90% H₂O (0,05% TFA)→ 95% Acetonitril in 1,9 min; 95% Acetonitril 0,5 min → 10% Acetonitril in 0,05 min; 1 ml/min. |

### Methode B:

| | |
|---|---|
| stationäre Phase: | YMC J'sphere ODS H80 20 x 2,1 mm |
| mobile Phase: | 96% H₂O (0,05% TFA)→ 95% Acetonitril in 2,0 min; 95% Acetonitril 0,4 min → 4% Acetonitril in 0,05 min; 1 ml/min. |

### Massenspektrometrie

Das Massenspektrometer war direkt an die HPLC gekoppelt (LCMS). Als Ionisationsmethode, falls nicht anders erwähnt, wurde Elektrospray (ESI⁺) verwandt. Die angegebenen Retentionszeiten beziehen sich auf das Signalmaximum des Ionenstroms der entsprechenden Verbindung, wie sie in der LCMS-Kopplung mit obigen HPLC-Bedingungen erhalten wurden.

### Beispiel 1: 3-[4-(6,8-Dichlor-2-methyl-1,2,3,4-tetrahydro-isochinolin-4-yl)-phenyl]-imidazolidin-2,4-dion

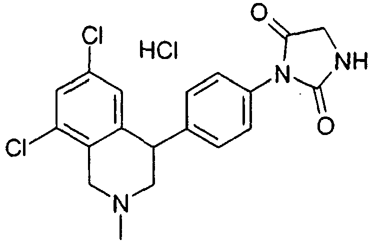

a) {3-[4-(6,8-Dichlor-2-methyl-1,2,3,4-tetrahydro-isochinolin-4-yl)-phenyl]-ureido}-essigsäureethylester-Hydrochlorid; 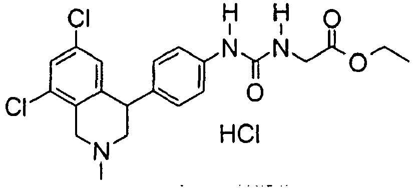 4-(6,8-Dichlor-2-methyl-1,2,3,4-tetrahydro-isochinolin-4-yl)-phenylamin (95 mg; Darstellung wie in WO2004085404 beschrieben) wurde in Acetonitril (2 ml) gelöst und unter Rühren Ethylisocyanatoacetat (30 mg) zugetropft. Nach vier Stunden wurde die Lösung eingeengt und der Rückstand mittels präparativer HPLC gereinigt. Die Produkt enthaltenden Fraktionen wurden vereinigt, das Acetonitril am Rotationsverdampfer abgezogen, der wässrige Rückstand mit Kaliumcarbonat neutralisiert und drei Mal mit Essigester extrahiert. Nach Trocknen über Magnesiumsulfat wurde zur Trockne gebracht. Der Rückstand wurde mit wässriger Salzsäure aufgenommen und gefriergetrocknet. Es wurden 107 mg der gewünschten Verbindung erhalten. LCMS-Rt (A): 1,14 min;
   [M+H⁺]: 436,5
b) 3-[4-(6,8-Dichlor-2-methyl-1,2,3,4-tetrahydro-isochinolin-4-yl)-phenyl]-imidazolidin-2,4-dion
   {3-[4-(6,8-Dichlor-2-methyl-1,2,3,4-tetrahydro-isochinolin-4-yl)-phenyl]-ureido}-essigsäureethylester-Hydrochlorid (30 mg) wurde vorgelegt. Wasser (3 ml) und 10%ige Salzsäure (231 µl) wurden zugegeben und unter Rühren anschließend drei Stunden rückflussiert. Nach Abkühlen und Gefriertrocknen der Reaktionslösung wurde ein weißer Feststoff erhalten.
   LCMS-Rt (A): 0,98 min;
   [M+H⁺]: 390,4

### Beispiel 2: 3-[4-((S)-6,8-Dichlor-2-methyl-1,2,3,4-tetrahydro-isochinolin-4-yl)-phenyl]-imidazolidin-2,4-dion-Hydrochlorid

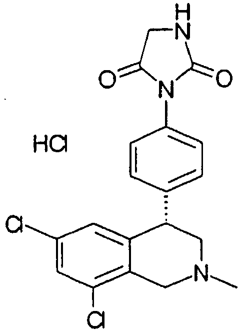

Beispiel 2 wurde analog Beispiel 1 synthetisiert. Das dafür benötigte (S)-4-(6,8-Dichlor-2-methyl-1,2,3,4-tetrahydro-isochinolin-4-yl)-phenylamin (Enantiomer B, Darstellung wie in WO2004085404 beschrieben) wurde aus dem Racemat durch Trennung an chiraler Phase erhalten.
LCMS-Rt (B): 0,89 min;
[M+H⁺]: 390,1

### Beispiel 3: 3-[3-(6,8-Dichlor-2-methyl-1,2,3,4-tetrahydro-isochinolin-4-yl)-phenyl]-imidazolidin-2,4-dion

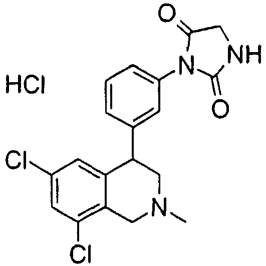

Beispiel 3 wurde analog Beispiel 1 synthetisiert. Das dafür benötigte 3-(6,8-Dichlor-2-methyl-1,2,3,4-tetrahydro-isochinolin-4-yl)-phenylamin wurde, wie in WO2004085404 beschrieben dargestellt.
LCMS-Rt (A): 0,96 min;
[M+H⁺]: 390,3

### Beispiel 4: 3-[3-((S)-6,8-Dichlor-2-methyl-1,2,3,4-tetrahydro-isochinolin-4-yl)-phenyl]-imidazolidin-2,4-dion-Hydrochlorid

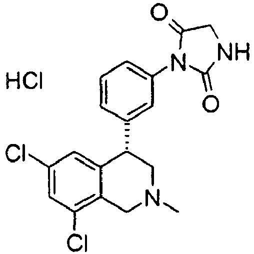

Beispiel 4 wurde analog Beispiel 1 synthetisiert. Das dafür benötigte (S)-3-(6,8-Dichlor-2-methyl-1,2,3,4-tetrahydro-isochinolin-4-yl)-phenylamin (Enantiomer B, Darstellung wie in WO2004085404 beschrieben) wurde aus dem Racemat durch Trennung an chiraler Phase erhalten.
LCMS-Rt (B): 0,90 min;
[M+H⁺]: 390,0

### Beispiel 5: 3-[2-(6,8-Dichlor-2-methyl-1,2,3,4-tetrahydro-isochinolin-4-yl)-phenyl]-imidazolidin-2,4-dion-Hydrochlorid

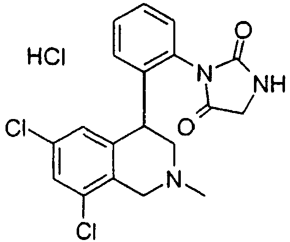

Beispiel 5 wurde analog Beispiel 1 synthetisiert. Das dafür benötigte 2-(6,8-Dichlor-2-methyl-1,2,3,4-tetrahydro-isochinolin-4-yl)-phenylamin wurde, wie in WO2004085404 beschrieben, erhalten.
LCMS-Rt (A): 1,04 min;
[M+H⁺]: 390,4

### Beispiel 6: 3-[2-((R)-6,8-Dichlor-2-methyl-1,2,3,4-tetrahydro-isochinolin-4-yl)-phenyl]-imidazolidin-2,4-dion-Hydrochlorid

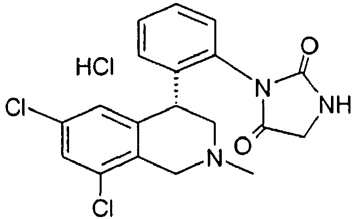

Beispiel 6 wurde analog Beispiel 1 synthetisiert. Das dafür benötigte (R)-2-(6,8-Dichlor-2-methyl-1,2,3,4-tetrahydro-isochinolin-4-yl)-phenylamin (Enantiomer B, Darstellung wie in WO2004085404 beschrieben) wurde aus dem Racemat durch Trennung an chiraler Phase erhalten.
LCMS-Rt (B): 0,98 min;
[M+H⁺]: 390,0

### Beispiel 7: 1-[2-((R)-6,8-Dichlor-2-methyl-1,2,3,4-tetrahydro-isochinolin-4-yl)-phenyl]-pyrrolidin-2,5-dion-Hydrochlorid

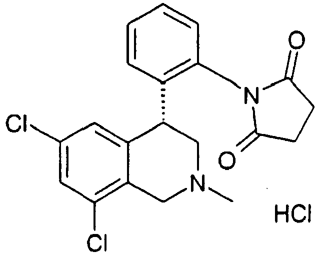

(R)-2-(6,8-Dichlor-2-methyl-1,2,3,4-tetrahydro-isochinolin-4-yl)-phenylamin (100 mg; Enantiomer B, Darstellung wie in WO2004085404 beschrieben) wurde in einem Kolben zusammen mit Bernsteinsäure (58 mg) vorgelegt. Dazu wurde Polyphosphorsäure (ca. 2 ml) gegeben. Das Reaktionsgemisch wurde anschließend bei 135 °C gerührt. Nach 4 Stunden wurde noch etwas Bernsteinsäure (∼9 mg) zugegeben. Nach weiteren 2 h bei 135°C wurde über Nacht bei Raumtemperatur stehen gelassen. Zur Aufarbeitung wurde auf Eiswasser gegossen, die saure Phase mit gesättigter Kaliumcarbonat-Lösung auf pH 10 gestellt und dann drei Mal mit Essigester extrahiert. Die vereinigten organischen Phasen wurden ein Mal mit Wasser gewaschen, über Magnesiumsulfat getrocknet, filtriert und eingeengt. Das Rohprodukt wurde mittels präparativer HPLC gereinigt. Die Produkt enthaltenden Fraktionen wurden vereinigt, das Acetonitril am Rotationsverdampfer abgezogen, der wässrige Rückstand mit Kaliumcarbonat neutralisiert und drei Mal mit Essigester extrahiert. Nach Trocknen über Magnesiumsulfat wurde filtriert und zur Trockne gebracht. Der Rückstand wurde mit wässriger Salzsäure aufgenommen und gefriergetrocknet. LCMS-Rt (B): 1,03 min;
[M+H⁺]: 389,0

### Beispiel 8: 6,8-Dichlor-4-[4-(1,1-dioxo-1-λ⁶-[1,2,5]thiadiazolidin-2-yl)-phenyl]-2-methyl-1,2,3,4- tetrahydro-isochinolin

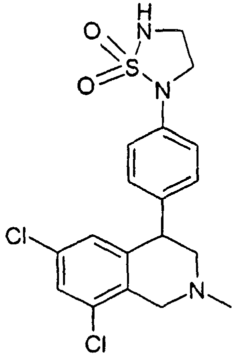

2-Chlorethylamin-Hydrochlorid (75 mg) wurde mit Sulfurylchlorid (0,32 ml) in Acetonitril (10 ml) über Nacht bei 75-80 °C gerührt. Das auf Raumtemperatur abgekühlte Gemisch wurde eingeengt und der Rückstand mit absolutem Ether (1 ml) aufgenommen. Die das Chlorsulfonamid enthaltende Etherphase wurde zu einer Lösung aus 4-(6,8-Dichlor-2-methyl-1,2,3,4-tetrahydro-isochinolin-4-yl)-phenylamin (110 mg, Darstellung wie in WO2004085404 beschrieben), absolutem Diethylether (1 ml) und Triethylamin (85 µl) bei -70°C getropft. Nach beendeter Zugabe ließ man das Reaktionsgemisch auf Raumtemperatur kommen und rührte 2,5 h nach. Anschließend wurde mit einem Gemisch aus Wasser und gesättigter Natriumhydrogencarbonat-Lösung (3:1) versetzt und noch etwas Ether zugegeben. Die Phasen wurden getrennt und die wässrige Phase noch zwei Mal mit Diethylether extrahiert. Die vereinigten Etherphasen wurden über Magnesiumsulfat getrocknet, filtriert und eingeengt.

Das Rohprodukt wurde mittels präparativer HPLC gereinigt. Die Produkt enthaltenden Fraktionen wurden vereinigt, das Acetonitril am Rotationsverdampfer abgezogen, der wässrige Rückstand mit Kaliumcarbonat neutralisiert und drei Mal mit Dichlormethan extrahiert. Nach Trocknen über Magnesiumsulfat wurde filtriert und zur Trockne gebracht.Ein Teil des so gewonnenen Produkts (20 mg) wurden in DMSO (0,5 ml) gelöst, mit Kaliumcarbonat (6,2 mg) versetzt und sechs Stunden bei Raumtemperatur gerührt. Nach Lagern im Tiefkühlschrank über Nacht wurde das DMSO im Hochvakuum entfernt und der Rückstand mit wenig Wasser, gesättigter Natriumhydrogencarbonat-Lösung und Ether versetzt. Die Phasen wurden getrennt und die wässrige Phase noch zwei Mal mit Diethylether extrahiert. Die vereinigten Etherphasen wurden über Magnesiumsulfat getrocknet, filtriert und eingeengt.

Das Rohprodukt wurde mittels präparativer HPLC gereinigt. Die Produkt enthaltenden Fraktionen wurden vereinigt, das Acetonitril am Rotationsverdampfer abgezogen, der wässrige Rückstand mit Kaliumcarbonat neutralisiert und drei Mal mit Dichlormethan extrahiert. Nach Trocknen über Magnesiumsulfat wurde filtriert und zur Trockne gebracht. Der Rückstand wurde mit wässriger Salzsäure aufgenommen und gefriergetrocknet.
LCMS-Rt (B): 1,02 min;
[M+H⁺]: 412,1

Ausgehend von den entsprechenden Aminen, die gegebenenfalls enatiomerenrein eingesetzt wurden, und den entsprechenden Dicarbonsäuren wurden analog die folgenden Verbindungen hergestellt.

| Beispiel | Struktur | Salz | Analog Beispiel | MS [M+H⁺] | LCMS-Rt [min] |
|---|---|---|---|---|---|
| 9 | 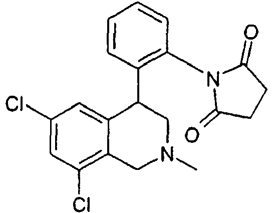 | HCl | 7 | 389,0 (ESI⁺) | 1,03 (B) |
| 10 | 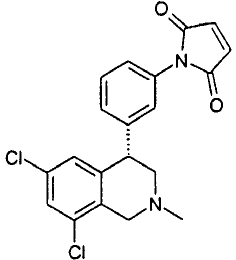 | HCl | 7 | 387,0 (ESI⁺) | 1,05 (B) |
| 11 | 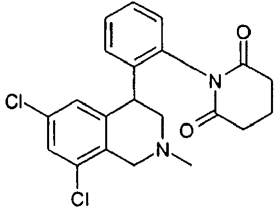 | HCl | 7 | 403,0 (ESI⁺) | 1,06 (B) |
| 12 | 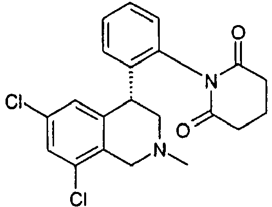 | HCl | 7 | 403,0 (ESI⁺) | 1,04 (B) |
| 13 | 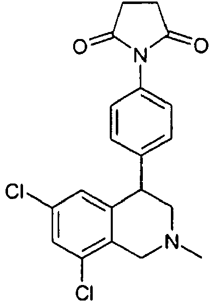 | HCl | 7 | 389,0 (ESI⁺) | 0,97 (B) |
| 14 | 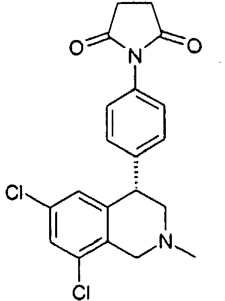 | HCl | 7 | 389,0 (ESI⁺) | 0,96 (B) |
| 15 | 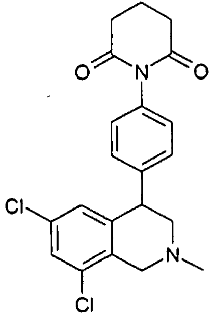 | HCl | 7 | 403,1 (ESI⁺) | 0,97 (B) |
| 16 | 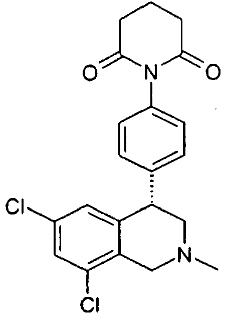 | HCl | 7 | 403,1 (ESI⁺) | 0,96 (B) |
| 17 | 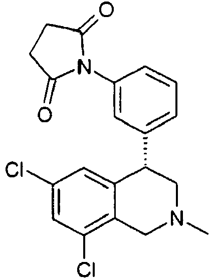 | HCl | 7 | 389,1 (ESI⁺) | 0,97 (B) |
| 18 | 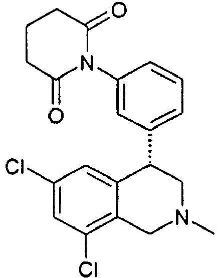 | HCl | 7 | 403,1 (ESI⁺) | 0,98 (B) |
| 19 | 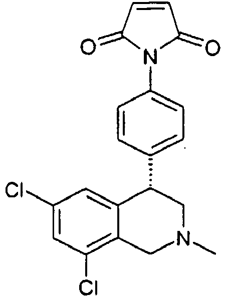 | HCl | 7 | 387,0 (ESI⁺) | 1,05 (B) |
| 20 | 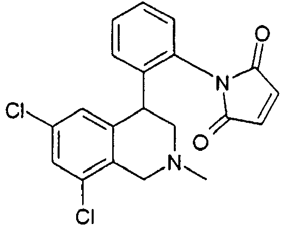 | HCl | 7 | 387,0 (ESI⁺) | 1,12 (B) |
| 21 | 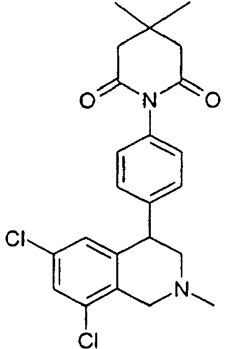 | HCl | 7 | 431,1 (ESI⁺) | 1,20 (B) |
| 22 | 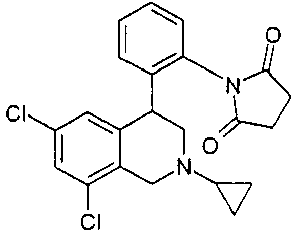 | HCl | 7 | 415,0 (ESI⁺) | 1,19 (B) |
| 23 | 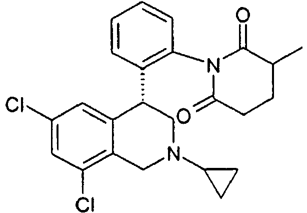 | HCl | 7 | 443,1 (ESI⁺) | 1,31 (B) |
| 24 | 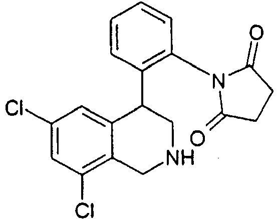 | TFA | 7 | 375,0 (ESI⁺) | 1,04 (B) |
| 25 | 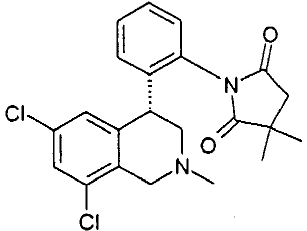 | TFA | 7 | 417,0 (ESI⁺) | 1,15 (B) |
| 26 | 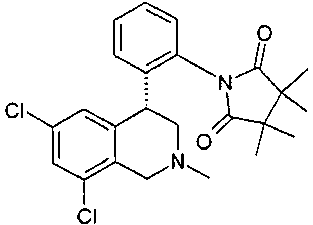 | TFA | 7 | 445,1 (ESI⁺) | 1,24 (B) |
| 27 | 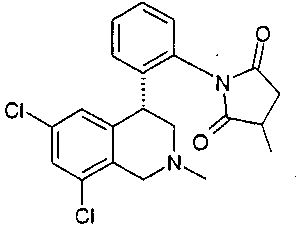 | HCl | 7 | 403,0 (ESI⁺) | 1,07 (B) |
| 28 | 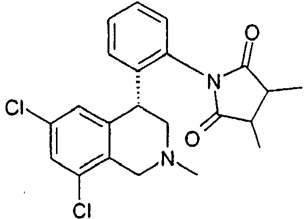 | TFA | 7 | 417,0 (ESI⁺) | 1,16 (B) |
| 29 | 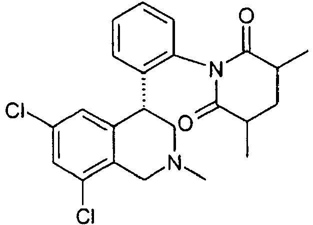 | TFA | 7 | 431,1 (ESI⁺) | 1,19 (B) |
| 30 | 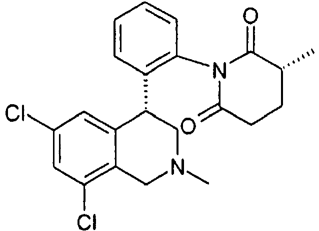 | TFA | 7 | 417,0 (ESI⁺) | 1,15 (B) |
| 31 | 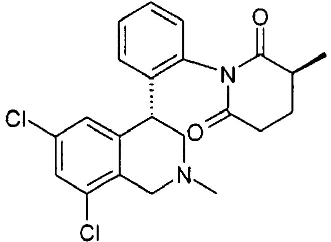 | TFA | 7 | 417,0 (ESI⁺) | 1,13 (B) |
| 32 | 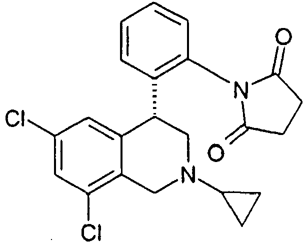 | HCl | 7 | 415,0 (ESI⁺) | 1,21 (B) |
| 33 | 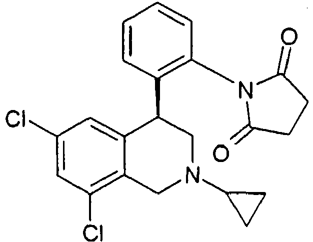 | HCl | 7 | 415,0 (ESI⁺) | 1,21 (B) |
| 34 | 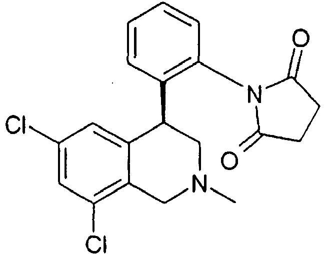 | HCl | 7 | 389,0 (ESI⁺) | 1,06 (B) |

### Beispiel 35: 1-[2-((R)-6,8-Dichlor-2-methyl-1,2,3,4-tetrahydro-isochinolin-4-yl)-phenyl]-pyrrolidin-2-on-Trifluoracetat

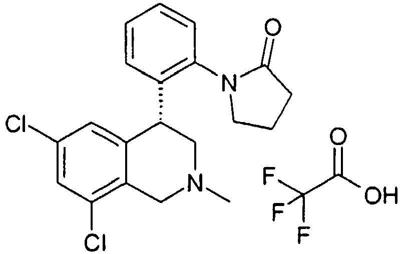

### a) N-[2-((R)-6,8-Dichlor-2-methyl-1,2,3,4-tetrahydro-isochinolin-4-yl)-phenyl]-4-hydroxy-butyramid

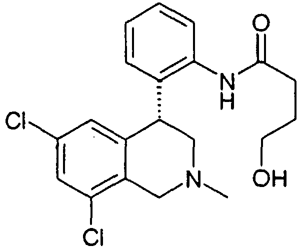

(R)-2-(6,8-Dichlor-2-methyl-1,2,3,4-tetrahydro-isochinolin-4-yl)-phenylamin (100 mg, Enantiomer B, Darstellung wie in WO2004085404 beschrieben) wurde mit *γ-*Butyrolacton (84 mg) in abs. THF (8 ml) vorlegt und dann Natriumhexamethyldisilazid (NaHMDS; 2 M/THF; 0,65 ml) bei 0°C innerhalb von 15 min. zugetropft. Nach 10 min. Rühren bei 0°C wurde das Eisbad entfernt und weitere 2 h gerührt. Die Reaktion wurde mit gesättigter Ammoniumchlorid-Lösung (0,6 ml), Essigester und Wasser versetzt. Die Phasen wurden getrennt und die wässrige Phase zwei Mal mit Essigester extrahiert. Die vereinigten organischen Phasen wurden ein Mal mit gesättigter Kochsalz-Lösung gewaschen, über Magnesiumsulfat getrocknet, filtriert und eingeengt. Das Rohprodukt wurde mittels präparativer HPLC gereinigt. Die Produkt enthaltenden Fraktionen wurden vereinigt, das Acetonitril am Rotationsverdampfer abgezogen, der wässrige Rückstand mit Kaliumcarbonat neutralisiert und drei Mal mit Dichlormethan extrahiert. Nach Trocknen der vereinigten Dichlormethan-Phasen über Magnesiumsulfat wurde filtriert und zur Trockne gebracht.
LCMS-Rt (B): 0,98 min;
[M+H⁺]: 393,1

b) 1-[2-((R)-6,8-Dichlor-2-methyl-1,2,3,4-tetrahydro-isochinolin-4-yl)-phenyl]-pyrrolidin-2-on-Trifluoracetat N-[2-((R)-6,8-Dichlor-2-methyl-1,2,3,4-tetrahydro-isochinolin-4-yl)-phenyl]-4-hydroxy- butyramid (100 mg) wurde in absolutem THF (4 ml) gelöst, und Triethylamin (77 µl) und eine Lösung aus Methansulfonsäurechlorid (44 µl) in THF (0,5 ml) zugetropft. Nach sechs Stunden Rühren bei Raumtemperatur wurde das THF abgezogen und der Rückstand mit gesättigter Natriumhydrogencarbonat-Lösung versetzt. Nach dreimaligem Extrahieren mit Essigester wurden die vereinigten organischen Phasen über Magnesiumsulfat getrocknet, filtriert und eingeengt. Ein Teil des Rückstands (44 mg) wurde in absolutem THF (2 ml) gelöst und das Reaktionsgemisch auf 0°C abgekühlt. Bei dieser Temperatur wurde innerhalb 10 min. Natriumhexamethyldisilazid (96 µl) zugetropft. Anschließend wurde noch 10 min. bei 0°C gerührt, bevor das Eisbad entfernt wurde. Nach 1,5 h wurde die Reaktion mit gesättigter Ammoniumchlorid-Lösung (0,2 ml) und Wasser versetzt. Dann wurde drei Mal mit Ethylacetat extrahiert. Die vereinigten organischen Phasen wurden über Magnesiumsulfat getrocknet, filtriert und eingeengt. Das Rohprodukt wurde mittels präparativer HPLC gereinigt. Die Produkt enthaltenden Fraktionen wurden vereinigt, das Acetonitril am Rotationsverdampfer abgezogen, der wässrige Rückstand mit Kaliumcarbonat neutralisiert und drei Mal mit Dichlormethan extrahiert. Nach Trocknen der vereinigten Dichlormethan-Phasen über Magnesiumsulfat wurde filtriert und zur Trockne gebracht.
LCMS-Rt (B): 1,04 min;
[M+H⁺]: 375,1

### Beispiel 36: 1-[2-((R)-6,8-Dichlor-2-methyl-1,2,3,4-tetrahydro-isochinolin-4-yl)-phenyl]-pyrrolidin-2-on-Hydrochlorid

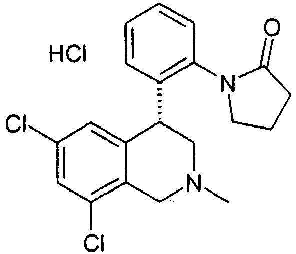

### a) 4-Chlor-N-[2-((R)-6,8-dichlor-2-methyl-1,2,3,4-tetrahydro-isochinolin-4-yl)-phenyl]-butyramid

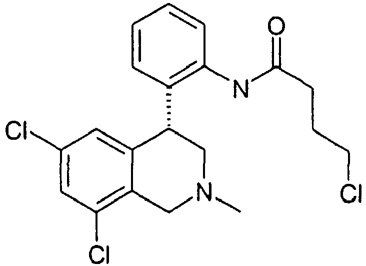

2-((R)-6,8-Dichlor-2-methyl-1,2,3,4-tetrahydro-isochinolin-4-yl)-phenylamin (250 mg) wurden in abs. THF (20 ml) gelöst. 4-Chlorbutyrylchlorid (115 mg) und Triethylamin (208 µl) wurden unter Rühren zugegeben. Es wurde ca. 3 h bei Raumtemperatur gerührt. Nach Stehen über Nacht wurde das Reaktionsgemisch eingeengt und der Rückstand mittels präparativer HPLC gereinigt. Die Produkt enthaltenden Fraktionen wurden vereinigt, das Acetonitril am Rotationsverdampfer abgezogen, der wässrige Rückstand mit Natriumhydrogencarbonat neutralisiert und drei Mal mit Essigester extrahiert. Nach Trocknen der vereinigten Essigester-Phasen über Magnesiumsulfat wurde filtriert und zur Trockne gebracht. Es wurden 290 mg eines öligen Produkts erhalten.
LCMS-Rt (B): 1,14 min;
[M+H⁺]: 411,0

b) 1-[2-((R)-6,8-Dichlor-2-methyl-1,2,3,4-tetrahydro-isochinolin-4-yl)-phenyl]-pyrrolidin-2-on-Hydrochlorid 4-Chlor-N-[2-((R)-6,8-dichlor-2-methyl-1,2,3,4-tetrahydro-isochinolin-4-yl)-phenyl]-butyramid (249 mg) wurde in abs. DMSO (10 ml) gelöst und fein gepulvertes und getrocknetes Kaliumcarbonat (250 mg) zugegeben. Unter Feuchtigkeitsausschluss wurde bei Raumtemperatur 4 h kräftig gerührt. Nach Stehen über Nacht wurde das Reaktionsgemisch mit Wasser (50 ml) verdünnt und dann drei Mal mit Essigester extrahiert. Die vereinigten Extrakte wurden über Magnesiumsulfat getrocknet, filtriert und eingeengt. Der Rückstand wurde mittels präparativer HPLC gereinigt. Die Produkt enthaltenden Fraktionen wurden vereinigt, das Acetonitril am Rotationsverdampfer abgezogen, der wässrige Rückstand mit
Natriumhydrogencarbonat neutralisiert und drei Mal mit Essigester extrahiert. Nach Trocknen der vereinigten Essigester-Phasen über Magnesiumsulfat wurde filtriert und zur Trockne gebracht. Der Rückstand wurde in Wasser / Acetonitril gelöst und mit 0,1 N HCl auf pH 2 gestellt. Nach Gefriertrocknung über Nacht wurde das gewünschte Produkt (116 mg) als weißes Pulver erhalten.
LCMS-Rt (B): 1,06 min;
[M+H⁺]: 375,1

### Beispiel 37: 1-[2-((R)-6,8-Dichlor-2-cyclopropyl-1,2,3,4-tetrahydro-isochinolin-4-yl)-phenyl]-pyrrolidin-2-on

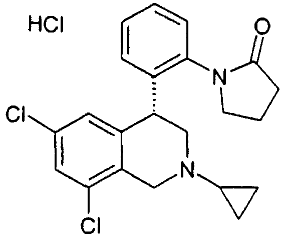

Die Titelverbindung wurde ausgehend von 2-((R)-6,8-Dichlor-2-cyclopropyl-1,2,3,4-tetrahydro-isochinolin-4-yl)-phenylamin (Beispiel 41 b) analog Beispiel 36 synthetisiert. LCMS-Rt (B): 1,12 min;
[M+H⁺]: 401,0

### Beispiel 38: 1-[3-((S)-6,8-Dichlor-2-methyl-1,2,3,4-tetrahydro-isochinolin-4-yl)-phenyl]-piperidin-2-on

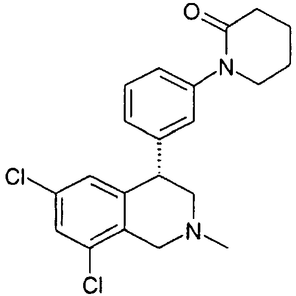

Ausgehend von (S)-3-(6,8-Dichlor-2-methyl-1,2,3,4-tetrahydro-isochinolin-4-yl)-phenylamin (Enantiomer B, Darstellung wie in WO2004085404 beschrieben) und δ-Valerolacton wurde 1-[3-((S)-6,8-Dichlor-2-methyl-1,2,3,4-tetrahydro-isochinolin-4-yl)-phenyl]-piperidin-2-on analog Beispiel 35 synthetisiert.
LCMS-Rt (B): 1,05 min;
[M+H⁺]: 389,1

### Beispiel 39: 1-[2-((R)-6,8-Dichlor-2-methyl-1,2,3,4-tetrahydro-isochinolin-4-yl)-phenyl]-imidazolidin-2-on-Hydrochlorid

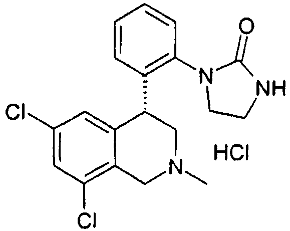

### a) 1-(2-Chlorethyl)-3-[2-((R)-6,8-dichlor-2-methyl-1,2,3,4-tetrahydro-isochinolin-4-yl)-phenyl]-Harnstoff

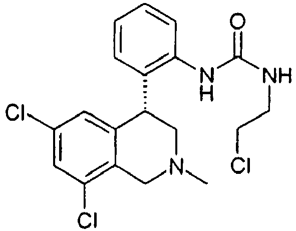

(R)-2-(6,8-Dichlor-2-methyl-1,2,3,4-tetrahydro-isochinolin-4-yl)-phenylamin (100 mg, Enantiomer B, Darstellung wie in WO2004085404 beschrieben) wurde in absolutem Dichlormethan (5 ml) gelöst und unter Rühren mit 4-Nitrophenylchloroformat (79 mg) versetzt. Nach 4 h Rühren bei Raumtemperatur wurde über Nacht stehen gelassen. Am nächsten Tag wurde weiteres 4-Nitrophenylchloroformat (10 mg) zugeben und 1 h bei Raumtemperatur gerührt. Das Reaktionsgemisch wurde im Vakuum zur Trockne gebracht und der Rückstand in absolutem Dichlormethan (5 ml) gelöst. Nach Zugabe von Triethylamin (180 µl) wurde eine Lösung aus 2-Chlorethylamin-Hydrochlorid (61 mg) in absolutem Dichlormethan (3 ml) zugetropft und vier Stunden bei Raumtemperatur gerührt. Anschließend wurde mit weiterem Methylenchlorid und verdünnter Kaliumcarbonat-Lösung versetzt und die organische Phase drei Mal mit verdünnter Kaliumcarbonat-Lösung extrahiert. Nach Trocknen über Magnesiumsulfat und Filtrieren wurde die organische Phase eingeengt und mittels präparativer HPLC gereinigt. Die Produkt enthaltenden Fraktionen wurden vereinigt, das Acetonitril am Rotationsverdampfer abgezogen, der wässrige Rückstand mit gesättigter Natriumhydrogencarbonat-Lösung neutralisiert und drei Mal mit Essigester extrahiert. Nach Trocknen der vereinigten Essigester-Phasen über Magnesiumsulfat wurde filtriert und zur Trockne gebracht. Es wurden 50 mg des gewünschten Produkts erhalten.
LCMS-Rt (B): 1,05 min;
[M+H⁺]: 412,1

### b) 1-[2-((R)-6,8-Dichlor-2-methyl-1,2,3,4-tetrahydro-isochinolin-4-yl)-phenyl]-imidazolidin-2-on-Hydrochlorid

1-(2-Chlorethyl)-3-[2-((R)-6,8-dichlor-2-methyl-1,2,3,4-tetrahydro-isochinolin-4-yl)-phenyl]-Harnstoff (40 mg) wurde in absolutem DMSO (1 ml) gelöst und unter Rühren mit Kaliumcarbonat (14 mg) versetzt. Nach vier Stunden Rühren bei Raumtemperatur wurde noch etwas Kaliumcarbonat (7 mg) nachgegeben. Nach Stehenlassen über Nacht wurde das DMSO im Vakuum abgezogen, der Rückstand mit wenig Wasser und gesättigter Natriumhydrogencarbonat-Lösung versetzt und das entstandene Gemisch anschließend drei Mal mit Dichlormethan extrahiert. Die vereinigten organischen Phasen wurden über Magnesiumsulfat getrocknet, filtriert und eingeengt. Der Rückstand wurde mittels präparativer HPLC aufgereinigt, wobei allerdings eine Verunreinigung nicht abgetrennt werden konnte, so dass eine weitere Chromatographie auf Kieselgel folgte (Dichlormethan/Methanol 100/0 auf 88/12 in 80 min. Die sauberen Fraktionen wurden vereinigt, zur Trockne gebracht und der Rückstand anschließend mit Wasser und etwas Salzsäure gefriergetrocknet. LCMS-Rt (B): 1,00 min; [M+H⁺]: 376,0

### Beispiel 40: 1-[2-((R)-6,8-Dichlor-2-methyl-1,2,3,4-tetrahydro-isochinolin-4-yl)-phenyl]-1,3-dihydro-imidazol-2-on-Hydrochlorid

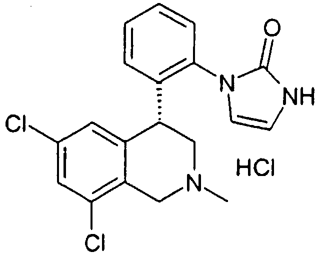

### a) 1-[2-((R)-6,8-Dichlor-2-methyl-1,2,3,4-tetrahydro-isoquinolin-4-yl)-phenyl]-3-(2,2-diethoxy-ethyl)-Harnstoff

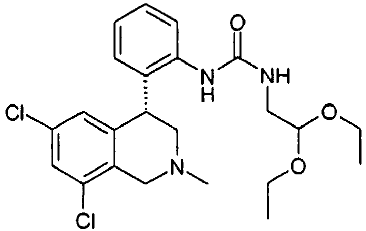

(R)-2-(6,8-Dichlor-2-methyl-1,2,3,4-tetrahydro-isochinolin-4-yl)-phenylamin (75 mg, Enantiomer B, Darstellung wie in WO2004085404 beschrieben) wurde analog zu Beispiel 25 a) mit 4-Nitrophenylchloroformat (64 mg) und anschließend mit 2,2-Diethoxy-ethylamin (46 mg) umgesetzt.
LCMS-Rt (B): 1,14 min;
[M+H⁺]: 466,1

### b) 1-[2-((R)-6,8-Dichlor-2-methyl-1,2,3,4-tetrahydro-isochinolin-4-yl)-phenyl]-1,3-dihydro-imidazol-2-on-Hydrochlorid

1-[2-((R)-6,8-Dichlor-2-methyl-1,2,3,4-tetrahydro-isoquinolin-4-yl)-phenyl]-3-(2,2-diethoxy-ethyl)-Harnstoff (65 mg) wurde in Ameisensäure (0,4 ml) gelöst und 2 Stunden bei Raumtemperatur gerührt. Anschließend wurde mit Wasser versetzt und mit gesättigter Natriumhydrogencarbonat-Lösung neutralisiert. Nach dreimaligem Extrahieren mit Essigester wurden die vereinigten organischen Phasen über Magnesiumsulfat getrocknet, filtriert, einengt und mittels präparativer Chromatographie gereinigt. Die Produkt enthaltenden Fraktionen wurden vereinigt, das Acetonitril am Rotationsverdampfer abgezogen, der wässrige Rückstand mit gesättigter Natriumhydrogencarbonat-Lösung neutralisiert und drei Mal mit Essigester extrahiert. Nach Trocknen der vereinigten Essigester-Phasen über Magnesiumsulfat wurde filtriert und zur Trockne gebracht. Der Rückstand wurde mit wässriger HCl aufgenommen und gefriergetrocknet.
LCMS-Rt (B): 1,05 min;
[M+H⁺]: 374,0

### Beispiel 41: 3-[2-((R)-6,8-Dichlor-2-cyclopropyl-1,2,3,4-tetrahydro-isochinolin-4-yl)-phenyl]-thiazolidin-2,4-dion

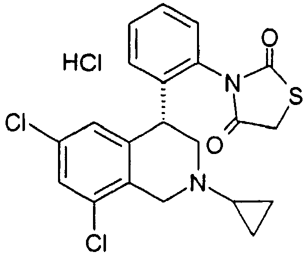

### a) 1-(2-Amino-phenyl)-2-[cyclopropyl-(2,4-dichlor-benzyl)-amino]-ethanol und 2-{2-[Cyclopropyl-(2,4-dichlor-benzyl)-amino]-1-methoxy-ethyl}-phenylamin

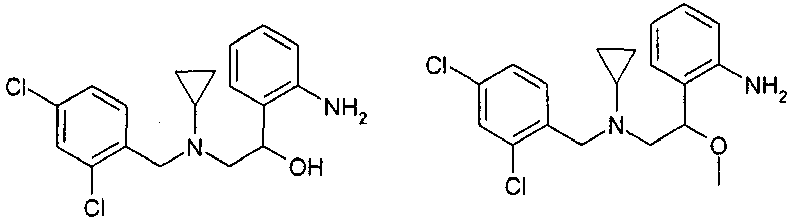

N-(2-{2-[Cyclopropyl-(2,4-dichlor-benzyl)-amino]-1-hydroxy-ethyl}-phenyl)-acetamid (46 g, erhalten analog zu Beispiel 15 in WO 03 48129) wurde in Methanol (250 ml) gelöst. Unter Rühren wurde 30 %ige Natriummethylatlösung dazu gegeben und dann das Gemisch 10 h rückflussiert. Zur Vervollständigung der Reaktion wurde festes Natriummethylat (10 g) zugegeben und weitere 4 h rückflussiert. Zur Aufarbeitung wurde das Reaktionsgemisch anschließend auf 1,5 l Eiswasser gegeben und drei Mal mit Essigester eluiert. Die vereinigten Essigesterphasen wurden einmal mit gesättigter Kochsalzlösung gewaschen, dann mit Magnesiumsulfat getrocknet, filtriert und eingeengt. Nach chromatographischer Aufreinigung an Kieselgel wurden 14 g 1-(2-Amino-phenyl)-2-[cyclopropyl-(2,4-dichlor-benzyl)-amino]-ethanol LCMS-Rt (B): 1,03 min;
[M+H⁺]: 351,0 ,
13,8 g 2-{2-[Cyclopropyl-(2,4-dichlor-benzyl)-amino]-1-methoxy-ethyl}-phenylamin LCMS-Rt (B):1,18 min;
[M+H⁺]: 365,0
und 5,5 g einer Mischfraktion der beiden Produkte erhalten.

Die drei Fraktionen lassen sich alle wie unten für 1-(2-Amino-phenyl)-2-[cyclopropyl-(2,4-dichlor-benzyl)-amino]-ethanol unter b) beschrieben mit Schwefelsäure zu 2-(6,8-Dichlor-2-cyclopropyl-1,2,3,4-tetrahydro-isoquinolin-4-yl)-phenylamin cyclisieren.

### b) 2-((R)-6,8-Dichlor-2-cyclopropyl-1,2,3,4-tetrahydro-isoquinolin-4-yl)-phenylamin und 2-((S)-6,8-Dichlor-2-cyclopropyl-1,2,3,4-tetrahydro-isoquinolin-4-yl)-phenylamin

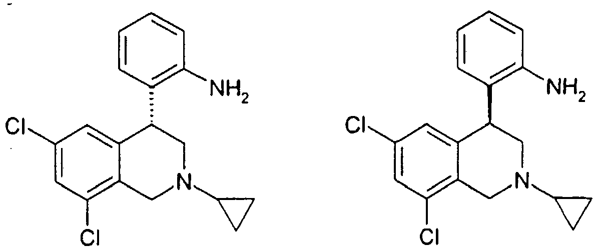

1-(2-Amino-phenyl)-2-[cyclopropyl-(2,4-dichlor-benzyl)-amino]-ethanol (14 g) wurde in Dichlormethan (250 ml) gelöst und unter Rühren auf 0 °C abgekühlt. In der Kälte wurde konz. Schwefelsäure (50 ml) zugetropft. Nach beendeter Zugabe wurde ca. 7 h bei Raumtemperatur gerührt und dann 2 h bei 45°C, wobei das Dichlormethan langsam verdampfte. Nach Stehen über Nacht bei Raumtemperatur wurde zur Vervollständigung der Reaktion erneut Schwefelsäure zugegeben (5 ml) und die Temperatur 12 h auf 65°C gehalten. Danach wurde das Reaktionsgemisch auf Eiswasser gegeben und mit 10 N Natronlauge auf pH 11 gestellt. Die Wasserphase wurde drei Mal mit Dichlormethan extrahiert. Die vereinigten Extrakte wurden über Magnesiumsulfat getrocknet, filtriert und eingeengt. Das Rohprodukt (12,3 g) wurde über Kieselgel chromatographiert (Essigester/n-Heptan 1:1 bis 4:1). Das erhaltene aufgereinigte racemische Produkt (10,7 g) wurde anschließend mittels chiraler HPLC (Chiralpak ADH/45, Eluent Heptan/Isopropanol/Methanol 20:1:1+0,1% TFA) in seine Enantiomere getrennt.
Erhalten wurden 7 g des zuerst eluierenden 2-((R)-6,8-Dichlor-2-cyclopropyl-1,2,3,4-tetrahydro-isoquinolin-4-yl)-phenylamin P1 und 6 g des langsamer eluierenden 2-((S)-6,8-Dichlor-2-cyclopropyl-1,2,3,4-tetrahydro-isoquinolin-4-yl)-phenylamin P2 in Form ihrer doppelten TFA-Salze.
Chirale HPLC:
Säule: Chiralpak ADH/45, 250 x 4,6 mm;
Eluent: Heptan/Isopropanol/Methanol: 20/1/1 + 0,1 % TFA
Fluss: 1 ml/min bei 30°C
P1: Rt: 7,27 min
P2: Rt: 12,81 min

### c) [2-((R)-6,8-Dichlor-2-cyclopropyl-1,2,3,4-tetrahydro-isoquinolin-4-yl)-phenyl]-thiocarbamidsäure-phenylester

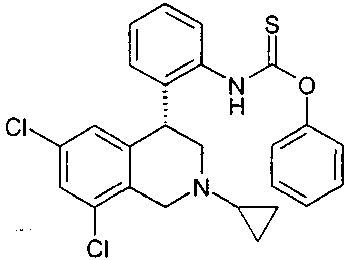

2-((R)-6,8-Dichlor-2-cyclopropyl-1,2,3,4-tetrahydro-isoquinolin-4-yl)-phenylamin (300 mg) wurde in absolutem THF (5 ml) gelöst und Phenyl-chlorthioformiat (75 µl) in abolutem THF (1 ml) gelöst zugetropft. Das Reaktionsgemisch rührte 4 h bei Raumtemperatur und stand über Nacht. Dann wurde das Lösungsmittel abgezogen und der Rückstand mittels präparativer HPLC gereinigt. Die Produkt enthaltenden Fraktionen wurden vereinigt, das Acetonitril am Rotationsverdampfer abgezogen, der wässrige Rückstand mit gesättigter Natriumhydrogencarbonat-Lösung neutralisiert und drei Mal mit Essigester extrahiert. Nach Trocknen der vereinigten Essigester-Phasen über Magnesiumsulfat wurde filtriert und zur Trockne gebracht.
LCMS-Rt (B): 1,87 min;
[M+H⁺]: 469,0

### d) 3-[2-((R)-6,8-Dichlor-2-cyclopropyl-1,2,3,4-tetrahydro-isochinolin-4-yl)-phenyl]-thiazolidin-2,4-dion

[2-((R)-6,8-Dichlor-2-cyclopropyl-1,2,3,4-tetrahydro-isochinolin-4-yl)-phenyl]-thiocarbamidsäure-phenylester (50 mg) wurde in 30%iger Natriummethylat-Lösung in Methanol (5 ml) gelöst. Zum Lösen wurde unter Rühren mit dem Fön leicht erwärmt. Die klare Lösung wurde ca. 3h bei Raumtemperatur gerührt und über Nacht stehen gelassen. Anschließend wurde das Raktionsgemisch mit Wasser verdünnt und drei Mal mit Essigester extrahiert. Die vereinigten Extrakte wurden mit Magnesiumsulfat getrocknet, filtriert und eingeengt. Das so erhaltene Rohprodukt (45 mg) wurde in absolutem Methylenchlorid (3,5 ml) gelöst und Bromacetylchlorid (16,5 mg) zugegeben. Es wurde bei Raumtemperatur unter Feuchtigkeitsausschluss 3 h gerührt. Nach Stehen über Nacht im Tiefkühlschrank wurden das Lösungsmittel und das überschüssige Säurechlorid abgedampft und der Rückstand mittels präparativer HPLC gereinigt. Die Produkt enthaltenden Fraktionen wurden vereinigt, das Acetonitril am Rotationsverdampfer abgezogen, der wässrige Rückstand mit gesättigter Natriumhydrogencarbonat-Lösung neutralisiert und drei Mal mit Essigester extrahiert. Nach Trocknen der vereinigten Essigester-Phasen über Magnesiumsulfat wurde filtriert und zur Trockne gebracht. Ein Teil (12 mg) des erhaltenen Rückstands (15 mg) wurde in Wasser / Acetonitril gelöst, etwas Salzsäure zugesetzt und die klare Lösung über Nacht gefriergetrocknet.
LCMS-Rt (B): 1,40 min;
[M+H⁺]: 433,0

### Beispiel 42: 3-[2-((R)-6,8-Dichlor-2-methyl-1,2,3,4-tetrahydro-isochinolin-4-yl)-phenyl]-thiazolidin-2,4-dion

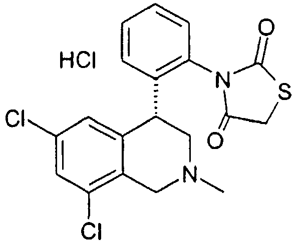

Analog zu Beispiel 41 wurde (R)-2-(6,8-Dichlor-2-methyl-1,2,3,4-tetrahydro-isochinolin-4-yl)-phenylamin (Enantiomer B, Darstellung wie in WO2004085404 beschrieben) umgesetzt. Das Rohprodukt zeigte einen Doppelpeak identischer Masse im LCMS, so dass beide Produkte mittels präparativer HPLC getrennt und anschließend identifiziert wurden. Neben einer verunreinigten Fraktion ließen sich die beiden Rotationsisomeren P1 und P2 isolieren.
P1:
LCMS-Rt (B): 1,09 min;
[M+H⁺]: 407,0
¹H-NMR (500 MHz, DMSO-d₆): 7,51-7,28 (m, 4 H), 7,11 (m, 1H), 6,72 (s, 1 H), 4,51 (d, 15 Hz, 1 H), 4,34 (d, 15 Hz, 1 H), 4,18 (m, 1 H), 3,64 (d, 15 Hz, 1 H), 3,50 (d, 15 Hz, 1 H), 2,70 (m, 1 H), 2,49 (m, mit DMSO), 2,33 (s, 3H) [in ppm]
P2:
LCMS-Rt (B): 1,14 min;
[M+H⁺]: 407,0
¹H-NMR (500 MHz, DMSO-d₆): 7,52-7,26 (m, 4 H), 7,19-7,09 (m, 1H), 6,72 (s, 1 H), 4,49 (d, 15 Hz, 1 H), 4,35 (d, 15 Hz, 1 H), 4,12 (m, 1 H), 3,65 (d, 15 Hz, 1 H), 3,50 (d, 15 Hz, 1 H), 2,72 (m, 1 H), 2,48 (m, mit DMSO), 2,33 (s, 3H) [in ppm]

### Alternativsynthese:

Verwendete man statt Bromacetylchlorid Chloracetylchlorid, so wurde hauptsächlich P2 erhalten, das nach präparativer HPLC nur geringfügig mit P1 verunreinigt war (<10 % laut ¹H-NMR).

### Beispiel 43: 3-[2-((R)-6,8-Dichlor-2-methyl-1,2,3,4-tetrahydro-isochinolin-4-yl)-phenyl]-oxazolidin-2,4-dion

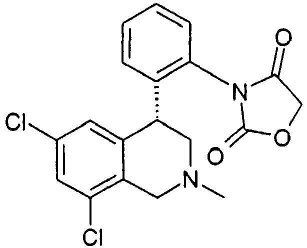

### a) Essigsäure-[2-((R)-6,8-dichlor-2-methyl-1,2,3,4-tetrahydro-isochinolin-4-yl)-phenyl-carbamoyl]-methylester

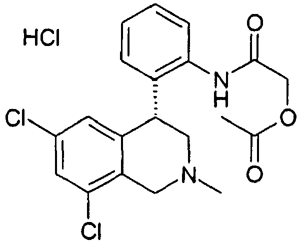

(R)-2-(6,8-Dichlor-2-methyl-1,2,3,4-tetrahydro-isochinolin-4-yl)-phenylamin (300 mg, Enantiomer B, Darstellung wie in WO2004085404 beschrieben) wurde in absolutem THF (10 ml) gelöst und bei Raumtemperatur unter Rühren mit Natriumhexamethyl-disilazid-Lösung (0,5 ml; 2 M in THF) versetzt. Nach 30 min. wurde Acetoxy-acetylchlorid (77 µl) zugegeben und 2 h bei Raumtemperatur gerührt. Anschließend wurde das Reaktionsgemisch eingeengt, der Rückstand in Essigester / Wasser gelöst, mit Natriumhydrogencarbonat-Lösung basisch gestellt und die Phasen getrennt. Die wässrige Phase wurde noch drei Mal mit Essigester extrahiert. Die vereinigten Extrakte wurden mit Magnesiumsulfat getrocknet, filtriert und eingeengt. Es wurden 380 mg des gewünschten Produkts erhalten. Davon wurden 40 mg in Acetonitril/Wasser gelöst, mit 0,1 N Salzsäure angesäuert und gefriergetrocknet. LCMS-Rt (B): 1,02 min;
[M+H⁺]: 407,0

### b) N-[2-((R)-6,8-Dichlor-2-methyl-1,2,3,4-tetrahydro-isochinolin-4-yl)-phenyl]-2-hydroxy-acetamid

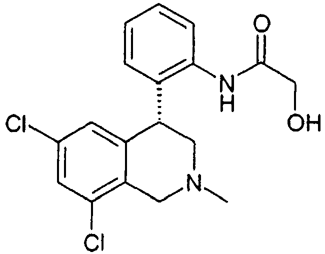

Essigsäure-[2-((R)-6,8-dichlor-2-methyl-1,2,3,4-tetrahydro-isochinolin-4-yl)-phenylcarbamoyl]-methylester180 mg) wurde in Methanol (5 ml) gelöst. Unter Rühren wurde Kaliumcarbonat (305 mg, fein gepulvert) zugegeben und dann 4 h bei Raumtemperatur gut gerührt. Anschließend wurde das Lösungsmittel abgedampft, der Rückstand in Essigester aufgenommen und drei Mal mit Wasser gewaschen. Die Essigesterphase wurde mit Magnesiumsulfat getrocknet, filtriert und eingeengt. Es wurden 160 mg des gewünschten Produkts erhalten.
LCMS-Rt (B): 0,94 min;
[M+H⁺]: 365,0

### c) 3-[2-((R)-6,8-Dichlor-2-methyl-1,2,3,4-tetrahydro-isochinolin-4-yl)-phenyl]-oxazolidin-2,4-dion

N-[2-((R)-6,8-Dichlor-2-methyl-1,2,3,4-tetrahydro-isochquinolin-4-yl)-phenyl]-2-hydroxy-acetamid (20 mg) wurde in absolutem THF (2 ml) gelöst und bei Raumtemperatur mit Natriumhexamethyldisilazid-Lösung (0,5 ml; 2 M in THF) unter Rühren umgesetzt. Danach wurde 1,1-Carbonyldiimidazol (13 mg) zugegeben und 2 h bei Raumtemperatur gerührt. Nach Stehen über Nacht wurde das Reaktionsgemisch eingeengt, der Rückstand in Essigester / Wasser gelöst, mit Natriumhydrogencarbonat-Lösung basisch gestellt und die Phasen getrennt. Die wässrige Phase wurde noch drei Mal mit Essigester extrahiert. Die vereinigten Extrakte wurden mit Magnesiumsulfat getrocknet, filtriert und eingeengt. Der Rückstand wurde mittels präparativer HPLC gereinigt. Die Produkt enthaltenden Fraktionen wurden vereinigt, das Acetonitril am Rotationsverdampfer abgezogen, der wässrige Rückstand mit gesättigter Natriumhydrogencarbonat-Lösung neutralisiert und drei Mal mit Essigester extrahiert. Nach Trocknen der vereinigten Essigester-Phasen über Magnesiumsulfat wurde filtriert und zur Trockne gebracht. Der Rückstand wurde in Wasser / Acetonitril gelöst und die klare Lösung über Nacht gefriergetrocknet. Es wurde 10 mg des gewünschten Produkts erhalten.
LCMS-Rt (B): 1,09 min;
[M+H⁺]: 391,0

### Beispiel 43a: 3-[2-((R)-6,8-Dichlor-2-cyclopropyl-1,2,3,4-tetrahydro-isochinolin-4-yl)-phenyl]-oxazolidin-2,4-dion-Hydrochlorid

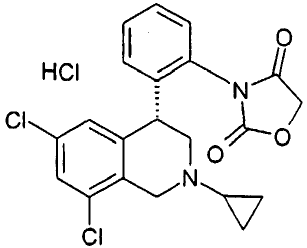

Analog der Sequenz in Beispiel 43 wurden 25 mg der Titelverbindung synthetisiert. Im Cyclisierungsschritt wurde statt 1,1-Carbonyldiimidazol Trichlormethylchloroformat eingesetzt und die Gefriertrocknung in Gegenwart von Salzsäure durchgeführt.LCMS-Rt (B): 1,31 min;
[M+H⁺]: 417,0

### Beispiel 44: 4-[2-((R)-6,8-Dichlor-2-methyl-1,2,3,4-tetrahydro-isochinolin-4-yl)-phenyl]-morpholin-3,5-dion-Trifluoressigsäuresalz

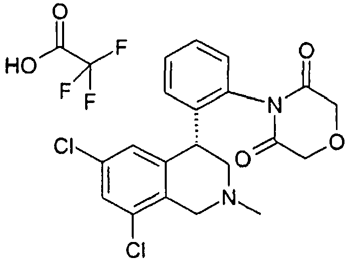

### a) 4 {[2-((R)-6,8-Dichlor-2-methyl-1,2,3,4-tetrahydro-isochinolin-4-yl)-phenylcarbamoyl]-methoxy}-essigsäure-Trifluoressigsäuresalz

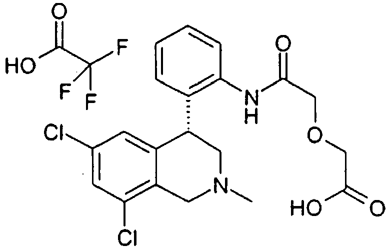

(R)-2-(6,8-Dichlor-2-methyl-1,2,3,4-tetrahydro-isochinolin-4-yl)-phenylamin (70 mg, Enantiomer B, Darstellung wie in WO2004085404 beschrieben) wurde in absolutem

Dichlormethan (5 ml) unter Rühren gelöst und mit Diglycolicanhydrid (27 mg) versetzt. Es wurde mehrere Stunden bei Raumtemperatur gerührt und über Nacht stehen gelassen. Nach Zugabe weiteren Diglycolicanhydrids (26 mg) wurde 10 h gerührt und über Nacht stehen gelassen. Nach Abziehen des Lösungsmittels wurde der Rückstand mittels präparativer HPLC gereinigt. Die Produkt enthaltenden Fraktionen wurden vereinigt und das Acetonitril am Rotationsverdampfer abgezogen und der wässrige Rückstand gefriergetrocknet.
LCMS-Rt (B): 0,93 min;
[M+H⁺]: 423,1

### b) 4-[2-((R)-6,8-Dichlor-2-methyl-1,2,3,4-tetrahydro-isochinolin-4-yl)-phenyl]-morpholin-3,5-dion-Trifluoressigsäuresalz

4 {[2-((R)-6,8-Dichlor-2-methyl-1,2,3,4-tetrahydro-isochinolin-4-yl)-phenylcarbamoyl)-methoxy}-essigsäure-Trifluoressigsäuresalz (50 mg) wurde in absolutem Dichlormethan (2 ml) unter Rühren gelöst und mit Hünig-Base (46 µl) versetzt. Dann wurde EDC (22 mg) zugegeben und bei Raumtemperatur mehrere Stunden gerührt. Nach Stehen über Nacht wurde weiteres EDC (22 mg) und Hünig-Base (46 µl) zugegeben. Nach 24 h wurde das Lösungsmittel abgezogen und der Rückstand mittels präparativer HPLC gereinigt. Die Produkt-enthaltenden Fraktionen werden vereinigt, vom Acetonitril befreit und gefriergetrocknet.
LCMS-Rt (B): 1,07 min;
[M+H⁺]: 405,0

### Beispiel 45: 3-[2-((R)-6,8-Dichlor-2-cyclopropyl-1,2,3,4-tetrahydro-isochinolin-4-yl)-phenyl]-oxazolidin-2-on

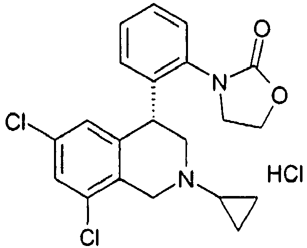

### a) [2-((R)-6,8-Dichlor-2-cyclopropyl-1,2,3,4-tetrahydro-isochinolin-4-yl)-phenyl]-carbamid-säure-2-chlor-ethylester

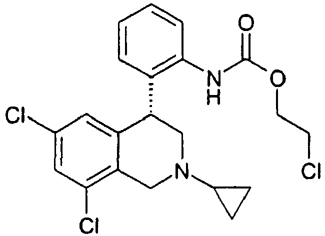

2-((R)-6,8-Dichlor-2-cyclopropyl-1,2,3,4-tetrahydro-isochinolin-4-yl)-phenylamin (400 mg) wurde in absolutem THF (25 ml) gelöst, und 2-Chlorethylchloroformat (102 mg) wurde unter Rühren zugegeben. Nach 3 h Rühren bei Raumtemperatur wurde der Ansatz über Nacht stehen gelassen. Dann wurde das Lösungsmittel abgezogen und der Rückstand mittels präparativer HPLC gereinigt. Die Produkt enthaltenden Fraktionen wurden vereinigt, das Acetonitril am Rotationsverdampfer abgezogen, der wässrige Rückstand mit gesättigter Natriumhydrogencarbonat-Lösung neutralisiert und drei Mal mit Essigester extrahiert. Nach Trocknen der vereinigten Essigester-Phasen über Magnesiumsulfat wurde filtriert und zur Trockne gebracht. Vom gewünschten Produkt wurden 380 mg erhalten.
LCMS-Rt (B): 1,66 min;
[M+H⁺]: 439,0

### b) 3-[2-((R)-6,8-Dichlor-2-cyclopropyl-1,2,3,4-tetrahydro-isochinolin-4-yl)-phenyl]-oxazolidin-2-on

[2-((R)-6,8-Dichlor-2-cyclopropyl-1,2,3,4-tetrahydro-isochinolin-4-yl)-phenyl]-carbamid-säure-2-chlor-ethylester320 mg) wurde in absolutem THF (20 ml) gelöst. Unter Rühren wurde Natriumhydrid (35 mg) zugegeben. Nach 2 h Rühren bei Raumtemperatur wurde das Reaktionsgemisch eingeengt, der Rückstand in Essigester gelöst und zwei Mal mit Wasser gewaschen. Die Essigesterphase wurde über Magnesiumsulfat getrocknet, filtriert und eingeengt. Der Rückstand wurde mittels präparativer HPLC gereinigt. Die Produkt enthaltenden Fraktionen wurden vereinigt, das Acetonitril am Rotationsverdampfer abgezogen, der wässrige Rückstand mit gesättigter Natriumhydrogencarbonat-Lösung neutralisiert und drei Mal mit Essigester extrahiert.

Nach Trocknen der vereinigten Essigester-Phasen über Magnesiumsulfat wurde filtriert und zur Trockne gebracht. Der Rückstand wurde in Acetonitril/Wasser gelöst, mit 0,1 N Salzsäure angesäuert und gefriergetrocknet. Es wurden 176 mg des gewünschten Produkts als Hydrochlorid erhalten.
LCMS-Rt (B): 1,16 min;
[M+H⁺]: 403,0

### Beispiel 46: 3-[2-((R)-6,8-Dichlor-2-methyl-1,2,3,4-tetrahydro-isochinolin-4-yl)-phenyl]-oxazolidin-2-on

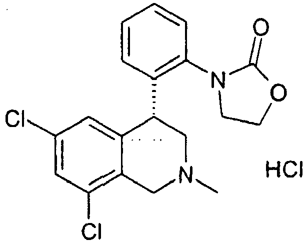

### a) [2-((R)-6,8-Dichlor-2-methyl-1,2,3,4-tetrahydro-isochinolin-4-yl)-phenyl]-carbamid saure-2-chlor-ethylester

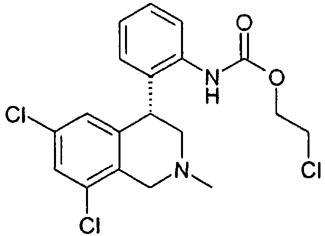

Analog zu Beispiel 45a) wurde (R)-2-(6,8-Dichlor-2-methyl-1,2,3,4-tetrahydro-isochinolin-4-yl)-phenylamin (300 mg, Enantiomer B, Darstellung wie in WO2004085404 beschrieben) mit 2-Chlorethylchloroformat (140 mg) umgesetzt. Es wurden 400 mg des gewünschten Produkts erhalten.
LCMS-Rt (B): 1,15 min;
[M+H⁺]: 413,0

### b) 3-[2-((R)-6,8-Dichlor-2-methyl-1,2,3,4-tetrahydro-isochinolin-4-yl)-phenyl]-oxazolidin-2-on

Analog zu Beispiel 45b) wurde 2-((R)-6,8-Dichlor-2-methyl-1,2,3,4-tetrahydro-isochinolin-4-yl)-phenyl]-carbamidsäure-2-chlor-ethylester (340 mg)mit Natriumhydrid (39 mg) umgesetzt. Es wurden 230 mg des gewünschten Produkts als Hydrochlorid erhalten.
LCMS-Rt (B): 1,01 min;
[M+H⁺]: 377,0

### Beispiel 47: (S)-1-[2-((R)-6,8-Dichlor-2-methyl-1,2,3,4-tetrahydro-isochinolin-4-yl)-phenyl]-3-hydroxy-pyrrolidin-2,5-dion

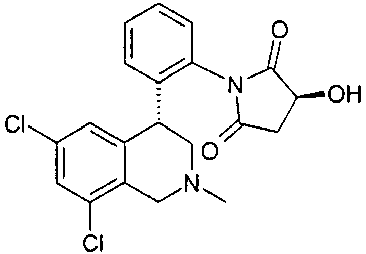

((S)-5-Oxo-2-trichlormethyl-[1,3]dioxolan-4-yl)-essigsäure (171 mg, siehe Synthesis 2002, 2165) und Thionylchlorid (2 ml) wurden vorgelegt und unter Rühren 4 h zum Rückfluss erhitzt. Anschließend wurde das überschüssige Thionylchlorid am Rotavapor abdestilliert, der Rückstand in absolutem Toluol (14 ml) gelöst, (R)-2-(6,8-Dichlor-2-methyl-1,2,3,4-tetrahydro-isochinolin-4-yl)-phenylamin und Triethylamin (0,91 ml) zugegeben und das Gemisch 2 h rückflussiert. Nach Stehen über Nacht wurde das Reaktionsgemisch eingeengt, der Rückstand mit Essigester/ Wasser versetzt, mit Natriumhydrogencarbonat-Lösung auf
pH 8 gestellt und drei Mal mit gesättigter Kochsalz-Lösung gewaschen. Die Essigesterphase wurde mit Magnesiumsulfat getrocknet, filtriert und eingeengt. Der Rückstand wurde aus Essigester/Heptan umkristallisiert. Es wurden 65 mg der gewünschten Verbindung erhalten, die Rotationsisomerie zeigt. Allerdings zeigte sich im LCMS nur eine schwache, nicht quantifizierbare Aufspaltung.
LCMS-Rt (B): 1.02 min;
[M+H⁺]: 405,0

### Beispiel 48: (R)-1-[2-((R)-6,8-Dichlor-2-methyl-1,2,3,4-tetrahydro-isochinolin-4-yl)-phenyl]-3-hydroxy-pyrrolidin-2,5-dion

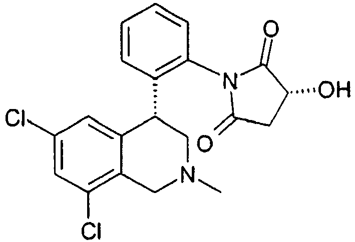

((R)-5-Oxo-2-trichlormethyl-[1,3]dioxolan-4-yl)-essigsäure (214 mg, siehe Synthesis 2002, 2165) und Thionylchlorid (2,5 ml) wurden vorgelegt und unter Rühren 4 h zum Rückfluss erhitzt. Anschließend wurde das überschüssige Thionylchlorid am Rotavapor abdestilliert, Rückstand in absolutem Toluol (18 ml) gelöst, (R)-2-(6,8-Dichlor-2-methyl-1,2,3,4-tetrahydro-isochinolin-4-yl)-phenylamin (250 mg) und Triethylamin (1,13 ml) zugegeben und das Gemisch 2 h rückflussiert. Anschließend wurde das Reaktionsgemisch eingeengt, der Rückstand mit Essigester/ Wasser versetzt, mit Natriumhydrogencarbonat-Lösung auf pH 8 gestellt und drei Mal mit gesättigter Kochsalz-Lösung gewaschen. Die Essigesterphase wurde mit Magnesiumsulfat getrocknet, filtriert und eingeengt. Anschließende Kristallisation aus Essigester/n-Heptan lieferte 240 mg des gewünschten Produkts, das Rotationsisomerie zeigt, so dass im LCMS zwei Signale identischer Masse gefunden wurden
LCMS-Rt (B): 0,99 und 1,04 min;
[M+H⁺]: 405,0

### Beispiel 49: (R)-1-[2-((R)-6,8-Dichlor-2-cyclopropyl-1,2,3,4-tetrahydro-isochinolin-4-yl)-phenyl]-3-hydroxy-pyrrolidin-2,5-dion

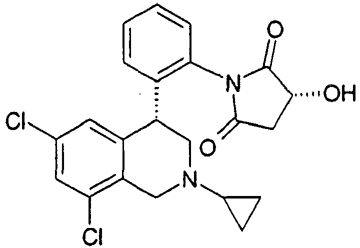

((R)-5-Oxo-2-trichlormethyl-[1,3]dioxolan-4-yl)-essigsäure (94 mg, siehe Synthesis 2002, 2165) und Thionylchlorid (2 ml) wurden vorgelegt und unter Rühren 4 h zum Rückfluss erhitzt. Das überschüssige Thionylchlorid wurde am Rotavapor abdestilliert und der Rückstand in absolutem Toluol (15 ml) gelöst. 2-((R)-6,8-Dichlor-2-cyclopropyl-1,2,3,4-tetrahydro-isoquinolin-4-yl)-phenylamin-Trifluoressigsäuresalz (200 mg) wurde fest zugegeben, gefolgt von Triethylamin (0,5 ml). Anschließend wurde die Mischung 2 h bei Raumtemperatur, dann 1 h bei 60°C, dann 2 h bei 80°C und abschließend 6 h bei 100°C gerührt. Nach Abkühlen und Entfernen des Lösungsmittels wurde mittels präparativer HPLC gereinigt. Die Produkt enthaltenden Fraktionen wurden vereinigt, das Acetonitril am Rotationsverdampfer abgezogen und der wässrige Rückstand gefriergetrocknet. Zur weiteren Aufreinigung wurde dann über Kieselgel nachgereinigt (Dichlormethan / Methanol 100:0 auf 90:10). Die sauberen Fraktionen wurden vereinigt und vom Lösungsmittel befreit. Gefriertrocknung aus Wasser/Acetonitril lieferte 20 mg des gewünschten Produkts.
LCMS-Rt (B): 1,15 min;
[M+H⁺]: 431,0

### Beispiel 50: (S)-1-[2-((R)-6,8-Dichlor-2-cyclopropyl-1,2,3,4-tetrahydro-isochinolin-4-yl)-phenyl]-3-hydroxy-pyrrolidin-2,5-dion

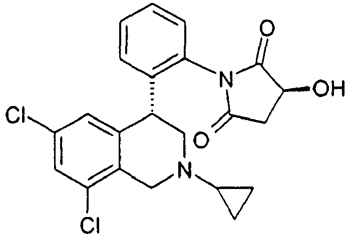

Analog zu Beispiel 49 wurden ((R)-5-Oxo-2-trichlormethyl-[1,3]dioxolan-4-yl)-essigsäure (94 mg, siehe Synthesis 2002, 2165), Thionylchlorid (2 ml) und 2-((R)-6,8-Dichlor-2-cyclopropyl-1,2,3,4-tetrahydro-isoquinolin-4-yl)-phenylamin-Trfluoressigsäuresalz (200 mg) umgesetzt. Die Gefriertrocknung aus Wasser/Acetonitril lieferte 31 mg des gewünschten Produkts.
LCMS-Rt (B):1,13 min;
[M+H⁺]: 413,0

### Beispiel 51: (R)-6,8-Dichlor-4-[2-(1,1-dioxo-1-λ⁶-isothiazolidin-2-yl)-phenyl]-2-methyl-1,2,3,4-tetrahydro-isochinolin-Hydrochlorid

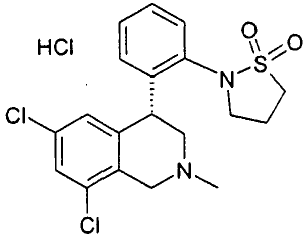

(R)-2-(6,8-Dichlor-2-methyl-1,2,3,4-tetrahydro-isochinolin-4-yl)-phenylamin (100 mg) wurde in absolutem THF (8 ml) vorgelegt und bei Raumtemperatur 3-Chlorpropansulfonylchlorid (116 mg, in absolutem THF (1,5 ml) gelöst), zugetropft und anschließend das Reaktionsgemisch 16 Stunden rückflussiert. Zur Vervollständigung der Reaktion wurde nochmals Chlorpropansulfonylchlorid (8 mg) zugegeben, gefolgt von Natriumhexamethydisilazid-Lösung (0,2 ml; 2 M in THF). Nach 30 min. Rühren bei Raumtemperatur und anschließendem Rückflussieren wurde zur Vervollständigung der Reaktion ein weiteres Äquivalent Natriumhexamethydisilazid-Lösung zugegeben und erneut rückflussiert. Anschließend wird bei Raumtemperatur mit Wasser versetzt und drei Mal gegen Essigester extrhiert. Die vereinigten Essigesterphasen wurden über Magnesiumsulfat getrocknet, filtriert und eingeengt. Der Rückstand wurde anschließend mittels präparativer HPLC gereinigt. Die Produkt enthaltenden Fraktionen wurden vereinigt, das Acetonitril am Rotationsverdampfer abgezogen, der wässrige Rückstand mit gesättigter Natriumhydrogencarbonat-Lösung neutralisiert und drei Mal mit Essigester extrahiert. Nach Trocknen der vereinigten Essigesterphasen über Magnesiumsulfat wurde filtriert und zur Trockne gebracht. Der Rückstand wurde mit Wasser/Salzsäure aufgenommen und gefriergetrocknet. Es wurden 12 mg der Titelverbindung erhalten.
LCMS-Rt (B): 1,05 min;
[M+H⁺]: 411,0

### Beispiel 52: 1-[2-((R)-6,8-Dichlor-1,2,3,4-tetrahydro-isochinolin-4-yl)-phenyl]-pyrrolidin-2,5-dion-Trifluoressigsäuresalz

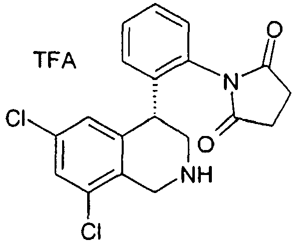

### a) N-{2-[2-(2,4-Dichlor-benzylamino)-1-hydroxy-ethyl]-phenyl}-acetamid

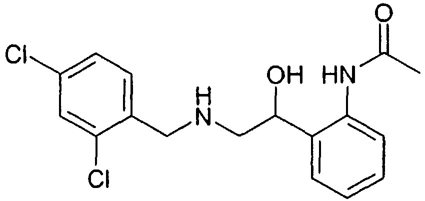

2,4-Dichlorbenzylamin (2,1 ml) wurde in absolutem Ethanol (20 ml) gelöst und N-[2-(2-Bromacetyl)-phenyl]-acetamid (2 g), gelöst in absolutem Ethanol (50 ml), unter Rühren bei Raumtemperatur zugetropft. Nach 30 min. wurde Natriumborhydrid (600 mg) unter Eiskühlung zugegeben und weitere 1,5 h gerührt. Anschließend wurde das Lösungsmittel abgezogen und der Rückstand in einem Essigester/Wasser-Gemisch aufgenommen. Die Phasen wurden getrennt und die wässrige Phase noch drei Mal mit Essigester extrhiert. Die vereinigten organischen Phasen wurden über Magnesiumsulfat getrocknet, filtriert und eingeengt. Der Rückstand wurde mittels präparativer Chromatographie gereinigt. Die Produkt enthaltenden Fraktionen wurden vereinigt, das Acetonitril am Rotationsverdampfer abgezogen und der wässrige Rückstand mit Kaliumcarbonat basisch gestellt und drei Mal mit Essigester extrahiert. Die vereinigten organischen Phasen wurden über Magnesiumsulfat getrocknet, filtriert und eingeengt. Es wurden 720 mg der Titelverbindung erhalten.
LCMS-Rt (B): 0,93 min;
[M+H⁺]: 353,0

### b) 1-(2-Amino-phenyl)-2-(2,4-dichlorbenzylamino)-ethanol

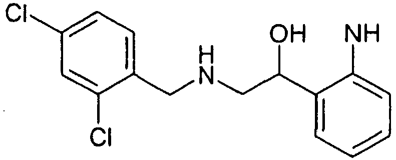

N-{2-[2-(2,4-Dichlor-benzylamino)-1-hydroxy-ethyl]-phenyl}-acetamid (100 mg) wurde in Methanol (5 ml) gelöst und mit Natriummethylat-Lösung (0,3 ml; 30%ig in Methanol) versetzt. Nach 6 h Rückfluss wird das Reaktionsgemisch auf Eiswasser gegeben, mit 2 N Salzsäure neutralisiert und drei Mal mit Essigester extrahiert. Die vereinigten organischen Phasen wurden über Magnesiumsulfat getrocknet, filtriert und eingeengt. Der Rückstand wurde mittels präparativer HPLC aufgereinigt. Die Produkt enthaltenden Fraktionen wurden vereinigt, das Acetonitril am Rotationsverdampfer abgezogen, der wässrige Rückstand mit gesättigter Kaliumcarbonat-Lösung neutralisiert und drei Mal mit Essigester extrahiert. Nach Trocknen der vereinigten Essigesterphasen über Magnesiumsulfat wurde filtriert und zur Trockne gebracht. Es wurden 34 mg der gewünschten Verbindung erhalten, die direkt weiter umgesetzt wurde.
LCMS-Rt (B):0,90 min;
[M+H⁺]: 311,0

### c) 2-((R)-6,8-Dichlor-1,2,3,4-tetrahydro-isochinolin-4-yl)-phenylamin

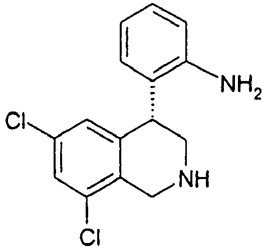

1-(2-Amino-phenyl)-2-(2,4-dichlorbenzylamino)-ethanol (34 mg) wurde in Dichlormethan (1 ml) gelöst und unter Eiskühlung und Rühren mit konzentrierter Schwefelsäure versetzt. Anschließend wurde das Eisbad entfernt und unter Abdampfen des Dichlormethans das Gemisch 10 Stunden bei 80°C gerührt. Nach Abkühlen wurde das Reaktionsgemisch unter Eiskühlung mit Eiswasser versetzt und mit 10 M Natriumhydroxid-Lösung alkalisch gestellt. Die wässrige Phase wurde drei Mal mit Dichlormethan extrahiert und die vereinigten organischen Phasen über Magnesiumsulfat getrocknet, filtiriert und eingeengt. Der Rückstand wurde über Kieselgel aufgereinigt. Es wurden 22 mg der gewünschten Verbindung erhalten. LCMS-Rt (B): 0,99 min;
[M+H⁺]: 293,0

### d) 1-[2-((R)-6,8-Dichlor-1,2,3,4-tetrahydro-isochinolin-4-yl)-phenyl]-pyrrolidin-2,5-dion-Trifluoressigsäuresalz

2-((R)-6,8-Dichlor-1,2,3,4-tetrahydro-isochinolin-4-yl)-phenylamin (22 mg) wurde zusammen mit Bernsteinsäure (10 mg) und Polyphosphorsäure (2 ml) in einem verschlossenen Schraubreagenzglas 3 h auf 150°C erwärmt. Zur Aufarbeitung wurde das noch heiße Reaktionsgemisch auf Eiswasser gegeben und mit gesättigter Kaliumcarbonat-Lösung alkalisch gestellt. Die wässrige Phase wurde drei Mal mit Essigester extrahiert und die vereinigten organischen Phasen über Magnesiumsulfat getrocknet, filtiriert und eingeengt. Der Rückstand wurde mittels präparativer HPLC aufgereinigt. Die Produkt enthaltenden Fraktionen wurden vereinigt, das Acetonitril am Rotationsverdampfer abgezogen und der Rückstand gefriergetrocknet. Es wurden 7 mg der Titelverbindung erhalten.
LCMS-Rt (B): 1.04 min;
[M+H⁺]:375,0

### Beispiel 53: 3-[2-((R)-6,8-Dichlor-2-cyclopropyl-1,2,3,4-tetrahydro-isochinolin-4-yl)-phenyl]-imidazolidin-2,4-dion-Hydrochlorid

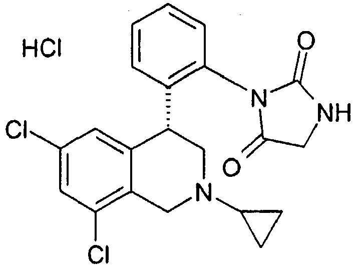

39 mg der Titelverbindung wurden ausgehende von 2-((R)-6,8-Dichlor-2-cyclopropyl-1,2,3,4-tetrahydro-isoquinolin-4-yl)-phenylamin (Beispiel 41b) und Ethylisocyanatoacetat analog Beispiel 1 erhalten.
LCMS-Rt (B): 1,14 min;
[M+H⁺]: 416,0

### Beispiel 54: 3-[2-((R)-6,8-Dichlor-2-methyl-1,2,3,4-tetrahydro-isochinolin-4-yl)-phenyl]-1-methyl-imidazolidin-2,4-dion-Hydrochlorid

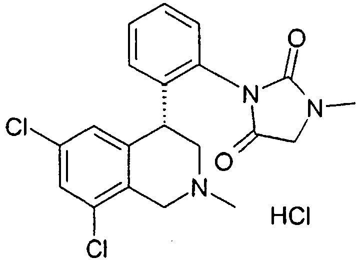

### a) [2-((R)-6,8-Dichlor-2-methyl-1,2,3,4-tetrahydro-isochinolin-4-yl)-phenyl]-carbamidsäure-4-nitro-phenylester

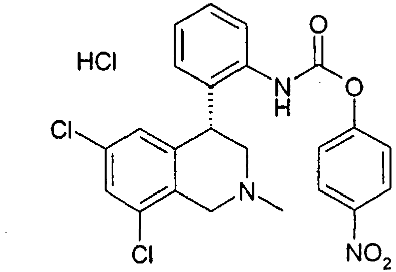

(R)-2-(6,8-Dichlor-2-methyl-1,2,3,4-tetrahydro-isochinolin-4-yl)-phenylamin (200 mg, Enantiomer B, Darstellung wie in WO2004085404 beschrieben) wurde in absolutem Dichlormethan (6 ml) gelöst und unter Rühren mit 4-Nitrophenylchloroformat (157 mg) versetzt. Nach 4 h wurden weitere 0,1 Äquivalente 4-Nitrophenylchloroformat zugegeben. Nach Stehen über Nacht wurde das Dichlormethan abgezogen und der Rückstand direkt in der nächsten Stufe eingesetzt.
LCMS-Rt (B):1,29 min;
[M+H⁺]: 472,0

b) 3-[2-((R)-6,8-Dichlor-2-methyl-1,2,3,4-tetrahydro-isochinolin-4-yl)-phenyl]-1-methyl-imidazolidin-2,4-dion-Hydrochlorid [2-((R)-6,8-Dichlor-2-methyl-1,2,3,4-tetrahydro-isochinolin-4-yl)-phenyl]-carbamid-säure-4-nitro-phenylester (125 mg) wurde in absolutem THF (5 ml) gelöst und unter Rühren Sarcosin-methylester-Hydrochlorid und Triethylamin zugetropft. Nach 5 h Rühren wurde das Reaktionsgemisch vom Lösungsmittel befreit, der Rückstand mit Wasser und 2 N Salzsäure versetzt und 2 h rückflussiert. Nach Stehen über Nacht wurde das Reaktionsgemisch eingeengt und mit Essigester, Wasser und gesättigter Kaliumcarbonat-Lösung versetzt. Nach Trennen der Phasen wurde die organische Phase fünf Mal mit gesättigter Kaliumcarbonat-Lösung und zwei Mal mit gesättigter Kochsalz-Lösung gewaschen. Nach Trocknen über Magnesiumsulfat und Filtrieren wurde eingeengt. Der Rückstand wurde mittels präparativer HPLC gereinigt. Die Produkt enthaltenden Fraktionen wurden vereinigt, das Acetonitril am Rotationsverdampfer abgezogen, der wässrige Rückstand mit gesättigter Kaliumcarbonat-Lösung neutralisiert und drei Mal mit Essigester extrahiert. Nach Trocknen der vereinigten Essigesterphasen über Magnesiumsulfat wurde filtriert und zur Trockne gebracht. Der Rückstand wurde mit Wasser/Salzsäure aufgenommen und gefriergetrocknet. Es wurden 60 mg der Titelverbindung erhalten.
LCMS-Rt (B): 1,02 min;
[M+H⁺]: 404,0

### Beispiel 55: 3-[2-((R)-6,8-Dichlor-2-cyclopropyl-1,2,3,4-tetrahydro-isochinolin-4-yl)-phenyl]-1-methyl-imidazolidin-2,4-dion-Hydrochlorid

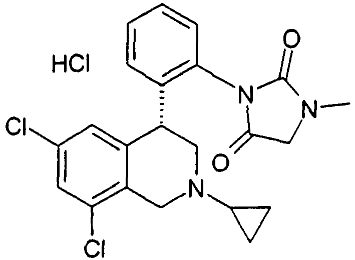

Ausgehend von 2-((R)-6,8-Dichlor-2-cyclopropyl-1,2,3,4-tetrahydro-isochinolin-4-yl)-phenylamin (Beispiel 41 b) wurde die Titelverbindung analog zu Beispiel 54 synthetisiert und 55 mg der Titelverbindung als Hydrochlorid gewonnen. LCMS-Rt (B): 1,17 min;
[M+H⁺]: 430,0

### Beispiel 56: 3-[2-((R)-6,8-Dichlor-2-methyl-1,2,3,4-tetrahydro-isochinolin-4-yl)-phenyl]-5,5-dimethyl-imidazolidin-2,4-dion-Hydrochlorid

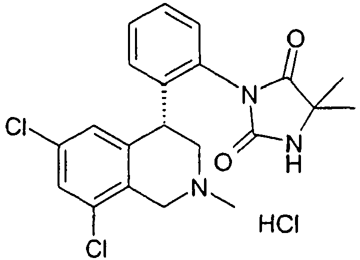

(R)-2-(6,8-Dichlor-2-methyl-1,2,3,4-tetrahydro-isochinolin-4-yl)-phenylamin (50 mg, Enantiomer B, Darstellung wie in WO2004085404 beschrieben) wurde in absolutem Dichlormethan (5 ml) gelöst und unter Rühren 2-Isocyanato-2-methylpropionsäure methylester zugetropft. Nach 3 h Rühren und Stehenlassen über Nacht wurde mit Wasser versetzt, die Phasen getrennt und die wässrige Phase noch zwei Mal mit Dichlormethan extrahiert. Die vereinigten organischen Phasen wurden über Magnesiumsulfatgetrocknet, filtriert und eingeengt. Der Rückstand wurde mittels präparativer HPLC gereinigt. Die Produkt enthaltenden Fraktionen wurden vereinigt, das Acetonitril am Rotationsverdampfer abgezogen, der wässrige Rückstand mit gesättigter Kaliumcarbonat-Lösung neutralisiert und drei Mal mit Essigester extrahiert. Nach Trocknen der vereinigten Essigesterphasen über Magnesiumsulfat wurde filtriert und zur Trockne gebracht. Der Rückstand wurde in Acetonitril (1 ml) gelöst und mit 10 %iger Salzsäure versetzt. Nach 2 h Rühren wurde mit Wasser (3 ml) verdünnt und gefriergetrocknet. Es wurden 68 mg der Titelverbindung erhalten.
LCMS-Rt (B): 1,08 min;
[M+H⁺]: 418,0

### Beispiel 57: (R und S)-3-[2-((R)-6,8-Dichlor-2-methyl-1,2,3,4-tetrahydro-isochinolin-4-yl)-phenyl]-5-(2-methylsulfanyl-ethyl)-imidazolidin-2,4-dion-Trifluoressigsäuresalz

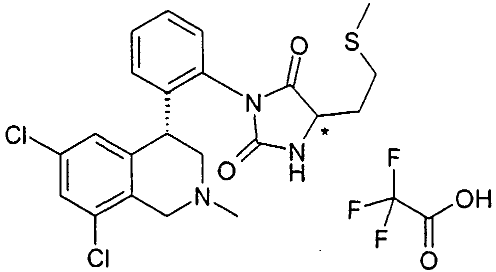

(R)-2-(6,8-Dichlor-2-methyl-1,2,3,4-tetrahydro-isochinolin-4-yl)-phenylamin (50 mg, Enantiomer B, Darstellung wie in WO2004085404 beschrieben) wurde analog zu Beispiel 56 mit Ethyl-2-isocyanato-4-(methylthio)-butyrat (33 mg) in Methylenchlorid umgesetzt, aufgereinigt und ringgeschlossen. Das dann erhaltene Rohprodukt wurde mittels präparativer HPLC gereinigt und konnte dabei in seine Diastereomeren, das polarere P1 und das unpolarere P2, getrennt werden.

P1:
LCMS-Rt (B): 1,11 min;
[M+H⁺]: 464,0

P2:
LCMS-Rt (B): 1,16 min;
[M+H⁺]: 464,0

Ausgehend von (R)-2-(6,8-Dichlor-2-methyl-1,2,3,4-tetrahydro-isochinolin-4-yl)-phenylamin (Enantiomer B, Darstellung wie in WO2004085404 beschrieben) und den entsprechenden Isocyanaten wurden analog Beispiel 56 folgende Produkte als Diastereomerengemische erhalten:

| Beispiel | Struktur | Salz | Analog Beispiel | MS [M+H⁺] | LCMS-Rt [min] |
|---|---|---|---|---|---|
| 58 | 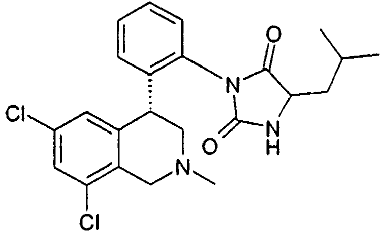 | HCl | 56 | 446,1 (ESI⁺) | 1,25 (B) |
| 59 | 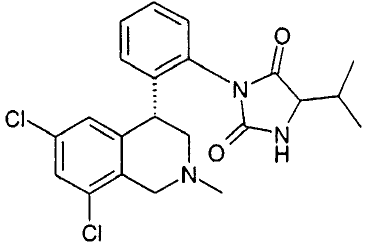 | HCl | 56 | 432,0 (ESI⁺) | 1,13 und 1,21 (B) |
| 60 | 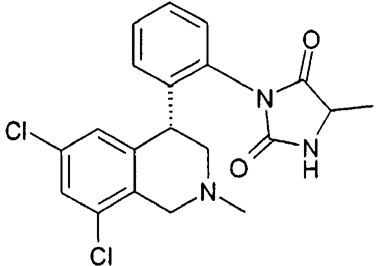 | - | 56 | 404,0 (ESI⁺) | 1,04 und 1,12 (B) |

### Beispiel 61: 1-[2-((R)-2-Methyl-1,2,3,4-tetrahydro-isochinolin-4-yl)-phenyl]-pyrrolidin-2,5-dion-Hydrochlorid

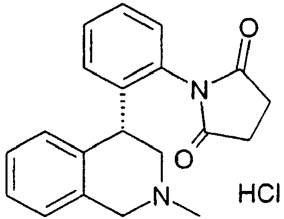

In einer Hydrierapparatur wurde zu einer Lösung aus 1-[2-((R)-6,8-Dichlor-2-methyl-1,2,3,4-tetrahydro-isochinolin-4-yl)-phenyl]-pyrrolidin-2,5-dion (68 mg, Beispiel 7) in Methanol (15 ml) eine Spatelspitze Palladium auf Aktivkohle (5 %) gegeben und nach aufbringen einer Wasserstoffatmosphäre 2 h geschüttelt. Nach Entfernen des Wasserstoffs wurde die Apparatur über Nacht unter Argon stehen gelassen und dann der Katalysator abfiltriert und mit Methanol gewaschen. Das Filtrat wurde zur Trockne gebracht. 10 mg des Rohprodukts wurden mit Wasser und 10 %iger Salzsäure aufgenommen und gefriergertocknet. Es wurden 10 mg der Titelverbindung erhalten.
LCMS-Rt (B): 0,87 min;
[M+H⁺]: 312,1

### Beispiel 62: 1-[2-((R)-6,8-Dichlor-2-methyl-1,2,3,4-tetrahydro-isochinolin-4-yl)-phenyl]-3-methyl-1,3-dihydro-imidazol-2-on-Hydrochlorid

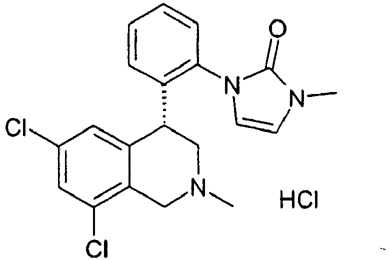

(R)-2-(6,8-Dichlor-2-methyl-1,2,3,4-tetrahydro-isochinolin-4-yl)-phenylamin (Enantiomer B, Darstellung wie in WO2004085404 beschrieben), 4-Nitrophenylchloroformat und Methylaminoacetaldehyd-dimethylacetal wurden analog Beispiel 40 umgesetzt und anschließend das erhaltene Zwischenprodukt (85 mg) mit einem Gemisch aus Wasser (1 ml) und 10 %iger Salzsäure (3,2 ml) cyclisiert. Aufarbeitung und Reinigung erfolgten analog Bespiel 40. Es wurden 65 mg der Titelverbindung isoliert.
LCMS-Rt (B): 1,02 min;
[M+H⁺]: 388,0

### Beispiel 63: 1-[2-(8-Brom-6-chlor-2-methyl-1,2,3,4-tetrahydro-isoquinolin-4-yl)-phenyl]-pyrrolidin-2,5-dion-Hydrochlorid

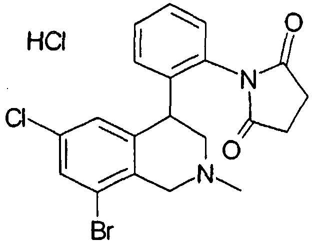

### a) 2-Brom-1-brommethyl-4-chlor-benzol

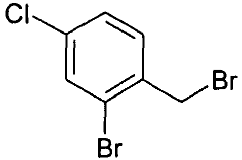

2-Brom-4-Chlortoluol (5 g) wurde in Tetrachlorkohlenstoff (120 ml) vorgelegt, und dann wurden nacheinander unter Rühren AIBN (400 mg) und NBS (4,8 g) eingetragen. Anschließend wurde das Reaktionsgemisch unter Rückfluss 6 h erhitzt und dann weiteres NBS (0,6 g) bei Raumtemperatur zugegeben. Nach weiteren 2 h unter Rückfluss wurde die Heizung entfernt und über Nacht stehen gelassen. Nach Abfiltrieren des Niederschlags wurde mit Tetrachlorkohlenstoff gewaschen und dann das Filtrat drei Mal mit 0,5 N Natriumhydrogencarbonat-Lösung extrahiert, gefolgt von mehreren Waschungen mit Wasser, bis der pH-Wert der Waschlösung neutral war. Die organische Phase wurde über Magnesiunsulfat getrocknet, filtriert und eingeengt. Nach Chromatographie über Kieselgel wurden 3,57 g der Titelverbindung erhalten. LCMS-Rt (B): 1,85 min;
[M+H⁺]: 202,6 (Cl⁺)

### b) (2-Brom-4-chlor-benzyl)-methyl-amin

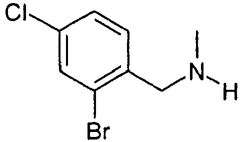

33%-ige ethanolische Methylamin-Lösung (13 ml) wurde vorgelegt und 2-Brom-1-brommethyl-4-chlor-benzol (3 g), in absolutem Ethanol (20 ml) gelöst, innerhalb von 30 min. unter Rühren zugetropft. Nach 4 h Rühren bei Raumtemperatur wurde über Nacht stehen gelassen. Nach Abziehen des Lösungsmittels wurde der Rückstand mit Essigester aufgenommen und einmal mit 1 N Salzsäure extrahiert. Die wässrige Phase wurde mit 10 N Natronlauge auf pH 11 gestellt und dann 3 Mal mit Essigester extrhiert. Die vereinigten Essigesterphasen wurden über Magnesiumsulfat getrocknet, filtriert und eingeengt. Es wurden 1,7 g der gewünschten Verbindung erhalten. LCMS-Rt (B): 0,76 min;
[M+H⁺]: 233,9

### c) N-(2-{2-[(2-Brom-4-chlor-benzyl)-methyl-amino]-acetyl}-phenyl)-acetamid

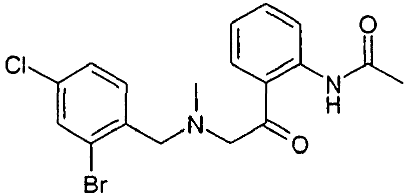

(2-Brom-4-chlor-benzyl)-methyl-amin (1,7 g) wurde in absolutem Ethanol (5 ml) vorgelegt. Nach Zugabe von Natriumhydrogencarbonat (1,2 g) wurde N-[2-(2-Brom-acetyl)-phenyl]-acetamid (2 g), gelöst in Ethanol (80 ml), unter Rühren zugetropft. Nach 4 h Rühren bei Raumtemperatur stand der Ansatz über Nacht. Zur Aufarbeitung wurde das Lösungsmittel abgezogen, der Rückstand mit Essigester/Wasser versetzt, die wässrige Phase noch zwei Mal mit Essigester extrhiert und anschließend die vereinigten Essigester Fraktionen über Magnesiumsulfat getrocknet, filtriert und eingeengt. Das Rohprodukt wurde mittels präparativer HPLC gereinigt. Die Produkt enthaltenden Fraktionen wurden vereinigt, das Acetonitril am Rotationsverdampfer abgezogen, der wässrige Rückstand mit gesättigter Natriumhydrogencarbonat-Lösung neutralisiert und drei Mal mit Essigester extrahiert. Nach Trocknen der vereinigten Essigesterphasen über Magnesiumsulfat wurde filtriert und zur Trockne gebracht. Es wurden 1,5 g des gewünschten Produkts erhalten.
LCMS-Rt (B): 1,07 min;
[M+H⁺]: 409,0

### d) N-(2-{2-[(2-Brom-4-chlor-benzyl)-methyl-amino]-1-hydroxy-ethyl}-phenyl)-acetamid

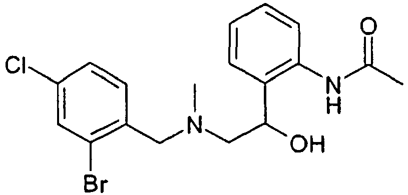

N-(2-{2-[(2-Brom-4-chlor-benzyl)-methyl-amino]-acetyl}-phenyl)-acetamid (1,5 g) wurden in Methanol (45 ml) vorgelegt und mit einem Eisbad auf 0°C abgekühlt. Nachdem Natriumborhydrid (266 mg) portionsweise eingetragen worden war, ließ man auf Raumtemperatur kommen und dann 2 h weiter rühren. Das Lösungsmittel wurde abgezogen und der Rückstand mit Essigester/Wasser versetzt, drei Mal mit Essigester extrahiert und die vereinigten organischen Phasen über Magnesiumsulfat getrocknet, filtriert und eingeengt. Es wurden 1,44 g der Titelverbindung erhalten.
LCMS-Rt (B): 0,94 min;
[M+H⁺]: 411,0

### e) 1-(2-Amino-phenyl)-2-[(2-brom-4-chlor-benzyl)-methyl-amino]-ethanol

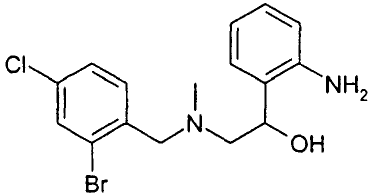

N-(2-{2-[(2-Brom-4-chlor-benzyl)-methyl-amino]-1-hydroxy-ethyl}-phenyl)-acetamid (1,4 g) wurde in absolutem Methanol (50 ml) gelöst und mit Natriummethylat-Lösung (3,5 ml) versetzt und 8h auf Rückfluss erhitzt. Nach erneuter Zugabe von NatriumMethylat-Lösung (1,5 ml) wurde weitere 4 h auf Rückfluss erhitzt. Das Reaktionsgemisch wurde auf Eiswasser gegeben, drei Mal mit Essigester extrahiert und die vereinigten organischen Phasen über Magnesiumsulfat getrocknet, filtriert und eingeengt. Der Rückstand wurde mittels präparativer HPLC gereinigt. Die Produkt enthaltenden Fraktionen wurden vereinigt, das Acetonitril am Rotationsverdampfer abgezogen, der wässrige Rückstand mit gesättigter Kaliumcarbonat-Lösung neutralisiert und drei Mal mit Essigester extrahiert. Nach Trocknen der vereinigten Essigesterphasen über Magnesiumsulfat wurde filtriert und zur Trockne gebracht. Es wurden 801 mg der Titelverbindung erhalten.
LCMS-Rt (B): 0,92 min;
[M+H⁺]: 369,0

### f) 2-(8-Brom-6-chlor-2-methyl-1,2,3,4-tetrahydro-isoquinolin-4-yl)-phenylamin

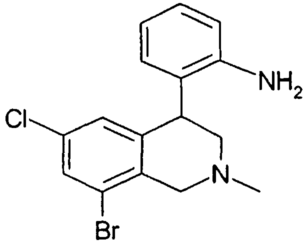

1-(2-Amino-phenyl)-2-[(2-brom-4-chlor-benzyl)-methyl-amino]-ethanol (800 mg) wurden in Dichlormethan (1 ml) gelöst und unter Eiskühlung mit konzentrierter Schwefelsäure (8 ml) versetzt. Anschließend wurde 7 h bei 60°C gerührt. Zur Aufarbeitung wurde das Reaktionsgemisch auf Eiswasser gegeben, mit 10 N Natronlauge alkalisch gestellt und drei Mal mit Essigester gewaschen. Die vereinigten organischen Phasen wurden mit Magnesiunsulfat getrocknet, filtriert und eingeengt. Der Rückstand wurde mittels präparativer HPLC gereinigt. Die Produkt enthaltenden Fraktionen wurden vereinigt, das Acetonitril am Rotationsverdampfer abgezogen, der wässrige Rückstand mit gesättigter Natriumhydrogencarbonat-Lösung neutralisiert und drei Mal mit Essigester extrahiert. Nach Trocknen der vereinigten Essigesterphasen über Magnesiumsulfat wurde filtriert und zur Trockne gebracht. Es wurden 438 mg der gewünschten Verbindung erhalten.
LCMS-Rt (B): 1,03 min;
[M+H⁺]: 351,0

### g) 1-[2-(8-Brom-6-chlor-2-methyl-1,2,3,4-tetrahydro-isochinolin-4-yl)-phenyl]-pyrrolidin-2,5-dion-Hydrochlorid

2-(8-Brom-6-chlor-2-methyl-1,2,3,4-tetrahydro-isochinolin-4-yl)-phenylamin (60 mg) wurde mit Bernsteinsäure (22 mg) versetzt und in Polyphosphorsäure analog Beispiel 7 umgesetzt. Es wurden 47 mg der Titelverbindung erhalten.
LCMS-Rt (B): 1,04 min;
[M+H0⁺]: 433,0

### Beispiel 64: 1-[2-(8-Brom-6-chlor-2-methyl-1,2,3,4-tetrahydro-isochinolin-4-yl)-phenyl]-pyrrolidin-2-on

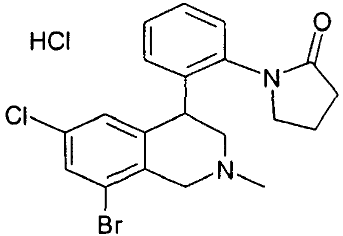

Analog Beispiel 36 wurde 2-(8-Brom-6-chlor-2-methyl-1,2,3,4-tetrahydro-isochinolin-4-yl)-phenylamin (80 mg) mit 4-Chlorbuttersäurechlorid (35 mg) umgesetzt und das erhaltene Amid anschließend in DMSO mit Kaliumcarbonat ringgeschlossen. LCMS-Rt (B): 1,05 min;
[M+H⁺]:419,0

### Pharmakologische Daten:

### Testbeschreibung:

In diesem Test wurde die Erholung des intrazellulären pH-Wertes (pHᵢ) von LAP1-Zellen, die den Natrium-Protonen-Austauscher Subtyp 3 (NHE3) stabil exprimieren, nach einer Ansäuerung ermittelt. Diese Erholung setzt bei funktionsfähigem NHE3 auch unter bicarbonatfreien Bedingungen ein. Dazu wurde der pHᵢ mit dem pHsensitiven Fluoreszenzfarbstoff BCECF (Molecular Probes, Eugene, OR, USA, eingesetzt wird die Vorstufe BCECF-AM) bestimmt. Die Zellen wurden zunächst mit BCECF (5 µM BCECF-AM) in NH₄Cl-Puffer (NH₄Cl-Puffer: 115 mM CholinCl, 20 mM NH₄Cl, 5 mM KCI, 1 mM CaCl₂, 1 mM MgCl₂, 20 mM Hepes, 5 mM Glucose, mit 1 M KOH wird ein pH von 7,4 eingestellt) inkubiert. Die intrazelluläre Ansäuerung wurde durch Waschen der in NH₄Cl-Puffer inkubierten Zellen mit NH₄Cl-freien Puffer (133,8 mM Cholinchlorid, 4,7 mM KCI, 1,25 mM CaCl₂, 1,25 mM MgCl₂, 0,97 mM K₂HPO₄, 0,23 mM KH₂PO₄, 5 mM Hepes, 5 mM Glucose, mit 1 M KOH wird ein pH von 7,4 eingestellt) induziert. Nach dem Waschvorgang wurden 90 µl des NH₄Cl-freien Puffer auf den Zellen belassen. Die pH-Erholung wurde durch die Zugabe vom 90 µl Na⁺-haltigem Puffer (133,8 mM NaCl, 4,7 mM KCl, 1,25 mM CaCl₂, 1,25 mM M_{g}Cl₂, 0,97 mM Na₂HPO₄, 0,23 mM NaH₂PO₄, 10 mM Hepes, 5 mM Glucose, mit 1 M NaOH wird ein pH von 7,4 eingestellt) im Messgerät (FLIPR, "Fluorometric Imaging Plate Reader", Molecular Devices, Sunnyvale, Ca., USA) gestartet. Die BCECF-Fluoreszenz wurde bei einer Anregungswellenlänge von 498 nm und dem FLIPR-Emissionsfilter 1 (Bandpass von 510 bis 570 nm) bestimmt. Die nachfolgenden Fluoreszenzänderungen als Maß der pH-Erholung wurden zwei Minuten registriert. Für die Berechnung der NHE3-inhibitorischen Potenz der getesteten Substanzen wurden die Zellen zunächst in Puffern untersucht, bei denen eine vollständige bzw. überhaupt keine pH-Erholung stattfand. Zur vollständigen pH-Erholung (100%) wurden die Zellen in Na⁺-haltigem Puffer (s.o.), für die Bestimmung des 0%-Wertes in Na⁺-freien Puffer (s.o.) inkubiert. Die zu testenden Substanzen wurden in Na⁺-haltigem Puffer angesetzt. Die Erholung des intrazellulären pHs bei jeder getesteten Konzentration einer Substanz wurde in Prozent der maximalen Erholung ausgedrückt. Aus den Prozentwerten der pH-Erholung wurde mittels des Programms XLFit (idbs, Surrey, UK) der IC₅₀-Wert der jeweiligen Substanz für den NHE3 berechnet.

Die inhibitorische Wirkung (IC₅₀-Werte) diverser Beispielverbindungen auf den NHE3 ist in nachfolgender Tabelle aufgeführt:

| Beispiel | IC₅₀ [µM] |
|---|---|
| 4 | 0,129 |
| 7 | 0,039 |
| 8 | 2,59 |
| 10 | 0,047 |
| 14 | 0,119 |
| 16 | 0,222 |
| 29 | 1,2 |
| 34 | 29,2 |
| 35 | 0,082 |
| 38 | 0,461 |
| 40 | 0,131 |
| 42 | 0,069 |
| 43 | 0,049 |
| 44 | 0,22 |
| 46 | 0,22 |
| 51 | 0,141 |
| 52 | 12,3 |
| 55 | 0,12 |
| 57 | 1,4 |
| 58 | 7,4 |
| 64 | 0,25 |

## Patentansprüche

1. Verbindungen der Formel I, 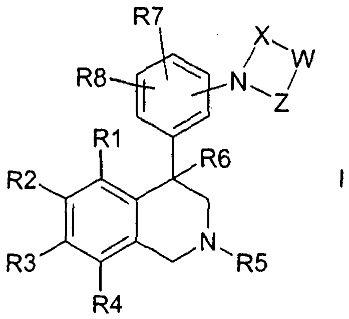 worin bedeuten:
R1, R2, R3 und R4 unabhängig voneinander Wasserstoff, F, Cl, Br, I, CN, NO₂ oder R11-(CₘH₂ₘ)-Aₙ-;
m Null, 1, 2, 3 oder 4;
n Null oder 1;
R11 Wasserstoff, Methyl oder CₚF₂ₚ₊₁;
A Sauerstoff, NH, N(CH₃) oder S(O)_{q};
p 1, 2 oder 3;
q Null, 1 oder 2;
R5 Wasserstoff, Alkyl mit 1, 2, 3, 4, 5 oder 6 C-Atomen oder Cycloalkyl mit 3, 4, 5 oder 6 C Atomen;
R6 Wasserstoff, OH, F, CF₃, Alkyl mit 1, 2, 3 oder 4 C-Atomen oder Cycloalkyl mit 3, 4, 5 oder 6 C Atomen;
R7 und R8 unabhängig voneinander Wasserstoff, F, Cl, Br, CN, CO₂R12, NR13R14 oder R16-(CₘₘH₂ₘₘ)-Bₙₙ-;
R12 Wasserstoff, Alkyl mit 1, 2, 3 oder 4 C-Atomen oder Cycloalkyl mit 3, 4, 5 oder 6 C-Atomen;
R13 und R14 unabhängig voneinander Wasserstoff, Alkyl mit 1, 2, 3 oder 4 C-Atomen oder Cycloalkyl mit 3, 4, 5 oder 6 C-Atomen;
oder
R13 und R14 bilden mit dem Stickstoffatom, an das sie gebunden sind, einen 4, 5, 6 oder 7 gliedrigen Ring, in dem eine CH₂-Gruppe durch NR15, S oder Sauerstoff ersetzt sein kann;
R15 Wasserstoff, Alkyl mit 1, 2, 3 oder 4 C-Atomen oder Cycloalkyl mit 3, 4, 5 oder 6 C-Atomen;
mm Null, 1, 2, 3 oder 4;
nn Null oder 1;
R16 Wasserstoff, Methyl oder CₚₚF₂ₚₚ₊₁;
B Sauerstoff oder S(O)_{qq};
pp 1, 2 oder 3;
qq Null, 1 oder 2;
W CᵣH₂ᵣ oder CₛH₂ₛ₋₂;
wobei eine oder mehrere CH₂-Gruppen in CᵣH₂ᵣ und CₛH₂ₛ₋₂ durch NR17, Sauerstoff oder S ersetzt sein können;
R17 Wasserstoff, Alkyl mit 1, 2, 3 oder 4 C-Atomen oder Cycloalkyl mit 3, 4, 5 oder 6 C-Atomen;
r 1, 2, 3, 4, 5, 6, 7 oder 8;
s 2, 3, 4, 5, 6, 7 oder 8;
X -C(O)- oder -S(O)₂-;
Z -C(O)- oder eine Bindung;
sowie deren pharmazeutisch verträgliche Salze und Trifluoracetate.

2. Verbindungen der Formel I nach Anspruch 1, worin bedeuten
R1, R2, R3 und R4 unabhängig voneinander Wasserstoff, F, Cl, Br, CN oder R11-(CₘH₂ₘ)-Aₙ-;
m Null oder 1;
n Null oder 1;
R11 Wasserstoff, Methyl oder CₚF₂ₚ₊₁;
A Sauerstoff, NCH₃ oder S(O)q;
p 1 oder 2;
q Null, 1 oder 2;
R5 Wasserstoff, Methyl, Ethyl oder Cyclopropyl;
R6 Wasserstoff oder Methyl;
R7 und R8 unabhängig voneinander Wasserstoff, F, Cl, CN, CO₂R12, NR13R14 oder R16-(CₘₘH₂ₘₘ)-Bₙₙ-;
W CᵣH₂ᵣ oder CₛH₂ₛ₋₂; wobei eine oder mehrere CH₂-Gruppen in CᵣH₂ᵣ und CₛH₂ₛ₋₂ durch NR 17, Sauerstoff oder S ersetzt sein können;
R12 Wasserstoff, Methyl oder Ethyl;
R13 und R14 unabhängig voneinander Wasserstoff, Alkyl mit 1, 2, 3 oder 4 C-Atomen oder Cycloalkyl mit 3, 4, 5 oder 6 C-Atomen;
oder
R13 und R14 bilden mit dem Stickstoffatom, an das sie gebunden sind, einen 5, 6 oder 7 gliedrigen Ring, in dem eine CH₂-Gruppe durch NR15, S oder Sauerstoff ersetzt sein kann;
R15 Wasserstoff, Alkyl mit 1, 2, 3 oder 4 C-Atomen oder Cycloalkyl mit 3, 4, 5 oder 6 C-Atomen;
mm Null, 1 oder 2;
nn Null oder 1;
R16 Wasserstoff, Methyl oder CₚₚF₂ₚₚ₊₁;
B Sauerstoff oder S(O)_{qq};
pp 1 oder 2;
qq Null, 1 oder 2;
R17 Wasserstoff, Alkyl mit 1, 2, 3 oder 4 C-Atomen oder Cycloalkyl mit 3, 4, 5 oder 6 C-Atomen;
r 2, 3, 4, 5, 6, 7 oder 8;
s 2, 3, 4, 5, 6, 7 oder 8;
X -C(O)- oder -S(O)₂-;
Z -C(O)-;
sowie deren pharmazeutisch verträglichen Salze und Trifluoracetate.

3. Verbindungen der Formel I nach Anspruch 1 und/oder 2, worin bedeuten
R1 und R3 Wasserstoff;
R2 und R4 unabhängig voneinander Wasserstoff, F, Cl, Br, NH₂, NHCH₃ oder N(CH₃)₂;
R5 Wasserstoff, Methyl, Ethyl oder Cyclopropyl;
R6 Wasserstoff oder Methyl;
R7 und R8 Wasserstoff;
W CᵣH₂ᵣ oder CₛH₂ₛ₋₂;
wobei eine oder mehrere CH₂-Gruppen in CᵣH₂ᵣ und CₛH₂ₛ₋₂ durch NR17, Sauerstoff oder S ersetzt sein können;
R17 Wasserstoff oder Methyl;
r 2, 3, 4, 5 oder 6;
s 2, 3, 4, 5 oder 6;
X -C(O)- oder -S(O)₂-;
Z -C(O)-;
sowie deren pharmazeutisch verträglichen Salze und Trifluoracetate.

4. Verbindungen der Formel I nach einem oder mehreren der Ansprüche 1 bis 3 ausgewählt aus der Gruppe:
1-[2-(8-Brom-6-chlor-2-methyl-1,2,3,4-tetrahydro-isoquinolin-4-yl)-phenyl]-pyrrolidin-2,5-dion,
3-[2-((R)-6,8-Dichlor-2-cyclopropyl-1,2,3,4-tetrahydro-isochinolin-4-yl)-phenyl]-1-methyl-imidazolidin-2,4-dion,
3-[2-((R)-6,8-Dichlor-2-methyl-1,2,3,4-tetrahydro-isochinolin-4-yl)-phenyl]-1-methyl-imidazolidin-2,4-dion
3-[2-((R)-6,8-Dichlor-2-cyclopropyl-1,2,3,4-tetrahydro-isochinolin-4-yl)-phenyl]-imidazolidin-2,4-dion
(S)-1-[2-((R)-6,8-Dichlor-2-cyclopropyl-1,2,3,4-tetrahydro-isochinolin-4-yl)-phenyl]-3-hydroxy-pyrrolidin-2,5-dion,
(R)-1-[2-((R)-6,8-Dich(or-2-cyclopropyl-1,2,3,4-tetrahydro-isochinolin-4-yl)-phenyl]-3-hydroxy-pyrrolidin-2,5-dion,
(R)-1-[2-((R)-6,8-Dichlor-2-methyl-1,2,3,4-tetrahydro-isochinolin-4-yl)-phenyl]-3-hydroxy-pyrrolidin-2,5-dion,
(S)-1-[2-((R)-6,8-Dichlor-2-methyl-1,2,3,4-tetrahydro-isochinolin-4-yl)-phenyl]-3-hydroxy-pyrrolidin-2,5-dion,
4-[2-((R)-6,8-Dichlor-2-methyl-1,2,3,4-tetrahydro-isochinolin-4-yl)-phenyl]-morpholin-3,5-dion,
3-[2-((R)-6,8-Dichlor-2-methyl-1,2,3,4-tetrahydro-isochinolin-4-yl)-phenyl]-oxazolidin-2,4-dion,
3-[2-((R)-6,8-Dichlor-2-methyl-1,2,3,4-tetrahydro-isochinolin-4-yl)-phenyl]-thiazolidin-2,4-dion,
1-[2-((R)-6,8-Dichlor-2-cyclopropyl-1,2,3,4-tetrahydro-isochinolin-4-yl)-phenyl]-pyrrolidin-2,5-dion,
(S)-1-[2-((R)-6,8-Dichlor-2-methyl-1,2,3,4-tetrahydro-isochinolin-4-yl)-phenyl]-3-methyl-piperidin-2,6-dion,
(R)-1-[2-((R)-6,8-Dichlor-2-methyl-1,2,3,4-tetrahydro-isochinolin-4-yl)-phenyl]-3-methyl-piperidin-2,6-dion,
1-[2-((R)-6,8-Dichlor-2-methyl-1,2,3,4-tetrahydro-isochinolin-4-yl)-phenyl]-3,5-dimethyl-piperidin-2,6-dion,
1-[2-((R)-6,8-Dichlor-2-methyl-1,2,3,4-tetrahydro-isochinolin-4-yl)-phenyl]-3-methyl-pyrrolidin-2,5-dion,
1-[2-((R)-6,8-Dichlor-2-methyl-1,2,3,4-tetrahydro-isochinolin-4-yl)-phenyl]-3,3-dimethyl-pyrrolidin-2,5-dion,
1-[2-(6,8-Dichlor-2-cyclopropyl-1,2,3,4-tetrahydro-isochinolin-4-y1)-phenyl]-pyrrolidin-2,5-dion,
1-[4-((S)-6,8-Dichlor-2-methyl-1,2,3,4-tetrahydro-isochinolin-4-yl)-phenyl]-piperidin-2,6-dion,
1-[4-((S)-6,8-Dichlor-2-methyl-1,2,3,4-tetrahydro-isochinolin-4-yl)-phenyl]-pyrrolidin-2,5-dion,
1-[4-(6,8-Dichlor-2-methyl-1,2,3,4-tetrahydro-isochinolin-4-yl)-phenyl]-pyrrolid in-2,5-dion,
1-[2-((R)-6,8-Dichlor-2-methyl-1,2,3,4-tetrahydro-isochinolin-4-yl)-phenyl]-piperidin-2,6-dion,
1-[2-(6,8-Dichlor-2-methyl-1,2,3,4-tetrahydro-isochinolin-4-yl)-phenyl]-piperidin-2,6-dion,
1-[3-((S)-6,8-Dichlor-2-methyl-1,2,3,4-tetrahydro-isochinolin-4-yl)-phenyl]-pyrrol-2,5-dion,
1-[2-(6,8-Dichlor-2-methyl-1,2,3,4-tetrahydro-isochinolin-4-yl)-phenyl]-pyrrolidin-2,5-dion,
1-[2-((R)-6,8-Dichlor-2-methyl-1,2,3,4-tetrahydro-isochinolin-4-yl)-phenyl]-pyrrolidin-2,5-dion,
3-[2-((R)-6,8-Dichlor-2-methyl-1,2,3,4-tetrahydro-isochinolin-4-yl)-phenyl]-imidazolidin-2,4-dion,
3-[2-(6,8-Dichlor-2-methyl-1,2,3,4-tetrahydro-isochinolin-4-yl)-phenyl]-imidazolidin-2,4-dion,
3-[3-((S)-6,8-Dichlor-2-methyl-1,2,3,4-tetrahydro-isochinolin-4-yl)-phenyl]-imidazolidin-2,4-dion,
3-[4-((S)-6,8-Dichlor-2-methyl-1,2,3,4-tetrahydro-isochinolin-4-yl)-phenyl]-imidazolidin-2,4-dion,
3-[4-(6,8-Dichlor-2-methyl-1,2,3,4-tetrahydro-isochinolin-4-yl)-phenyl]-imidazolidin-2,4-dion,
3-[2-((R)-6,8-Dichlor-2-cyclopropyl-1,2,3,4-tetrahydro-isochinolin-4-yl)-phenyl]-oxazolidin-2,4-dion,
3-[2-((R)-6,8-Dichlor-2-methyl-1,2,3,4-tetrahydro-isochinolin-4-yl)-phenyl]-5-methyl-imidazolidin-2,4-dion,
(3R,4S)-1-[2-((R)-6,8-Dichlor-2-methyl-1,2,3,4-tetrahydro-isochinolin-4-yl)-phenyl]-3,4-dimethyl-pyrrolidin-2,5-dion,
1-[4-((S)-6,8-Dichlor-2-methyl-1,2,3,4-tetrahydro-isochinolin-4-yl)-phenyl]-pyrrol-2,5-dion,
1-[3-((S)-6,8-Dichlor-2-methyl-1,2,3,4-tetrahydro-isochinolin-4-yl)-phenyl]-piperidin-2,6-dion,
1-[3-((S)-6,8-Dichlor-2-methyl-1,2,3,4-tetrahydro-isochinolin-4-yl)-phenyl]-pyrrolidin-2,5-dion,
1-[4-(6,8-Dichlor-2-methyl-1,2,3,4-tetrahydro-isochinolin-4-yl)-phenyl]-piperidin-2,6-dion,
3-[3-(6,8-Dichlor-2-methyl-1,2,3,4-tetrahydro-isochinolin-4-yl)-phenyl]-imidazolidin-2,4-dion,
1-[2-((R)-2-Methyl-1,2,3,4-tetrahydro-isochinolin-4-yl)-phenyl]-pyrrolidin-2,5-dion, 3-[2-((R)-6,8-Dichlor-2-methyl-1,2,3,4-tetrahydro-isoquinolin-4-yl)-phenyl]-5-isopropyl-imidazolidin-2,4-dion,
3-[2-((R)-6,8-Dichlor-2-methyl-1,2,3,4-tetrahydro-isoquinolin-4-yl)-phenyl]-5-isobutyl-imidazolidin-2,4-dion,
(R und S)-3-[2-((R)-6,8-Dichlor-2-methyl-1,2,3,4-tetrahydro-isochinolin-4-yl)-phenyl]-5-(2-methylsulfanyl-ethyl)-imidazolidin-2,4-dion,
3-[2-((R)-6,8-Dichlor-2-methyl-1,2,3,4-tetrahydro-isochinolin-4-yl)-phenyl]-5,5-dimethyl-imidazolidin-2,4-dion,
1-[2-((R)-6,8-Dichlor-1,2,3,4-tetrahydro-isochinolin-4-yl)-phenyl]-pyrrolidin-2,5-dion, 3-[2-((R)-6,8-Dichlor-2-cyclopropyl-1,2,3,4-tetrahydro-isochinolin-4-yl)-phenyl]-thiazolidin-2,4-dion,
1-[2-((S)-6,8-Dichlor-2-methyl-1,2,3,4-tetrahydro-isochinolin-4-yl)-phenyl]-pyrrolidin-2,5-dion,
1-[2-((S)-6,8-Dichlor-2-cyclopropyl-1,2,3,4-tetrahydro-isochinolin-4-yl)-phenyl]-pyrrolidin-2,5-dion,
1-[2-((R)-6,8-Dichlor-2-methyl-1,2,3,4-tetrahydro-isochinolin-4-yl)-phenyl]-3,3,4,4-tetramethyl-pyrrolidin-2,5-dion,
(S)-1-[2-((R)-6,8-Dichlor-2-cyclopropyl-1,2,3,4-tetrahydro-isochinolin-4-yl)-phenyl]-3-methyl-piperidin-2,6-dion,
1-[4-(6,8-Dichlor-2-methyl-1,2,3,4-tetrahydro-isochinolin-4-yl)-phenyl]-4,4-dimethyl-piperidin-2,6-dion
und1-[2-(6,8-Dichlor-2-methyl-1,2,3,4-tetrahydro-isochinolin-4-yl)-phenyl]-pyrrol-2,5-dion,
sowie deren pharmazeutisch verträgliche Salze und Trifluoracetate.

5. Verbindungen der Formel I mach einem oder mehreren der Ansprüche 1 bis 4 ausgewählt aus der Gruppe:
1-[2-(8-Brom-6-chlor-2-methyl-1,2,3,4-tetrahydro-isoquinolin-4-yl)-phenyl]-pyrrolidin-2,5-dion,
3-[2-((R)-6,8-Dichlor-2-cyclopropyl-1,2,3,4-tetrahydro-isochinolin-4-yl)-phenyl]-1-methyl-imidazolidin-2,4-dion,
3-[2-((R)-6,8-Dichlor-2-methyl-1,2,3,4-tetrahydro-isochinolin-4-yl)-phenyl]-1-methyl-imidazolidin-2,4-dion
3-[2-((R)-6,8-Dichlor-2-cyclopropyl-1,2,3,4-tetrahydro-isochinolin-4-yl)-phenyl]-imidazolidin-2,4-dion
(S)-1-[2-((R)-6,8-Dichlor-2-cyclopropyl-1,2,3,4-tetrahydro-isochinolin-4-yl)-phenyl]-3-hydroxy-pyrrolidin-2,5-dion,
(R)-1-[2-((R)-6,8-Dichlor-2-cyclopropyl-1,2,3,4-tetrahydro-isochinolin-4-yl)-phenyl]-3-hydroxy-pyrrolidin-2,5-dion,
(R)-1-[2-((R)-6,8-Dichlor-2-methyl-1,2,3,4-tetrahydro-isochinolin-4-yl)-phenyl]-3-hydroxy-pyrrolidin-2,5-dion,
(S)-1-[2-((R)-6,8-Dichlor-2-methyl-1,2,3,4-tetrahydro-isochinolin-4-yl)-phenyl]-3-hydroxy-pyrrolidin-2,5-dion,
4-[2-((R)-6,8-Dichlor-2-methyl-1,2,3,4-tetrahydro-isochinolin-4-yl)-phenyl]-morpholin-3,5-dion,
3-[2-((R)-6,8-Dichlor-2-methyl-1,2,3,4-tetrahydro-isochinolin-4-yl)-phenyl]-oxazolidin-2,4-dion,
3-[2-((R)-6,8-Dichlor-2-methyl-1,2,3,4-tetrahydro-isochinolin-4-yl)-phenyl]-thiazolidin-2,4-dion,
1-[2-((R)-6,8-Dichlor-2-cyclopropyl-1,2,3,4-tetrahydro-isochinolin-4-yl)-phenyl]-pyrrolidin-2,5-dion,
(S)-1-[2-((R)-6,8-Dichlor-2-methyl-1,2,3,4-tetrahydro-isochinolin-4-yl)-phenyl]-3-methyl-piperidin-2,6-dion,
(R)-1-[2-((R)-6,8-Dichlor-2-methyl-1,2,3,4-tetrahydro-isochinolin-4-yl)-phenyl]-3-methyl-piperidin-2,6-dion,
1-[2-((R)-6,8-Dichlor-2-methyl-1,2,3,4-tetrahydro-isochinolin-4-yl)-phenyl]-3,5-dimethyl-piperidin-2,6-dion,
1-[2-((R)-6,8-Dichlor-2-methyl-1,2,3,4-tetrahydro-isochinolin-4-yl)-phenyl]-3-methyl-pyrrolidin-2,5-dion,
1-[2-((R)-6,8-Dichlor-2-methyl-1,2,3,4-tetrahydro-isochinolin-4-yl)-phenyl]-3,3-dimethyl-pyrrolidin-2,5-dion,
1-[2-(6,8-Dichlor-2-cyclopropyl-1,2,3,4-tetrahydro-isochinolin-4-yl)-phenyl]-pyrrolidin-2,5-dion,
1-[4-((S)-6,8-Dichlor-2-methyl-1,2,3,4-tetrahydro-isochinolin-4-yl)-phenyl]-piperidin-2,6-dion,
1-[4-((S)-6,8-Dichlor-2-methyl-1,2,3,4-tetrahydro-isochinolin-4-yl)-phenyl]-pyrrolidin-2,5-dion,
1-[4-(6,8-Dichlor-2-methyl-1,2,3,4-tetrahydro-isochinolin-4-yl)-phenyl]-pyrrolidin-2,5-dion,
1-[2-((R)-6,8-Dichlor-2-methyl-1,2,3,4-tetrahydro-isochinolin-4-yl)-phenyl]-piperidin-2,6-dion,
1-[2-(6,8-Dichlor-2-methyl-1,2,3,4-tetrahydro-isochinolin-4-yl)-phenyl]-piperidin-2,6-dion,
1-[3-((S)-6,8-Dichlor-2-methyl-1,2,3,4-tetrahydro-isochinolin-4-yl)-phenyl]-pyrrol-2,5-dion,
1-[2-(6,8-Dichlor-2-methyl-1,2,3,4-tetrahydro-isochinolin-4-yl)-phenyl]-pyrrolidin-2,5-dion,
1-[2-((R)-6,8-Dichlor-2-methyl-1,2,3,4-tetrahydro-isochinolin-4-yl)-phenyl]-pyrrolidin-2,5-dion,
3-[2-((R)-6,8-Dichlor-2-methyl-1,2,3,4-tetrahydro-isochinolin-4-yl)-phenyl]-imidazolidin-2,4-dion,
3-[2-(6,8-Dichlor-2-methyl-1,2,3,4-tetrahydro-isochinolin-4-yl)-phenyl]-imidazolidin-2,4-dion,
3-[3-((S)-6,8-Dichlor-2-methyl-1,2,3,4-tetrahydro-isochinolin-4-yl)-phenyl]-imidazolidin-2,4-dion,
3-[4-((S)-6,8-Dichlor-2-methyl-1,2,3,4-tetrahydro-isochinolin-4-yl)-phenyl]-imidazolidin-2,4-dion,
3-[4-(6,8-Dichlor-2-methyl-1,2,3,4-tetrahydro-isochinolin-4-yl)-phenyl]-imidazolidin-2,4-dion
und
3-[2-((R)-6,8-Dichlor-2-cyclopropyl-1,2,3,4-tetrahydro-isochinolin-4-yl)-phenyl]-oxazolidin-2,4-dion,
sowie deren pharmazeutisch verträgliche Salze und Trifluoracetate.

6. Verbindungen der Formel I nach Anspruch 1, worin bedeuten
R1, R2, R3 und R4 unabhängig voneinander Wasserstoff, F, Cl, Br, CN oder R11-(CₘH₂ₘ)-Aₙ-;
m Null oder 1;
n Null oder 1;
R11 Wasserstoff, Methyl oder CₚF₂ₚ₊₁;
A Sauerstoff, NCH₃ oder S(O)q;
p 1 oder 2;
q Null, 1 oder 2;
R5 Wasserstoff, Methyl, Ethyl oder Cyclopropyl;
R6 Wasserstoff oder Methyl;
R7 und R8 unabhängig voneinander Wasserstoff, F, Cl, CN, CO₂R12, NR13R14 oder R16-(CₘₘH₂ₘₘ)-Bₙₙ-;
R12 Wasserstoff, Methyl oder Ethyl;
R13, R14 unabhängig voneinander Wasserstoff, Alkyl mit 1, 2, 3 oder 4 C-Atomen oder Cycloalkyl mit 3, 4, 5 oder 6 C-Atomen;
oder
R13 und R14 bilden mit dem Stickstoffatom, an das sie gebunden sind, einen 5, 6 oder 7 gliedrigen Ring, in dem eine CH₂-Gruppe durch NR15, S oder Sauerstoff ersetzt sein kann;
R15 Wasserstoff, Alkyl mit 1, 2, 3 oder 4 C-Atomen oder Cycloalkyl mit 3, 4, 5 oder 6 C-Atomen;
mm Null, 1 oder 2;
nn Null oder 1;
R16 Wasserstoff, Methyl oder CₚₚF₂ₚₚ₊₁;
B Sauerstoff oder S(O)_{qq};
pp 1 oder 2;
qq Null, 1 oder 2;
W CᵣH₂ᵣ oder CₛH₂ₛ₋₂;
wobei eine oder mehrere CH₂-Gruppen in CᵣH₂ᵣ und CₛH₂ₛ₋₂ durch NR17, Sauerstoff oder S ersetzt sein können;
R17 Wasserstoff, Alkyl mit 1, 2, 3 oder 4 C-Atomen oder Cycloalkyl mit 3, 4, 5 oder 6 C-Atomen;
r 1, 2, 3, 4, 5, 6, 7 oder 8;
s 2, 3, 4, 5, 6, 7 oder 8;
X -C(O)- oder -S(O)₂-;
Z eine Bindung;
sowie deren pharmazeutisch verträglichen Salze und Trifluoracetate.

7. Verbindungen der Formel I nach Anspruch 1 und/oder 6, worin bedeuten
R1 und R3 Wasserstoff
R2 und R4 unabhängig voneinander Wasserstoff, F, Cl, Br, NH₂, NHCH₃ oder N(CH₃)₂;
R5 Wasserstoff, Methyl, Ethyl oder Cyclopropyl;
R6 Wasserstoff oder Methyl;
R7 und R8 Wasserstoff;
W CᵣH₂ᵣ oder CₛH₂ₛ₋₂;
wobei eine oder mehrere CH₂-Gruppen in CᵣH₂ᵣ und CₛH₂ₛ₋₂ durch NR17, Sauerstoff oder S ersetzt sein können;
R17 Wasserstoff oder Methyl
r 1, 2, 3, 4, 5 oder 6;
s 2, 3, 4, 5 oder 6;
X -C(O)- oder -S(O)₂-;
Z eine Bindung;
sowie deren pharmazeutisch verträglichen Salze und Trifluoracetate.

8. Verbindungen der Formel I mach einem oder mehreren der Ansprüche 1, 6 und 7 ausgewählt aus der Gruppe:
1-[2-(8-Brom-6-chlor-2-methyl-1,2,3,4-tetrahydro-isochinolin-4-yl)-phenyl]-pyrrolidin-2-on,
1-[2-((R)-6,8-Dichlor-2-methyl-1,2,3,4-tetrahydro-isochinolin-4-yl)-phenyl]-3-methyl-1,3-dihydro-imidazol-2-on,
(R)-6,8-Dichlor-4-[2-(1,1-dioxo-1-λ6-isothiazolidin-2-yl)-phenyl]-2-methyl-1,2,3,4-tetrahydro-isochinolin,
3-[2-((R)-6,8-Dichlor-2-methyl-1,2,3,4-tetrahydro-isochinolin-4-yl)-phenyl]-oxazolidin-2-on,
1-[2-((R)-6,8-Dichlor-2-methyl-1,2,3,4-tetrahydro-isochinolin-4-yl)-phenyl]-1,3-dihydroimidazol-2-on,
1-[2-((R)-6,8-Dichlor-2-methyl-1,2,3,4-tetrahydro-isochinolin-4-yl)-phenyl]-imidazolidin-2-on,
1-[2-((R)-6,8-Dichlor-2-methyl-1,2,3,4-tetrahydro-isochinolin-4-yl)-phenyl]-pyrrolidin-2-on,
1-[2-((R)-6,8-Dichlor-2-methyl-1,2,3,4-tetrahydro-isochinolin-4-yl)-phenyl]-pyrrolidin-2-on,
1-[3-((S)-6,8-Dichlor-2-methyl-1,2,3,4-tetrahydro-isochinolin-4-yl)-phenyl]-piperidin-2-on,
1-[2-((R)-6,8-Dichlor-2-cyclopropyl-1,2,3,4-tetrahydro-isochinolin-4-yl)-phenyl]-pyrrolidin-2-on,
3-[2-((R)-6,8-Dichlor-2-cyclopropyl-1,2,3,4-tetrahydro-isochinolin-4-yl)-phenyl]-oxazolidin-2-on
und
6,8-Dichlor-4-[4-(1,1-dioxo-1-λ⁶-[1,2,5]thiadiazolidin-2-yl)-phenyl]-2-methyl-1,2,3,4-tetrahydro-isochinoline,
sowie deren pharmazeutisch verträglichen Salze und Trifluoracetate.

9. Verbindungen der Formel I nach einem oder mehreren der Ansprüche 1, 6, 7 und 8 ausgewählt aus der Gruppe:
1-[2-(8-Brom-6-chlor-2-methyl-1,2,3,4-tetrahydro-isochinolin-4-yl)-phenyl]-pyrrolidin-2-on,
1-[2-((R)-6,8-Dichlor-2-methyl-1,2,3,4-tetrahydro-isochinolin-4-yl)-phenyl]-3-methyl-1,3-dihydro-imidazol-2-on,
(R)-6,8-Dichlor-4-[2-(1,1-dioxo-1-λ6-isothiazolidin-2-yl)-phenyl]-2-methyl-1,2,3,4-tetrahydro-isochinolin,
3-[2-((R)-6,8-Dichlor-2-methyl-1,2,3,4-tetrahydro-isochinolin-4-yl)-phenyl]-oxazolidin-2-on,
1-[2-((R)-6,8-Dichlor-2-methyl-1,2,3,4-tetrahydro-isochinolin-4-yl)-phenyl]-1,3-dihydroimidazol-2-on,
1-[2-((R)-6,8-Dichlor-2-methyl-1,2,3,4-tetrahydro-isochinolin-4-yl)-phenyl]-imidazolidin-2-on,
1-[2-((R)-6,8-Dichlor-2-methyl-1,2,3,4-tetrahydro-isochinolin-4-yl)-phenyl]-pyrrolidin-2-on
und
1-[2-((R)-6,8-Dichlor-2-methyl-1,2,3,4-tetrahydro-isochinolin-4-yl)-phenyl]-pyrrolidin-2-on,
sowie deren pharmazeutisch verträglichen Salze und Trifluoracetate.

10. Verbindungen der Formel I and deren pharmazeutisch verträgliche Salze nach einem oder mehreren der Ansprüche 1 bis 9 zur Verwendung als Medikament.

11. Verbindungen der Formel I and deren pharmazeutisch verträgliche Salze nach einem oder mehreren der Ansprüche 1 bis 9 zur Herstellung eines Medikaments zur Behandlung oder Prophylaxe von Störungen des Atemantriebs, von Atemstörungen, Schlaf-bedingten Atemstörungen, Schlafapnoen, von Schnarchen, von akuten und chronischen Nierenerkrankungen, des akuten Nierenversagens und des chronischen Nierenversagens, von Störungen der Darmfunktion, des Bluthochdrucks, der essentiellen Hypertonie, von Erkrankungen des Zentralnervensystems, von Erkrankungen, die durch ZNS-Übererregbarkeit resultieren, Epilepsie und zentral ausgelöste Krämpfe oder von Angstzuständen, Depressionen und Psychosen, von ischämischen Zuständen des peripheren oder zentralen Nervensystems oder des Schlaganfalls, von akuten und chronischen Schäden und Erkrankungen peripherer Organe oder Gliedmaßen, die durch Ischämie- oder durch Reperfusionsereignisse verursacht werden, von Atherosklerose, von Störungen des Fettstoffwechseis, von Thrombosen, von Störungen der Gallenfunktion, von Befall durch Ektoparasiten, von Erkrankungen infolge endothelialer Dysfunktion, von Protozoen-Erkrankungen, der Malaria, zur Konservierung und Lagerung von Transplantaten für chirurgische Maßnahmen, für den Einsatz bei chirurgischen Operationen und Organtransplantationen, zum Einsatz bei Bypassoperationen, zum Einsatz bei Wiederbelebung nach Herzstillstand, oder zur Behandlung von Schockzuständen oder der Zuckerkrankheit und diabetischer Spätschäden oder von Krankheiten, bei denen die Zellproliferation eine primäre oder sekundäre Ursache darstellt und zur Gesunderhaltung und Lebensverlängerung.

12. Verwendung einer Verbindung der Formel I and deren pharmazeutisch verträgliche Salze nach einem oder mehreren der Ansprüche der Ansprüche 1 bis 9 in Kombination mit anderen Arzneimitteln oder Wirkstoffen zur Herstellung eines Medikaments zur Behandlung oder Prophylaxe von Störungen des Atemantriebs, von Atemstörungen, Schlaf-bedingten Atemstörungen, Schlafapnoen, von Schnarchen, von akuten und chronischen Nierenerkrankungen, des akuten Nierenversagens und des chronischen Nierenversagens, von Störungen der Darmfunktion, des Bluthochdrucks, der essentiellen Hypertonie, von Erkrankungen des Zentralnervensystems, von Erkrankungen, die durch ZNS-Übererregbarkeit resultieren, Epilepsie und zentral ausgelöste Krämpfe oder von Angstzuständen, Depressionen und Psychosen, von ischämischen Zuständen des peripheren oder zentralen Nervensystems oder des Schlaganfalls, von akuten und chronischen Schäden und Erkrankungen peripherer Organe oder Gliedmaßen, die durch Ischämie- oder durch Reperfusionsereignisse verursacht werden, von Atherosklerose, von Störungen des Fettstoffwechsels, von Thrombosen, von Störungen der Gallenfunktion, von Befall durch Ektoparasiten, von Erkrankungen infolge endothelialer Dysfunktion, von Protozoen-Erkrankungen, der Malaria, zur Konservierung und Lagerung von Transplantaten für chirurgische Maßnahmen, für den Einsatz bei chirurgischen Operationen und Organtransplantationen, zum Einsatz bei Bypassoperationen, zum Einsatz bei Wiederbelebung nach Herzstillstand, oder zur Behandlung von Schockzuständen oder der Zuckerkrankheit und diabetischer Spätschäden oder von Krankheiten, bei denen die Zellproliferation eine primäre oder sekundäre Ursache darstellt und zur Gesunderhaltung und Lebensverlängerung.

13. Verwendung einer Verbindung der Formel I und/oder deren pharmazeutisch verträglicher Salze nach einem oder mehreren der Ansprüche 1 bis 9 allein oder in Kombination mit anderen Arzneimitteln oder Wirkstoffen zur Herstellung eines Medikaments zur Behandlung oder Prophylaxe von Störungen des Atemantriebs und/oder von Schlaf-bedingten Atemstörungen wie Schlafapnoen.

14. Verwendung einer Verbindung der Formel I und/oder deren pharmazeutisch verträglicher Salze nach einem oder mehreren der Ansprüche 1 bis 9 allein oder in Kombination mit anderen Arzneimitteln oder Wirkstoffen zur Herstellung eines Medikaments zur Behandlung oder Prophylaxe des Schnarchens.

15. Verwendung einer Verbindung der Formel I und/oder deren pharmazeutisch verträglicher Salze nach einem oder mehreren der Ansprüche 1 bis 9 allein oder in Kombination mit anderen Arzneimitteln oder Wirkstoffen zur Herstellung eines Medikaments zur Behandlung oder zur Prophylaxe von akuten oder chronischen Nierenerkrankungen, des akuten Nierenversagens oder des chronischen Nierenversagens.

16. Verwendung einer Verbindung der Formel I und/oder deren pharmazeutisch verträglicher Salze nach einem oder mehreren der Ansprüche 1 bis 9 allein oder in Kombination mit anderen Arzneimitteln oder Wirkstoffen zur Herstellung eines Medikaments zur Behandlung oder Prophylaxe von Störungen der Darmfunktion.

17. Pharmazeutische Zubereitung für die humane, veterinäre oder phytoprotektive Anwendung enthaltend eine wirksame Menge einer Verbindung der Formel I und/oder eines pharmazeutisch verträglichen Salzes davon nach einem oder mehreren der Ansprüche 1 bis 9.

18. Pharmazeutische Zubereitung für die humane, veterinäre oder phytoprotektive Anwendung enthaltend eine wirksame Menge einer Verbindung der Formel und/oder eines pharmazeutisch verträglichen Salzes davon nach einem oder mehreren der Ansprüche 1 bis 9, in Kombination mit anderen pharmakologischen Wirkstoffen oder Arzneimitteln.

## Claims

1. A compound of the formula I 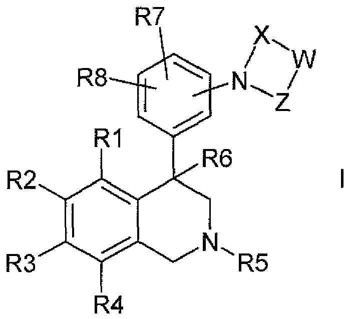 in which:
R1, R2, R3 and R4 are each independently hydrogen, F, Cl, Br, I, CN, NO₂ or R11-(CₘH₂ₘ)-Aₙ-:
m is zero, 1, 2, 3 or 4;
n is zero or 1;
R11 is hydrogen, methyl or CₚF₂ₚ₊₁;
A is oxygen, NH, N(CH₃) or S(O)_{q};
p is 1, 2 or 3;
q is zero, 1 or 2;
R5 is hydrogen, alkyl having 1, 2, 3, 4, 5 or 6 carbon atoms or cycloalkyl having 3, 4, 5 or 6 carbon atoms;
R6 is hydrogen, OH, F, CF₃, alkyl having 1, 2, 3 or 4 carbon atoms or cycloalkyl having 3, 4, 5 or 6 carbon atoms;
R7 and R8 are each independently hydrogen, F, Cl, Br, CN, CO₂R12, NR13R14 or R16-(CₘₘH₂ₘₘ)-Bₙₙ-;
R12 is hydrogen, alkyl having 1, 2, 3 or 4 carbon atoms or cycloalkyl having 3, 4, 5 or 6 carbon atoms;
R13 and R14 are each independently hydrogen, alkyl having 1, 2, 3 or 4 carbon atoms or cycloalkyl having 3, 4, 5 or 6 carbon atoms;
or
R13 and R14, with the nitrogen atom to which they are bonded, form a 4-, 5-, 6- or 7-membered ring in which one CH₂ group may be replaced by NR15, S or oxygen;
R15 is hydrogen, alkyl having 1, 2, 3 or 4 carbon atoms or cycloalkyl having 3, 4, 5 or 6 carbon atoms;
mm is zero, 1, 2, 3 or 4;
nn is zero or 1;
R16 is hydrogen, methyl or CₚₚF₂ₚₚ₊₁;
B is oxygen or S(O)_{qq};
pp is 1, 2 or 3;
qq is zero, 1 or 2;
W is CᵣH₂ᵣ or CₛH₂ₛ₋₂;
where one or more CH₂ groups in CᵣH₂ᵣ and CₛH₂ₛ₋₂ may be replaced by NR17, oxygen or S;
R17 is hydrogen, alkyl having 1, 2, 3 or 4 carbon atoms or cycloalkyl having 3, 4, 5 or 6 carbon atoms;
r is 1, 2, 3, 4, 5, 6, 7 or 8;
s is 2, 3, 4, 5, 6, 7 or 8;
X is -C(O)- or -S(O)₂-;
Z is -C(O)- or a bond;
and also its pharmaceutically acceptable salts and trifluoroacetates.

2. A compound of the formula I as claimed in claim 1 in which
R1, R2, R3 and R4 are each independently hydrogen, F, Cl, Br, CN or R11-(CₘH₂ₘ)-Aₙ-;
m is zero or 1;
n is zero or 1;
R11 is hydrogen, methyl or CₚF₂ₚ₊₁;
A is oxygen, NCH₃ or S(O)_{q};
p is 1 or 2;
q is zero, 1 or 2;
R5 is hydrogen, methyl, ethyl or cyclopropyl;
R6 is hydrogen or methyl;
R7 and R8 are each independently hydrogen, F, Cl, CN, CO₂R12, NR13R14 or R16-(CₘₘH₂ₘₘ)-Bₙₙ-;
R12 is hydrogen, methyl or ethyl;
R13 and R14 are each independently hydrogen, alkyl having 1, 2, 3 or 4 carbon atoms or cycloalkyl having 3, 4, 5 or 6 carbon atoms;
or
R13 and R14, with the nitrogen atom to which they are bonded, form a 5-, 6- or 7-membered ring in which one CH₂ group may be replaced by NR15, S or oxygen;
R15 is hydrogen, alkyl having 1, 2, 3 or 4 carbon atoms or cycloalkyl having 3, 4, 5 or 6 carbon atoms;
mm is zero, 1 or 2;
nn is zero or 1;
R16 is hydrogen, methyl or CₚₚF₂ₚₚ₊₁;
B is oxygen or S(O)_{qq};
pp is 1 or 2;
qq is zero, 1 or 2;
W is CᵣH₂ᵣ or CₛH₂ₛ₋₂;where one or more CH₂ groups in CᵣH₂ᵣ and CₛH₂ₛ₋₂ may be replaced by NR17, oxygen or S;
R17 is hydrogen, alkyl having 1, 2, 3 or 4 carbon atoms or cycloalkyl having 3, 4, 5 or 6 carbon atoms;
r is 2, 3, 4, 5, 6, 7 or 8;
s is 2, 3, 4, 5, 6, 7 or 8;
X is -C(O)- or -S(O)₂-;
Z is -C(O)-;
and also its pharmaceutically acceptable salts and trifluoroacetates.

3. A compound of the formula I as claimed in claim 1 and/or 2, in which
R1 and R3 are each hydrogen;
R2 and R4 are each independently hydrogen, F, Cl, Br, NH₂, NHCH₃ or N(CH₃)₂;
R5 is hydrogen, methyl, ethyl or cyclopropyl;
R6 is hydrogen or methyl;
R7 and R8 are each hydrogen;
W is CᵣH₂ᵣ or C_{S}H₂ₛ₋₂; where one or more CH₂ groups in CᵣH₂ᵣ and CₛH₂ₛ₋₂ may be replaced by NR17, oxygen or S;
R17 is hydrogen or methyl;
r is 2, 3, 4, 5 or 6;
s is 2, 3, 4, 5 or 6;
X is -C(O)- or -S(O)₂-;
Z is -C(O)-;
and also its pharmaceutically acceptable salts and
trifluoroacetates.

4. A compound of the formula I as claimed in one or more of claims 1 to 3, selected from the group of:
1-[2-(8-bromo-6-chloro-2-methyl-1,2,3,4-tetrahydroisoquinolin-4-yl)phenyl]pyrrolidine-2,5-dione,
3-[2-((R)-6,8-dichloro-2-cyclopropyl-1,2,3,4-tetrahydroisoquinolin-4-yl)phenyl]-1-methylimidazolidine-2,4-dione,
3-[2-((R)-6,8-dichloro-2-methyl-1,2,3,4-tetrahydroisoquinolin-4-yl)phenyl]-1-methyl-imidazolidine-2,4-dione
3-[2-((R)-6,8-dichloro-2-cyclopropyl-1,2,3,4-tetrahydroisoquinolin-4-yl)phenyl]imidazolidine-2,4-dione
(S)-1-[2-((R)-6,8-dichloro-2-cyclopropyl-1,2,3,4-tetrahydroisochinolin-4-yl)phenyl]-3-hydroxypyrrolidine-2,5-dione,
(R)-1-[2-((R)-6,8-dichloro-2-cyclopropyl-1,2,3,4-tetrahydroisoquinolin-4-yl)phenyl]-3-hydroxypyrrolidine-2,5-dione,
(R)-1-[2-((R)-6,8-dichloro-2-methyl-1,2,3,4-tetrahydrosoquinolin-4-yl)phenyl]-3-hydroxypyrrolidine-2,5-dione,
(S)-1-[2-((R)-6,8-dichloro-2-methyl-1,2,3, 4-tetrahydroisoquinolin-4-yl)phenyl]-3-hydroxypyrrolidine-2,5-dione,
4-[2-((R)-6,8-dichloro-2-methyl-1,2,3,4-tetrahydroisoquinolin-4-yl)phenyl]morpholine-3,5-dione,
3-[2-((R)-6,8-dichloro-2-methyl-1,2,3,4-tetrahydroisoquinolin-4-yl)phenyl]oxazolidine-2,4-dione,
3-[2-((R)-6,8-dichloro-2-methyl-1,2,3,4-tetrahydroisoquinolin-4-yl)phenyl]thiazolidine-2,4-dione,
1-[2-((R)-6,8-dichloro-2-cyclopropyl-1,2,3,4-tetrahydroisoquinolin-4-yl)phenyl]pyrrolidine-2,5-dione,
(S)-1-[2-((R)-6,8-dichloro-2-methyl-1,2,3,4-tetrahydroisoquinolin-4-yl)phenyl]-3-methylpiperidine-2,6-dione,
(R)-1-[2-((R)-6,8-dichloro-2-methyl-1,2,3,4-tetrahydroisoquinolin-4-yl)phenyl]-3-methylpiperidine-2,6-dione,
1-[2-((R)-6,8-dichloro-2-methyl-1,2,3,4-tetrahydroisoquinolin-4-yl)phenyl]-3,5-dimethyl-piperidine-2,5-dione,
1-[2-((R)-6,8-dichloro-2-methyl-1,2,3,4-tetrahydroisoquinolin-4-yl)phenyl]-3-methylpyrrolidine-2,5-dione,
1-[2-((R)-6,8-dichloro-2-methyl-1,2,3, 4-tetrahydroisoquinolin-4-yl)phenyl]-3,3-dimethyl-pyrrolidine-2,5-dione,
1-[2-(6,8-dichloro-2-cyclopropyl-1,2,3, 4-tetrahydroisoquinolin-4-yl)phenyl]pyrrolidine-2,5-dione,
1-[4-((S)-6,8-dichloro-2-methyl-1,2,3,4-tetrahydroisoquinolin-4-yl)phenyl]piperidine-2,6-dione,
1-[4-((S)-6,8-dichloro-2-methyl-1,2,3,4-tetrahydroisaquinolin-4-yl)phenyl]pyrrolidine-2,5-dione,
1-[4-(6,8-dichloro-2-methyl-1,2,3,4-tetrahydroisoquinolin-4-yl)phenyl]pyrrolidine-2,5-dione,
1-[2-((R)-6,8-dichloro-2-methyl-1,2,3,4-tetrahydroisoquinolin-4-yl)phenyl]piperidine-2,6-dione,
1-[2-(6,8-dichloro-2-methyl-1,2,3,4-tetrahydroisoquinolin-4-yl)phenyl]piperidine-2,6-dione,
1-[3-((S)-6,8-dichloro-2-methyl-1,2,3,4-tetrahydroisoquinolin-4-yl)phenyl]pyrrole-2,5-dione,
1-[2-(6,8-dichloro-2-methyl-1,2,3,4-tetrahydroisoquinolin-4-yl)phenyl]pyrrolidine-2,5-dione,
1-[2-((R)-6,8-dichloro-2-methyl-1,2,3,4-tetrahydroisoquinolin-4-yl)phenyl]pyrrolidine-2,5-dione,
3-[2-((R)-6,8-dichloro-2-methyl-1,2,3,4-tetrahydroisoquinolin-4-yl)phenyl]imidazolidine-2,4-dione,
3-[2-(6,8-dichloro-2-methyl-1,2,3,4-tetrahydroisoquinolin-4-yl)phenyl]imidazolidine-2,4-dione,
3-[3-((S)-6,8-dichloro-2-methyl-1,2,3,4-tetrahydroisoquinolin-4-yl)phenyl]imidazolidine-2,4-dione,
3-[4-((S)-6,8-dichloro-2-methyl-1,2,3,4-tetrahydroisoquinolin-4-yl)phenyl]imidazolidine-2,4-dione,
3-[4-(6,8-dichloro-2-methyl-1,2,3,4-tetrahydroisoquinolin-4-yl)phenyl]imidazolidine-2,4-dione,
3-[2-((R)-6,8-dichloro-2-cyclopropyl-1,2,3,4-tetrahydroisoquinolin-4-yl)phenyl]-oxazolidine-2,4-dione,
3-[2-((R)-6,8-dichloro-2-methyl-1,2,3,4-tetrahydroisoquinolin-4-yl)phenyl]-5-methyl-imidazolidine-2,4-dione,
(3R,4S)-1-[2-((R)-6,8-dichloro-2-methyl-1,2,3,4-tetrahydroisoquinolin-4-yl)phenyl]-3,4-dimethylpyrrolidine-2,5-dione,
1-[4-((S)-6,8-dichloro-2-methyl-1,2,3,4-tetrahydroisoquinolin-4-yl)phenyl]-pyrrole-2,5-dione,
1-[3-((S)-6,8-dichloro-2-methyl-1,2,3,4-tetrahydroisoquinolin-4-yl)phenyl]piperidine-2,6-dione,
1-[3-((S)-6,8-dichloro-2-methyl-1,2,3,4-tetrahydroisoquinolin-4-yl)phenyl]pyrrolidine-2,5-dione,
1-[4-(6,8-dichloro-2-methyl-1,2,3,4-tetrahydroisoquinolin-4-yl)phenyl]piperidine-2,6-dione,
3-[3-(6,8-dichloro-2-methyl-1,2,3,4-tetrahydroisoquinolin-4-yl)phenyl]imidazolidine-2,4-dione,
1-[2-((R)-2-methyl-1,2,3,4-tetrahydroisoquinolin-4-yl)phenyl]pyrrolidine-2,5-dione,
3-[2-((R)-6,8-dichloro-2-methyl-1,2,3,4-tetrahydroisoquinolin-4-yl)phenyl]-5-isopropyl-imidazolidine-2,4-dione,
3-[2-((R)-6,8-dichloro-2-methyl-1,2,3,4-tetrahydroisoquinolin-4-yl)phenyl]-5-isobutyl-imidazolidine-2,4-dione,
(R und S)-3-[2-((R)-6,8-dichloro-2-methyl-1,2,3,4-tetrahydroisoquinolin-4-yl)phenyl]-5-(2-methylsulfanylethyl)imidazolidine-2,4-dione,
3-[2-((R)-6,8-dichloro-2-methyl-1,2,3,4-tetrahydroisoquinolin-4-yl)phenyl]-5,5-dimethyl-imidazolidine-2,4-dione,
1-[2-((R)-6,8-dichloro-1,2,3,4-tetrahydroisoquinolin-4-yl)phenyl]pyrrolidine-2,5-dione,
3-[2-((R)-6,8-dichloro-2-cyclopropyl-1,2,3,4-tetrahydroisoquinolin-4-yl)phenyl]thiazolidine-2,4-dione,
1-[2-((S)-6,8-dichloro-2-methyl-1,2,3,4-tetrahydroisoquinolin-4-yl)phenyl]pyrrolidine-2,5-dione,
1-[2-((S)-6,8-dichloro-2-cyclopropyl-1,2,3,4-tetrahydroisoquinolin-4-yl)-phenyl]pyrrolidine-2,5-dione,
1-[2-((R)-6,8-dichloro-2-methyl-1,2,3,4-tetrahydroisoquinolin-4-yl)phenyl]-3,3,4,4-tetra-methylpyxrolidine-2,5-dione,
(S)-1-[2-((R)-6,8-dichloro-2-cyclopropyl-1,2,3,4-tetrahydroisoquinolin-4-yl)phenyl]-3-methylpiperidine-2,6-dione,
1-[4-(6,8-dichloro-2-methyl-1,2,3,4-tetrahydroisoquinolin-4-yl)phenyl]-4,4-dimethyl-piperidine-2,6-dione
and 1-[2-(6,8-dichloro-2-methyl-1,2,3,4-tetrahydroisoquinolin-4-yl)phenyl]pyrrole-2,5-dione,
and also its pharmaceutically acceptable salts and trifluoroacetates.

5. A compound of the formula I as claimed in one or more of claims 1 to 4, selected from the group of:
1-[2-(8-bromo-5-chloro-2-methyl-1,2,3,4-tetrahydroisoquinolin-4-yl)phenyl]pyrrolidine-2,5-dione,
3-[2-((R)-6,8-dichloro-2-cyclopropyl-1,2,3,4-tetrahydroisochinolin-4-yl)phenyl]-1-methylimidazolidine-2,4-dione,
3-[2-((R)-6,8-dichloro-2-methyl-1,2,3,4-tetrahydroisoquinolin-4-yl)phenyl]-1-methylimidazolidine-2,4-dione
3-[2-((R)-6,8-dichloro-2-cyclopropyl-1,2,3,4-tetrahydroisoquinolin-4-yl)phenyl]-imidazolidine-2,4-dione
(S)-1-[2-((R)-6,8-dichloro-2-cyclopropyl-1,2,3,4-tetrahydroisoquinolin-4-yl)phenyl]-3-hydroxypyrrolidine-2,5-dione,
(R)-1-[2-((R)-6,8-dichloro-2-cyclopropyl-1,2,3,4-tetrahydroisoquinolin-4-yl)phenyl]-3-hydroxypyrrolidine-2,5-dione,
(R)-1-[2-((R)-6,8-dichloro-2-methyl-1,2,3,4-tetrahydroisoquinolin-4-yl)phenyl]-3-hydroxypyrrolidine-2,5-dione,
(S)-1-[2-((R)-6,8-dichloro-2-methyl-1,2,3,4-tetrahydroisoquinolin-4-yl)phenyl]-3-hydroxypyrrolidin2,5-dione,
4-[2-((R)-6,8-dichloro-2-methyl-1,2,3,4-tetrahydroisoquinolin-4-yl)phenyl]morpholine-3,5-dione,
3-[2-((R)-6,8-dichloro-2-methyl-1,2,3,4-tetrahydroisoquinolin-4-yl)phenyl]oxazolidine-2,4-dione,
3-[2-((R)-6,8-dichloro-2-methyl-1,2,3,4-tetrahydroisoquinolin-4-yl)phenyl]thiazolidine-2,4-dione,
1-[2-((R)-6,8-dichloro-2-cyclopropyl1,2,3,4-tetrahydroisoquinolin-4-yl)phenyl]-pyrrolidine-2,5-dione,
(S)-1-[2-((R)-6,8-dichloro-2-methyl-1,2,3,4-tetrahydroisoquinolin-4-yl)phenyl]-3-methyl-piperidine-2,6-dione,
(R)-1-[2-((R)-6,8-dichloro-2-methyl-1,2,3,4-tetrahydroisoquinolin-4-yl)phenyl]-3-methyl-piperidine-2,6-done,
1-[2-((R)-6,8-dichloro-2-methyl-1,2,3,4-tetrahydroisoquinolin-4-yl)phenyl]-3,5-dimethylpiperidine-2,6-dione,
1-[2-((R)-6,8-dichloro-2-methyl-1,2,3,4-tetrahydroisoquinolin-4-yl)phenyl]-3-methylpyrrolidine-2,5-dione,
1-[2-((R)-6,8-dichloro-2-methyl-1,2,3,4-tetrahydroisoquinolin-4-yl)phenyl]-3,3-dimethylpyrrolidine-2,5-dione,
1-[2-(6,8-dichloro-2-cyclopropyl-1,2,3,4-tetrahydroisoquinolin-4-yl)phenyl]pyrrolidine-2,5-dione,
1-[4-((S)-6,8-dichloro-2-methyl-1,2,3,4-tetrahydroisoquinolin-4-yl)phenyl]piperidine-2,6-dione,
1-[4-((S)-6,8-dichloro-2-methyl-1,2,3,4-tetrahydroisoquinolin-4-yl)phenyl]pyrrolidine-2,5-dione,
1-[4-(6,8-dichloro-2-methyl-1,2,3,4-tetrahydroisoquinolin-4-yl)phenyl]pyrrolidine-2,5-dione,
1-[2-((R)-6,8-dichloro-2-methyl-1,2,3,4-tetrahydroisoquinolin-4-yl)phenyl]piperidine-2,6-dione,
1-[2-(6,8-dichloro-2-methyl-1,2,3,4-tetrahydroisoquinolin-4-yl)phenyl]piperidine-2,6-dione,
1-[3-((S)-6,8-dichloro-2-methyl-1,2,3,4-tetrahydroisoquinolin-4-yl)phenyl]pyrrole-2,5-dione,
1-[2-(6,8-dichloro-2-methyl-1,2,3,4-tetrahydroisoquinolin-4-yl)phenyl]pyrrolidine-2,5-dione,
1-[2-((R)-5,8-dichloro-2-methyl-1,2,3,4-tetrahydroisoquinolin-4-yl)phenyl]pyrrolidine-2,5-dione,
3-[2-((R)-6,8-dichloro-2-methyl-1,2,3,4-tetrahydroisoquinolin-4-yl)phenyl]imidazolidine-2,4-dione,
3-[2-(6,8-dichloro-2-methyl-1,2,3,4-tetrahydroisoquinolin-4-yl)phenyl]imidazolidine-2,4-dione,
3-[3-((S)-6,8-dichloro-2-methyl-1,2,3,4-tetrahydroisoquinolin-4-yl)phenyl]imidazalidine-2,4-dione,
3-[4-((S)-6,8-dichloro-2-methyl-1,2,3,4-tetrahydroisoquinolin-4-yl)phenyl]imidazolidine-2,4-dione,
3-[4-(6,8-dichloro-2-methyl-1,2,3,4-tetrahydroisoquinolin-4-yl)phenyl]imidazolidine-2,4-dione and
3-[2-((R)-6,8-dichloro-2-cyclopropyl-1,2,3,4-tetrahydroisoquinolin-4-yl)phenyl]-oxazolidine-2,4-dione,
and also its pharmaceutically acceptable salts and trifluoroacetates.

6. A compound of the formula I as claimed in claim 1, in which
R1, R2, R3 and R4 are each independently F, Cl, Br, CN or R11-(CₘH₂ₘ)-Aₙ-;
m is zero or 1;
n is zero or 1;
R11 is hydrogen, methyl or CₚF₂ₚ₊₁;
A is oxygen, NCH₃ or S(O)_{q};
p is 1 or 2;
q is zero, 1 or 2;
R5 is hydrogen, methyl, ethyl or cyclopropyl;
R6 is hydrogen or methyl;
R7 and R8 are each independently hydrogen, F, Cl, CN, CO₂R12, NR13R14 or R16-(CₘₘH₂ₘₘ)-Bₙₙ-;
R12 is hydrogen, methyl or ethyl;
R13, R14 are each independently hydrogen, alkyl having 1, 2, 3 or 4 carbon atoms or cycloalkyl having 3, 4, 5 or 6 carbon atoms; or
R13 and R14, with the nitrogen atom to which they are bonded, form a 5-, 6- or 7-membered ring in which one CH₂ group may be replaced by NR15, S or oxygen;
R15 is hydrogen, alkyl having 1, 2, 3 or 4 carbon atoms or cycloalkyl having 3, 4, 5 or 6 carbon atoms;
mm is zero, 1 or 2;
nn is zero or 1;
R16 is hydrogen, methyl or CₚₚF₂ₚₚ₊₁;
B is oxygen or S(O)_{qq};
pp is 1 or 2;
qq is zero, 1 or 2;
W is CᵣH₂ᵣ or CₛH₂ₛ₋₂;where one or more CH₂ groups in CᵣH₂ᵣ and CₛH₂ₛ₋₂ may be replaced by NR17, oxygen or S;
R17 is hydrogen, alkyl having 1, 2, 3 or 4 carbon atoms or cycloalkyl having 3, 4, 5 or 6 carbon atoms;
r is 1, 2, 3, 4, 5, 6, 7 or 8;
s is 2, 3, 4, 5, 6, 7 or 8;
X is -C(O)- or -S(O)₂-;
Z is a bond;
and also its pharmaceutically acceptable salts and trifluoroacetates.

7. A compound of the formula I as claimed in claim 1 and/or 6, in which
R1 and R3 are each hydrogen;
R2 and R4 are each independently hydrogen, F, Cl, Br, NH₂, NHCH₃ or N(CH₃)₂;
R5 is hydrogen, methyl, ethyl or cyclopropyl;
R6 is hydrogen or methyl;
R7 and R8 are each hydrogen;
W is CᵣH₂ᵣ or CₛH₂ₛ₋₂;where one or more CH₂ groups in CᵣH₂ᵣ and CₛH₂ₛ₋₂ may be replaced by NR17, oxygen or S;
R17 is hydrogen or methyl;
r is 1, 2, 3, 4, 5 or 6;
s is 2, 3, 4, or 6;
X is -C(O)- or -S(O)₂-;
Z is a bond;
and also its pharmaceutically acceptable salts and trifluoroacetates.

8. A compound of the formula I as claimed in one or more of claims 1, 6 and 7, selected from the group of:
1-[2-(8-bromo-6-chloro-2-methyl-1,2,3,4-tetrahydroisoquinolin-4-yl)phenyl]pyrrolidin-2-one,
1-[2-((R)-6,8-dichloro-2-methyl-1,2,3,4-tetrahydroisoquinolin-4-yl)phenyl]-3-methyl-l,3-dihydroimidazol-2-one,
(R)-6,8-dichloro-4-[2-(1,1-dioxo-1-λ6-isothiazolidin-2-yl)phenyl]-2-methyl-1,2,3,4-tetrahydroisoquinoline,
3-[2-((R)-6,8-dichloro-2-methyl-1,2,3,4-tetrahydroisoquinolin-4-yl)phenyl]oxazolidin-2-one,
1-[2-((R)-6,8-dichloro-2-methyl-1,2,3,4-tetrahydroisoquinolin-4-yl)phenyl]-1,3-dihydroimidazol-2-one,
1-[2-((R)-6,8-dichloro-2-methyl-1,2,3,4-tetrahydroisoquinolin-4-yl)phenyl]imidazolidin-2-one,
1-[2-((R)-6,8-dichloro-2-methyl-1,2,3,4-tetrahydroisoquinolin-4-yl)phenyl]pyrrolidin-2-one, 1-[2-((R)-6,8-dichloro-2-methyl-1,2,3,4-tetrahydroisoquinolin-4-yl)phenyl]pyrrolidin-2-one,
1-[3-((S)-6,8-dichloro-2-methyl-1,2,3,4-tetrahydroisoquinolin-4-yl)phenyl]piperidin-2-one,
1-[2-((R)-6,8-dichloro-2-cyclopropyl-1,2,3,4-tetrahydroisoquinolin-4-yl)phenyl]-pyrrolidin-2-one,
3-[2-((R)-6,8-dichloro-2-cyclopropyl-1,2,3,4-tetrahydroisoquinolin-4-yl)phenyl]oxazolidin-2-one
and
6,8-dichloro-4-[4-(1,1-dioxo-1-λ⁶-[1,2,5]thiadiazolidin-2-yl)phenyl]-2-methyl-1,2,3,4-tetrahydroisoquinolines,
and also its pharmaceutically acceptable salts and trifluoroacetates.

9. A compound of the formula I as claimed in one or more of claims 1, 6, 7 and 8, selected from the group of:
1-[2-(8-bromo-6-chloro-2-methyl-1,2,3,4-tetrahydroisoquinolin-4-yl)phenyl]pyrrolidin-2-one,
1-[2-((R)-6,8-dichloro-2-methyl-1,2,3,4-tetrahydroisoquinolin-4-yl)phenyl]-3-methyl-1,3-dihydroimidazol-2-one,
(R)-6,8-dichloro-4-[2-(1,1-dioxo-1-λ⁶-isothiazolidin-2-yl)phenyl]-2-methyl-1,2,3,4-tetrahydroisoquinoline,
3-[2-((R)-6,8-dichloro-2-methyl-1,2,3,4-tetrahydroisoquinolin-4-yl)phenyl]oxazolidin-2-one,
1-[2-((R)-6,8-dichloro-2-methyl-1,2,3,4-tetrahydroisoquinolin-4-yl)phenyl]-1,3-dihydro-imidazol-2-one,
1-[2-((R)-6,8-dichloro-2-methyl-1,2,3,4-tetrahydroisochinolin-4-yl)phenyl]imidazolidin-2-one,
1-[2-((R)-6,8-dichloro-2-methyl-1,2,3,4-tetrahydroisoquinolin-4-yl)phenyl]pyrrolidin-2-one and
1-[2-((R)-6,8-dichloro-2-methyl-1,2,3,4-tetrahydroisoquinolin-4-yl)phenyl]pyrrolidin-2-one,
and also its pharmaceutically acceptable salts and trifluoroacetates.

10. A compound of the formula I and its pharmaceutically acceptable salts as claimed in one or more of claims 1 to 9 for use as a medicament.

11. A compound of the formula I and its pharmaceutically acceptable salts as claimed in one or more of claims 1 to 9 for preparing a medicament for the treatment or prophylaxis of disorders of respiratory drive, of respiratory disorders, sleep-related respiratory disorders, sleep apneas, of snoring, of acute and chronic renal disorders, of acute kidney failure and of chronic kidney failure, of disorders of intestinal function, of high blood pressure, of essential hypertension, of disorders of the central nervous system, of disorders resulting from CNS overexcitability, epilepsy and centrally induced convulsions or of states of anxiety, depressions and psychoses, of ischemic states of the peripheral or central nervous system or of stroke, of acute and chronic damage to and disorders of peripheral organs or limbs caused by ischemic events or by reperfusion events, of atherosclerosis, of disorders of lipid metabolism, of thromboses, of disorders of biliary function, of infestation by ectoparasites, of disorders caused by endothelial dysfunction, of protozoal disorders, of malaria, for the preservation and storage of transplants for surgical procedures, for use in surgical operations and organ transplants, for use in bypass operations, in resuscitation after cardiac arrest, or for the treatment of states of shock or of diabetes and late damage from diabetes, or of diseases in which cellular proliferation constitutes a primary or secondary cause, and for maintaining health and prolonging life.

12. The use of a compound of the formula I and its pharmaceutically acceptable salts as claimed in one or more of claims 1 to 9 in combination with other medicaments or active ingredients for preparing a medicament for the treatment or prophylaxis of disorders of respiratory drive, of respiratory disorders, sleep-related respiratory disorders, sleep apneas, of snoring, of acute and chronic renal disorders, of acute kidney failure and of chronic kidney failure, of disorders of intestinal function, of high blood pressure, of essential hypertension, of disorders of the central nervous system, of disorders resulting from CNS overexcitability, epilepsy and centrally induced convulsions or of states of anxiety, depressions and psychoses, of ischemic states of the peripheral or central nervous system or of stroke, of acute and chronic damage to and disorders of peripheral organs or limbs caused by ischemic events or by reperfusion events, of atherosclerosis, of disorders of lipid metabolism, of thromboses, of disorders of biliary function, of infestation by ectoparasites, of disorders caused by endothelial dysfunction, of protozoal disorders, of malaria, for the preservation and storage of transplants for surgical procedures, for use in surgical operations and organ transplants, for use in bypass operations, in resuscitation after cardiac arrest, or for the treatment of states of shock or of diabetes and late damage from diabetes, or of diseases in which cellular proliferation constitutes a primary or secondary cause, and for maintaining health and prolonging life.

13. The use of a compound of the formula I and/or its pharmaceutically acceptable salts as claimed in one or more of claims 1 to 9 alone or in combination with other medicaments or active ingredients for preparing a medicament for the treatment or prophylaxis of disorders of respiratory drive and/or of sleep-related respiratory disorders such as sleep apneas.

14. The use of a compound of the formula I and/or its pharmaceutically acceptable salts as claimed in one or more of claims 1 to 9 alone or in combination with other medicaments or active ingredients for preparing a medicament for the treatment or prophylaxis of snoring.

15. The use of a compound of the formula I and/or its pharmaceutically acceptable salts as claimed in one or more of claims 1 to 9 alone or in combination with other medicaments or active ingredients for preparing a medicament for the treatment or prophylaxis of acute or chronic renal disorders, of acute kidney failure or of chronic kidney failure.

16. The use of a compound of the formula I and/or its pharmaceutically acceptable salts as claimed in one or more of claims 1 to 9 alone or in combination with other medicaments or active ingredients for preparing a medicament for the treatment or prophylaxis of disorders of intestinal function.

17. A pharmaceutical preparation for human, veterinary or phytoprotective use, comprising an effective amount of a compound of the formula I and/or of a pharmaceutically acceptable salt thereof as claimed in one or more of claims 1 to 9.

18. A pharmaceutical preparation for human, veterinary or phytoprotective use, comprising an effective amount of a compound of the formula I and/or of a pharmaceutically acceptable salt thereof as claimed in one or more of claims 1 to 9, in combination with other pharmacological active ingredients or medicaments.

## Revendications

1. Composés de formule I 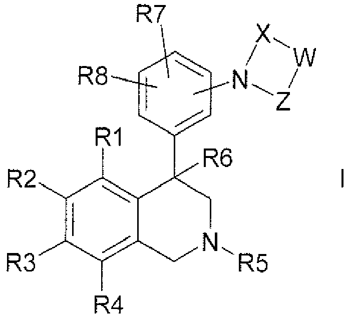 dans laquelle :
R1, R2, R3 et R4 représentent indépendamment les uns des autres hydrogène, F, Cl, Br, I, CN, NO₂ ou R11-(CₘH₂ₘ)-Aₙ- ;
m représente zéro, 1, 2, 3 ou 4 ;
n représente zéro ou 1 ;
R11 représente hydrogène, 1 méthyle ou CₚF₂ₚ₊₁ ;
A représente oxygène, NH, N(CH₃) ou S(O)_{q} ;
p représente 1, 2 ou 3 ;
q représente zéro, 1 ou 2 ;
R5 représente l'hydrogène, un allyle ayant 1, 2, 3, 4, 5 ou 6 atomes de carbone ou un cycloalkyle ayant 3, 4, 5 ou 6 atomes de carbone ;
R6 représente l'hydrogène, OH, F, CF₃, un alkyle ayant 1, 2, 3 ou 4 atomes de carbone ou un cycloalkyle ayant 3, 4, 5 ou 6 atomes de carbone ;
R7 et R8 représentent indépendamment l'un de l'autre hydrogène, F, Cl, Br, CN, CO₂R12, NR13R14 ou R16- (CₘₘH₂ₘₘ) -Bₙₙ- ;
R12 représente l'hydrogène, un alkyle ayant 1, 2, 3 ou 4 atomes de carbone ou un cycloalkyle ayant 3, 4, 5 ou 6 atomes de carbone ;
R13 et R14 représentent indépendamment l'un de l'autre l'hydrogène, un alkyle ayant 1, 2, 3 ou 4 atomes de carbone ou un cycloalkyle ayant 3, 4, 5 ou 6 atomes de carbone ; ou
R13 et R14 forment avec l'atome d'azote auquel ils sont liés un cycle à 4, 5, 6 ou 7 éléments dans lequel un groupement CH₂ peut être remplacé par NR15, S ou oxygène ;
R15 représente l'hydrogène, un alkyle ayant 1, 2, 3 ou 4 atomes de carbone ou un cycloalkyle ayant 3, 4, 5 ou 6 atomes de carbone ;
mm représente zéro, 1, 2, 3 ou 4 ;
nn représente zéro ou 1 ;
R16 représente hydrogène, méthyle ou CₚₚF₂ₚₚ₊₁ ;
B représente oxygène ou S(O)_{qq} ;
pp représente 1, 2 ou 3 ;
qq représente zéro, 1 ou 2 ;
W représente CᵣH₂ᵣ ou CₛH₂ₛ₋₂ ;un ou plusieurs groupements CH₂ dans CᵣH₂, et CₛH₂ₛ₋₂ pouvant être remplacés par NR17, oxygène ou S ;
R17 représente l'hydrogène, un alkyle ayant 1, 2, 3 ou 4 atomes de carbone ou un cycloalkyle ayant 3, 4, 5 ou 6 atomes de carbone;
r représente 1, 2, 3, 4, 5, 6, 7 ou 8 ;
s représente 2, 3, 4, 5, 6, 7 ou 8 ;
X représente -C(O)- ou -S(O)₂- ;
Z représente -C(O)- ou une liaison ;
ainsi que leurs sels et trifluoroacétates pharmaceutiquement acceptables.

2. Composés de formule I selon la revendication 1, dans laquelle
R1, R2, R3 et R4 représentent indépendamment les uns des autres hydrogène, F, Cl, Br, CN ou R11-(CₘH₂ₘ)-Aₙ- ;
m représente zéro ou 1 ;
n représente zéro ou 1 ;
R11 représente hydrogène, méthyle ou CₚF₂ₚ₊₁ ;
A représente oxygène, NCH₃ ou S(O)_{q} ;
p représente 1 ou 2 ;
q représente zéro, 1 ou 2 ;
R5 représente hydrogène, méthyle, éthyle ou cyclopropyle ;
R6 représente hydrogène ou méthyle ;
R7 et R8 représentent indépendamment l'un de l'autre hydrogène, F, Cl, CN, CO₂R12, NR13R14 ou R16- (CₘₘH₂ₘₘ) - Bₙₙ- ;
R12 représente hydrogène, méthyle ou éthyle ;
R13 et R14 représentent indépendamment l'un de l'autre l'hydrogène, un alkyle ayant 1, 2, 3 ou 4 atomes de carbone ou un cycloalkyle ayant 3, 4, 5 ou 6 atomes de carbone ; ou
R13 et R14 forment avec l'atome d'azote auquel ils sont liés un cycle à 5, 6 ou 7 éléments dans lequel un groupement CH₂ peut être remplacé par NR15, S ou oxygène ;
R15 représente l'hydrogène, un alkyle ayant 1, 2, 3 ou 4 atomes de carbone ou un cycloalkyle ayant 3, 4, 5 ou 6 atomes de carbone ;
mm représente zéro, 1 ou 2 ;
nn représente zéro ou 1 ;
R16 représente hydrogène, méthyle ou CₚₚF₂ₚₚ₊₁ ;
B représente oxygène ou S(O)_{qq} ;
pp représente 1 ou 2 ;
qq représente zéro, 1 ou 2 ;
W représente CᵣH₂ᵣ ou CₛH₂ₛ₋₂ ; un ou plusieurs groupements CH₂ dans CᵣH₂ᵣ et CₛH₂ₛ₋₂ pouvant être remplacés par NR17, oxygène ou S ;
R17 représente l'hydrogène, un alkyle ayant 1, 2, 3 ou 4 atomes de carbone ou un cycloalkyle ayant 3, 4, 5 ou 6 atomes de carbone ;
r représente 2, 3, 4, 5, 6, 7 ou 8 ;
s représente 2, 3, 4, 5, 6, 7 ou 8 ;
X représente -C(O)- ou -S(O)₂- ;
Z représente -C(O)- ;
ainsi que leurs sels et trifluoroacétates pharmaceutiquement acceptables.

3. Composés de formule I selon la revendication 1 et/ou 2, dans laquelle
R1 et R3 représentent chacun l'hydrogène ;
R2 et R4 représentent indépendamment l'un de l'autre hydrogène, F, Cl, Br, NH₂, NHCH₃ ou N(CH₃)₂ ;
R5 représente hydrogène, méthyle, éthyle ou cyclopropyle ;
R6 représente hydrogène ou méthyle ;
R7 et R8 représentent chacun l'hydrogène ;
W représente CᵣH₂ᵣ ou CₛH₂ₛ₋₂ ;
un ou plusieurs groupements CH₂ dans CᵣH₂ᵣ et CₛH₂ₛ₋₂ pouvant être remplacés par NR17, oxygène ou S ;
R17 représente hydrogène ou méthyle ;
r représente 2, 3, 4, 5 ou 6 ;
s représente 2, 3, 4, 5 ou 6 ;
X représente -C(O)- ou -S(O)₂- ;
Z représente -C(O) - ;
ainsi que leurs sels et trifluoroacétates pharmaceutiquement acceptables.

4. Composés de formule I selon une ou plusieurs des revendications 1 à 3, choisis dans le groupe comprenant :
1-[2-(8-bromo-6-chloro-2-méthyl-1,2,3,4-tétrahydroisoquinoléin-4-yl)phényl]pyrrolidine-2,5-dione,
3-[2-((R)-6,8-dichloro-2-cyclopropyl-1,2,3,4-tétrahydroisoquinoléin-4-yl)phényl]-1-méthylimidazolidine-2,4-dione,
3-[2-((R)-6,8-dichloro-2-méthyl-1,2,3,4-tétrahydroisoquinoléin-4-yl)phényl]-1-méthylimidazolidine-2,4-dione
3-[2-((R)-6,8-dichloro-2-cyclopropyl-1,2,3,4-tétrahydroisoquinoléin-4-yl)phényl]imidazolidine-2,4-dione
(S)-1-[2-((R)-6,8-dichloro-2-cyclopropyl-1,2,3,4-tétrahydroisoquinoléin-4-yl)phényl] -3-hydroxypyrrolidine-2,5-dione,
(R)-1-[2-((R)-6,8-dichloro-2-cyclopropyl-1,2,3,4-tétrahydroisoquinoléin-4-yl)phényl] -3-hydroxypyrrolidine-2,5-dione,
(R)-1-[2-((R)-6,8-dichloro-2-méthyl-1,2,3,4-tétrahydroisoquinoléin-4-yl)phényl]-3-hydroxypyrrolidine-2,5-dione,
(S)-1-[2-((R)-6,8-dichloro-2-méthyl-1,2,3,4-tétrahydroisoquinoléin-4-yl)phényl]-3-hydroxypyrrolidine-2,5-dione,
4-[2-((R)-6,8-dichloro-2-méthyl-1,2,3,4-tétrahydroisoquinoléin-4-yl)phényl]morpholine-3,5-dione,
3-[2-((R)-6,8-dichloro-2-méthyl-1,2,3,4-tétrahydroisoquinoléin-4-yl)phényl]oxazolidine-2,4-dione,
3-[2-((R)-6,8-dichloro-2-méthyl-1,2,3,4-tétrahydroisoquinoléin-4-yl)phényl]thiazolidine-2,4-dione,
1-[2-((R)-6,8-dichloro-2-cyclopropyl-1,2,3,4-tétrahydroisoquinoléin-4-yl)phényl]pyrrolidine-2,5-dione,
(S)-1-[2-((R)-6,8-dichloro-2-méthyl-1,2,3,4-tétrahydroisoquinoléin-4-yl)phényl]-3-méthylpipéridine-2,6-dione,
(R)-1-[2-((R)-6,8-dichloro-2-méthyl-1,2,3,4-tétrahydroisoquinoléin-4-yl)phényl] -3-méthylpipéridine-2,6-dione,
1-[2-((R)-6,8-dichloro-2-méthyl-1,2,3,4-tétrahydroisoquinoléin-4-yl)phényl]-3,5-diméthyl-pipéridine-2,6-dione,
1-[2-((R)-6,8-dichloro-2-méthyl-1,2,3,4-tétrahydroisoquinoléin-4-yl)phényl]-3-méthylpyrrolidine-2,5-dione,
1-[2-((R)-6,8-dichloro-2-méthyl-1,2,3,4-tétrahydroisoquinoléin-4-yl)phényl]-3,3-diméthyl-pyrrolidine-2,5-dione,
1-[2-(6,8-dichloro-2-cyclopropyl-1,2,3,4-tétrahydroisoquinoléin-4-yl)phényl]pyrrolidine-2,5-dione,
1-[4-((S)-6,8-dichloro-2-méthyl-1,2,3,4-tétrahydroisoquinoléin-4-yl)phényl]pipéridine-2,6-dione,
1-[4-((S)-6,8-dichloro-2-méthyl-1,2,3,4-tétrahydroisoquinoléin-4-yl)phényl]pyrrolidine-2,5-dione,
1-[4-(6,8-dichloro-2-méthyl-1,2,3,4-tétrahydroisoquinoléin-4-yl)phényl]pyrrolidine-2,5-dione,
1-[2-((R)-6,8-dichloro-2-méthyl-1,2,3,4-tétrahydroisoquinoléin-4-yl)phényl]pipéridine-2,6-dione,
1-[2-(6,8-dichloro-2-méthyl-1,2,3,4-tétrahydroisoquinoléin-4-yl)phényl]pipéridine-2,6-dione,
1-[3-((S)-6,8-dichloro-2-méthyl-1,2,3,4-tétrahydroisoquinoléin-4-yl)phényl]pyrrole-2,5-dione,
1-[2-(6,8-dichloro-2-méthyl-1,2,3,4-tétrahydroisoquinoléin-4-yl)phényl]pyrrolidine-2,5-dione,
1-[2-((R)-6,8-dichloro-2-méthyl-1,2,3,4-tétrahydroisoquinoléin-4-yl)phényl]pyrrolidine-2,5-dione,
3-[2-((R)-6,8-dichloro-2-méthyl-1,2,3,4-tétrahydroisoquinoléin-4-yl)phényl]imidazolidine-2,4-dione,
3-[2-(6,8-dichloro-2-méthyl-1,2,3,4-tétrahydroisoquinoléin-4-yl)phényl]imidazolidine-2,4-dione,
3-[3-((S)-6,8-dichloro-2-méthyl-1,2,3,4-tétrahydroisoquinoléin-4-yl)phényl]imidazolidine-2,4-dione,
3-[4-((S)-6,8-dichloro-2-méthyl-1,2,3,4-tétrahydroisoquinoléin-4-yl)phényl]imidazolidine-2,4-dione,
3-[4-(6,8-dichloro-2-méthyl-1,2,3,4-tétrahydroisoquinoléin-4-yl)phényl]imidazolidine-2,4-dione,
3-[2-((R)-6,8-dichloro-2-cyclopropyl-1,2,3,4-tétrahydroisoquinoléin-4-yl)phényl]-oxazolidine-2,4-dione,
3-[2-((R)-6,8-dichloro-2-méthyl-1,2,3,4-tétrahydroisoquinoléin-4-yl)phényl]-5-méthylimidazolidine-2,4-dione,
(3R,4S)-1-[2-((R)-6,8-dichloro-2-méthyl-1,2,3,4-tétrahydroisoquinoléin-4-yl)phényl]-3,4-diméthylpyrrolidine-2,5-dione,
1-[4-((S)-6,8-dichloro-2-méthyl-1,2,3,4-tétrahydroisoquinoléin-4-yl)phényl]pyrrole-2,5-dione,
1-[3-((S)-6,8-dichloro-2-méthyl-1,2,3,4-tétrahydroisoquinoléin-4-yl)phényl]pipéridine-2,6-dione,
1-[3-((S)-6,8-dichloro-2-méthyl-1,2,3,4-tétrahydroisoquinoléin-4-yl)phényl]pyrrolidine-2,5-dione,
1-[4-(6,8-dichloro-2-méthyl-1,2,3,4-tétrahydroisoquinoléin-4-yl)phényl]pipéridine-2,6-dione,
3-[3-(6,8-dichloro-2-méthyl-1,2,3,4-tétrahydroisoquinoléin-4-yl)phényl]imidazolidine-2,4-dione,
1-[2-((R)-2-méthyl-1,2,3,4-tétrahydroisoquinoléin-4-yl)phényl]pyrrolidine-2,5-dione,
3-[2-((R)-6,8-dichloro-2-méthyl-1,2,3,4-tétrahydroisoquinoléin-4-yl)phényl]-5-isopropyl-imidazolidine-2,4-dione,
3-[2-((R)-6,8-dichloro-2-méthyl-2,2,3,4-tétrahydroisoquinoléin-4-yl)phényl]-5-isobutyl-imidazolidine-2,4-dione,
(R et S)-3-[2-((R)-6,8-dichloro-2-méthyl-1,2,3,4-tétrahydroisoquinoléin-4-yl)phényl]-5-(2-méthylsulfanyléthyl)imidazolidine-2,4-dione,
3-[2-((R)-6,8-dichloro-2-méthyl-1,2,3,4-tétrahydroisoquinoléin-4-yl)phényl]-5,5-diméthyl-imidazolidine-2,4-dione,
1-[2-((R)-6,8-dichloro-1,2,3,4-tétrahydroisoquinoléin-4-yl)phényl]pyrrolidine-2,5-dione,
3-[2-((R)-6,8-dichloro-2-cyclopropyl-1,2,3,4-tétrahydroisoquinoléin-4-yl)phényl]thiazolidine-2,4-dione,
1-[2-((S)-6,8-dichloro-2-méthyl-1,2,3,4-tétrahydroisoquinoléin-4-yl)phényl]pyrrolidine-2,5-dione,
1-[2-((S)-6,8-dichloro-2-cyclopropyl-1,2,3,4-tétrahydroisoquinoléin-4-yl)phényl]pyrrolidine-2,5-dione,
1-[2-((R)-6,8-dichloro-2-méthyl-1,2,3,4-tétrahydroisoquinoléin-4-yl)phényl]-3,3,4,4-tétra-méthylpyrrolidine-2,5-dione,
(S)-1-[2-((R)-6,8-dichloro-2-cyclopropyl-1,2,3,4-tétrahydroisoquinoléin-4-yl)phényl]-3-méthylpipéridine-2,6-dione,
1-[4-(6,8-dichloro-2-méthyl-1,2,3,4-tétrahydroisoquinoléin-4-yl)phényl]-4,4-diméthyl-pipéridine-2,6-dione
et
1-[2-(6,8-dichloro-2-méthyl-1,2,3,4-tétrahydroisoquinoléin-4-yl)phényl]pyrrole-2,5-dione,
ainsi que leurs sels et trifluoroacétates pharmaceutiquement acceptables.

5. Composés de formule I selon une ou plusieurs des revendications 1 à 4, choisis dans le groupe comprenant :
1-[2-(8-bromo-6-chloro-2-méthyl-1,2,3,4-tétrahydroisoquinoléin-4-yl)phényl]pyrrolidine-2,5-dione,
3-[2-((R)-6,8-dichloro-2-cyclopropyl-1,2,3,4-tétrahydroisoquinoléin-4-yl)phényl]-1-méthyl-imidazolidine-2,4-dione,
3-[2-((R)-6,8-dichloro-2-méthyl-1,2,3,4-tétrahydroisoquinoléin-4-yl)phényl]-1-méthyl-imidazolidine-2,4-dione
3-[2-((R)-6,8-dichloro-2-cyolopropyl-1,2,3,4-tétrahydroisoquinoléin-4-yl)phényl]-imidazolidine-2,4-dione
(S)-1-[2-((R)-6,8-dichloro-2-cyclopropyl-1,2,3,4-tétrahydroisoquinoléin-4-yl)phényl]-3-hydroxypyrrolidine-2,5-dione,
(R)-1-[2-((R)-6,8-dichloro-2-cyclopropyl-1,2,3,4-tétrahydroisoquinoléin-4-yl)phényl]-3-hydroxypyrrolidine-2,5-dione,
(R)-1-[2-((R)-6,8-dichloro-2-méthyl-1,2,3,4-tétrahydroisoquinoléin-4-yl)phényl]-3-hydroxypyrrolidine-2,5-dione,
(S)-1-[2-((R)-6,8-dichloro-2-méthyl-1,2,3,4-tétrahydroisoquinoléin-4-yl)phényl]-3-hydroxypyrrolidine-2,5-dione,
4-[2-((R)-6,8-dichloro-2-méthyl-1,2,3,4-tétrahydroisoquinoléin-4-yl)phényl]morpholine-3,5-dione,
3-[2-((R)-6,8-dichloro-2-méthyl-1,2,3,4-tétrahydroisoquinoléin-4-yl)phényl]oxazolidine-2,4-dione,
3-[2-((R)-6,8-dichloro-2-méthyl-1,2,3,4-tétrahydroisoquinoléin-4-yl)phényl]thiazolidine-2,4-dione,
1-[2-((R)-6,8-dichloro-2-cyclopropyl-1,2,3,4-tétrahydroisoquinoléin-4-yl)phenyl]-pyrrolidine-2,5-dione,
(S)-1-[2-((R)-5,8-dichloro-2-méthyl-1,2,3,4-tétrahydroisoquinoléin-4-yl)phényl]-3-méthylpipéridine-2,6-dione,
(R)-1-[2-((R)-6,8-dichloro-2-méthyl-1,2,3,4-tétrahydroisoquinoléin-4-yl)phényl]-3-méthylpipéridine-2,6-dione,
1-[2-((R)-6,8-dichloro-2-méthyl-1,2,3,4-tétrahydroisoquinoléin-4-yl)phényl]-3,5-diméthylpipéridine-2,6-dione,
1-[2-((R)-6,8-dichloro-2-méthyl-1,2,3,4-tétrahydroisoquinoléin-4-yl)phényl]-3-méthyl-pyrrolidine-2,5-dione,
1-[2-((R)-6,8-dichloro-2-méthyl-1,2,3,4-tétrahydroisoquinoléin-4-yl)phényl]-3,3-diméthylpyrrolidine-2,5-dione,
1-[2-(6,8-dichloro-2-cyclopropyl-1,2,3,4-tétrahydroisoquinoléin-4-yl)phényl]pyrrolidine-2,5-dione,
1-[4-((S)-6,8-dichloro-2-méthyl-1,2,3,4-tétrahydroisoquinoléin-4-yl)phényl]pipéridine-2,6-dione,
1-[4-((S)-6,8-dichloro-2-méthyl-1,2,3,4-tétrahydroisoquinoléin-4-yl)phényl]pyrrolidine-2,5-dione,
1-[4-(6,8-dichloro-2-méthyl-1,2,3,4-tétrahydroisoquinoléin-4-yl)phényl]pyrrolidine-2,5-dione,
1-[2-((R)-6,8-dichloro-2-méthyl-1,2,3,4-tétrahydroisoquinoléin-4-yl)phényl]pipéridine-2,6-dione,
1-[2-(6,8-dichloro-2-méthyl-1,2,3,4-tétrahydroisoquinoléin-4-yl)phényl]pipéridine-2,6-dione,
1-[3-((S)-6,8-dichloro-2-méthyl-1,2,3,4-tétrahydroisoquinoléin-4-yl)phényl]pyrrole-2,5-dione,
1-[2-(6,8-dichloro-2-méthyl-1,2,3,4-tétrahydroisoquinoléin-4-yl)phényl]pyrrolidine-2,5-dione,
1-[2-((R)-6,8-dichloro-2-méthyl-1,2,3,4-tétrahydroisoquinoléin-4-yl)phényl]pyrrolidine-2,5-dione,
3-[2-((R)-6,8-dichloro-2-méthyl-1,2,3,4-tétrahydroisoquinoléin-4-yl)phényl]imidazolidine-2,4-dione,
3-[2-(6,8-dichloro-2-méthyl-1,2,3,4-tétrahydroisoquinoléin-4-yl)phényl]imidazolidine-2,4-dione,
3-[3-((S)-6,8-dichloro-2-méthyl-1,2,3,4-tétrahydroisoquinoléin-4-yl)phényl]imidazolidine-2,4-dione,
3-[4-((S)-6,8-dichloro-2-méthyl-1,2,3,4-tétrahydroisoquinoléin-4-yl)phényl]imidazolidine-2,4-dione,
3-[4-(6,8-dichloro-2-méthyl-1,2,3,4-tétrahydroisoquinoléin-4-yl)phényl]imidazolidine-2,4-dione et
3-[2-((R)-6,8-dichloro-2-cyclopropyl-1,2,3,4-tétrahydroisoquinoléin-4-yl)phényl]-oxazolidine-2,4-dione,
ainsi que leurs sels et trifluoroacétates pharmaceutiquement acceptables.

6. Composés de formule I selon la revendication 1, dans laquelle
R1, R2, R3 et R4 représentent indépendamment les uns des autres hydrogène, F, Cl, Br, CN ou R11-(CₘH₂ₘ)-Aₙ- ;
m représente zéro ou 1 ;
n représente zéro ou 1 ;
R11 représente hydrogène, méthyle ou CₚF₂ₚ₊₁ ;
A représente oxygène, NCH₃ ou S(O)_{q} ;
p représente 1 ou 2 ;
q représente zéro, 1 ou 2 ;
R5 représente hydrogène, méthyle, éthyle ou cyclopropyle ;
R6 représente hydrogène ou méthyle ;
R7 et R8 représentent indépendamment l'un de l'autre hydrogène, F, Cl, CN, CO₂R12, NR13R14 ou R16- (CₘₘH₂ₘₘ) -Bₙₙ - ;
R12 représente hydrogène, méthyle ou éthyle ;
R13, R14 représentent indépendamment l'un de l'autre l'hydrogène, un alkyle ayant 1, 2, 3 ou 4 atomes de carbone ou un cycloalkyle ayant 3, 4, 5 ou 6 atomes de carbone ; ou
R13 et R14 forment avec l'atome d'azote auquel ils sont liés un cycle à 5, 6 ou 7 éléments dans lequel un groupement CH₂ peut être remplacé par NR15, S ou oxygène ;
R15 représente l'hydrogène, un alkyle ayant 1, 2, 3 ou 4 atomes de carbone ou un cycloalkyle ayant 3, 4, 5 ou 6 atomes de carbone ;
mm représente zéro, 1 ou 2 ;
nn représente zéro ou 1 ;
R16 représente hydrogène, méthyle ou CₚₚF₂ₚₚ₊₁;
B représente oxygène ou S(O)_{qq} ;
pp représente 1 ou 2 ;
qq représente zéro, 1 ou 2 ;
W représente CᵣH₂ᵣ ou CₛH₂ₛ₋₂ ; un ou plusieurs groupements CH₂ dans CᵣH₂ᵣ et CₛH₂ₛ₋₂ pouvant être remplacés par NR17 , oxygène ou S ;
R17 représente l'hydrogène, un alkyle ayant 1, 2, 3 ou 4 atomes de carbone ou un cycloalkyle ayant 3, 4, 5 ou 6 atomes de carbone ;
r représente 1, 2, 3, 4, 5, 6, 7 ou 8 ;
s représente 2, 3, 4, 5, 6, 7 ou 8 ;
X représente -C(O)- ou -S(O)₂- ;
Z représente une liaison ;
ainsi que leurs sels et trifluoroacétates pharmaceutiquement acceptables.

7. Composés de formule I selon la revendication 1 et/ou 6, dans laquelle
R1 et R3 représentent chacun l'hydrogène ;
R2 et R4 représentent indépendamment l'un de l'autre hydrogène, F, Cl, Br, NH₂, NHCH₃ ou N(CH₃)₂ ;
R5 représente hydrogène, méthyle, éthyle ou cyclopropyle ;
R6 représente hydrogène ou méthyle ;
R7 et R8 représentent chacun l'hydrogène ;
W représente CᵣH₂ᵣ ou CₛH₂ₛ₋₂ ;
un ou plusieurs groupements CH₂ dans CᵣH₂ᵣ et CₛH₂ₛ₋₂ pouvant être remplacés par NR17, oxygène ou S ;
R17 représente hydrogène ou méthyle ;
r représente 1, 2, 3, 4, 5 ou 6 ;
s représente 2, 3, 4, 5 ou 6 ;
X représente -C(O)- ou -S(O)₂- ;
Z représente une liaison ;
ainsi que leurs sels et trifluoroacétates pharmaceutiquement acceptables.

8. Composés de formule I selon une ou plusieurs des revendications 1, 6 et 7, choisis dans le groupe comprenant :
1-[2-(8-bromo-6-chloro-2-méthyl-1,2,3,4-tétrahydroisoquinoléin-4-yl)phényl]pyrrolidin-2-one,
1-[2-((R)-6,8-dichloro-2-méthyl-1,2,3,4-tétrahydroisoquinoléin-4-yl)phényl]-3-méthyl-1,3-dihydroimidazol-2-one,
(R)-6,8-dichloro-4-[2-(1,1-dioxo-1-λ⁶-isothiazolidin-2-yl)phényl]-2-méthyl-1,2,3,4-tétrahydroisoquinoléine,
3-[2-((R)-8,8-dichloro-2-méthyl-1,2,3,4-tétrahydroisoquinoléin-4-yl)phényl]oxazolidin-2-one,
1-[2-((R)-6,8-dichloro-2-méthyl-1,2,3,4-tétrahydroisoquinoléin-4-yl)phényl]-1,3-dihydro-imidazol-2-one,
1-[2-((R)-6,8-dichloro-2-méthyl-1,2,3,4-tétrahydroisoquinoléin-4-yl)phényl]imidazolidin-2-one,
1-[2-((R)-6,8-dichloro-2-méthyl-1,2,3,4-tétrahydroisoquinoléin-4-yl)phényl]pyrrolidin-2-one,
1-[2-((R)-6,8-dichloro-2-méthyl-1,2,3,4-tétrahydroisoquinoléin-4-yl)phényl]pyrrolidin-2-one,
1-[3-((S)-6,8-dichloro-2-méthyl-1,2,3,4-tétrahydroisoquinoléin-4-yl)phényl]pipéridin-2-one,
1-[2-((R)-6,8-dichloro-2-cyclopropyl-1,2,3,4-tétrahydroisoquinoléin-4-yl)phényl]-pyrrolidin-2-one,
3-[2-((R)-6,8-dichloro-2-cyclopropyl-1,2,3,4-tétrahydroisoquinoléin-4-yl)phényl]oxazolidin-2-one
et
6,8-dichloro-4-[4-(1,1-dioxo-1-λ⁶-[1,2,5]thiadiazolidin-2-yl)phényl]-2-méthyl-1,2,3,4-tétrahydroisoquinoléines,
ainsi que leurs sels et trifluoroacétates pharmaceutiquement acceptables.

9. Composés de formule I selon une ou plusieurs des revendications 1, 6, 7 et 8, choisis dans le groupe comprenant :
1-[2-(8-bromo-6-chloro-2-méthyl-1,2,3,4-tétrahydroisoquinoléin-4-yl)phényl]pyrrolidin-2-one,
1-[2-((R)-6,8-dichloro-2-méthyl-1,2,3,4-tétrahydroisoquinoléin-4-yl)phényl]-3-méthyl-1,3-dihydroimidazol-2-one,
(R)-6,8-dichloro-4-[2-(1,1-dioxo-λ⁶-isothiazolidin-2-yl)phényl]-2-méthyl-1,2,3,4-tétrahydroisoquinoléine,
3-[2-((R)-6,8-dichloro-2-méthyl-1,2,3,4-tétrahydroisoquinoléin-4-yl)phényl]oxazolidin-2-one,
1-[2-((R)-6,8-dichloro-2-méthyl-1,2,3,4-tétrahydroisoquinoléin-4-yl)phényl]-1,3-dihydro-imidazol-2-one,
1-[2-((R)-6,8-dichloro-2-méthyl-1,2,3,4-tétrahydroisoquinoléin-4-yl)phényl]imidazolidin-2-one;
1-[2-((R)-6,8-dichloro-2-méthyl-1,2,3,4-tétrahydroisoquinoléin-4-yl)phényl]pyrrolidin-2-one
et
1-[2-((R)-6,8-dichloro-2-méthyl-1,2,3,4-tétrahydroisoquinoléin-4-yl)phényl]pyrrolidin-2-one,
ainsi que leurs sels et trifluoroacétates pharmaceutiquement acceptables.

10. Composés de formule I et leurs sels pharmaceutiquement acceptables selon une ou plusieurs des revendications 1 à 9 destinés à une utilisation en tant que médicament.

11. Composés de formule I et leurs sels pharmaceutiquement acceptables selon une ou plusieurs des revendications 1 à 9 destinés à la fabrication d'un médicament pour le traitement ou la prophylaxie de maladies de la commande respiratoire, de maladies respiratoires, de maladies respiratoires liées au sommeil, d'apnées du sommeil, des ronflements, de maladies rénales aiguës et chroniques, de l'insuffisance rénale aiguë et de l'insuffisance rénale chronique, de troubles de la fonction intestinale, de la pression artérielle élevée, de l'hypertension artérielle essentielle, de maladies du système nerveux central, de maladies résultant d'une surexcitabilité du SNC, de l'épilepsie et de convulsions induites centralement ou des états d'anxiété, de dépressions et de psychoses, d'états ischémiques du système nerveux central ou périphérique ou d'accidents cérébrovasculaires, de lésions aiguës et chroniques et de maladies des organes périphériques ou des membres causées par des événements ischémiques ou par des évènements de reperfusion, de l'athérosclérose, de maladies du métabolisme lipidique, de thromboses, de troubles de la fonction biliaire, de l'infestation par des ectoparasites, de maladies causées par un dysfonctionnement endothélial, de maladies causées par des protozoaires, du paludisme, pour la conservation et le stockage de greffons pour des procédures chirurgicales, pour une utilisation lors d'opérations chirurgicales et de greffes d'organes, lors de la réalisation d'opérations de pontage, lors d'une ressuscitation après un arrêt cardiaque ou pour le traitement d'états de choc ou du diabète et des lésions tardives du diabète, ou de maladies dans lesquelles la prolifération cellulaire constitue une cause primaire ou secondaire, et pour maintenir la santé et prolonger la vie.

12. Utilisation d'un composé de formule I et de ses sels pharmaceutiquement acceptables selon une ou plusieurs des revendications 1 à 9 en combinaison avec d'autres médicaments ou ingrédients actifs pour la fabrication d'un médicament pour le traitement ou la prophylaxie de maladies de la commande respiratoire, de maladies respiratoires, de maladies respiratoires liées au sommeil, d'apnées du sommeil, des ronflements, de maladies rénales aiguës et chroniques, de l'insuffisance rénale aiguë et de l'insuffisance rénale chronique, de troubles de la fonction intestinale, de la pression artérielle élevée, de l'hypertension artérielle essentielle, de maladies du système nerveux central, de maladies résultant d'une surexcitabilité du SNC, de l'épilepsie et de convulsions induites centralement ou d'états d'anxiété, de dépressions et de psychoses, d'états ischémiques du système nerveux central ou périphérique ou d'accidents cérébrovasculaires, de lésions aiguës et chroniques et de maladies des organes périphériques ou des membres causées par des évènements ischémiques ou par des évènements de reperfusion, de l'athérosclérose, de maladies du métabolisme lipidique, de thromboses, de troubles de la fonction biliaire, de l'infestation par des ectoparasites, de maladies causées par un dysfonctionnement endothélial, de maladies causées par des protozoaires, du paludisme, pour la conservation et le stockage de greffons pour des procédures chirurgicales, pour une utilisation lors d'opérations chirurgicales et de greffes d'organes, lors de la réalisation d'opérations de pontage, lors d'une ressuscitation après un arrêt cardiaque ou pour le traitement d'états de choc ou du diabète et des lésions tardives du diabète, ou de maladies dans lesquelles la prolifération cellulaire constitue une cause primaire ou secondaire, et pour maintenir la santé et prolonger la vie.

13. Utilisation d'un composé de formule I et/ou de ses sels pharmaceutiquement acceptables selon une ou plusieurs des revendications 1 à 9 seuls ou en combinaison avec d'autres médicaments ou ingrédients actifs pour la fabrication d'un médicament pour le traitement ou la prophylaxie de maladies de la commande respiratoire et/ou de maladies respiratoires liées au sommeil telles que les apnées du sommeil.

14. Utilisation d'un composé de formule I et/ou de ses sels pharmaceutiquement acceptables selon une ou plusieurs des revendications 1 à 9 seuls ou en combinaison avec d'autres médicaments ou ingrédients actifs pour la fabrication d'un médicament pour le traitement ou la prophylaxie des ronflements.

15. Utilisation d'un composé de formule I et/ou de ses sels pharmaceutiquement acceptables selon une ou plusieurs des revendications 1 à 9 seuls ou en combinaison avec d'autres médicaments ou ingrédients actifs pour la fabrication d'un médicament pour le traitement ou la prophylaxie de maladies rénales aiguës ou chroniques, de l'insuffisance rénale aiguë ou de l'insuffisance rénale chronique.

16. Utilisation d'un composé de formule I et/ou de ses sels pharmaceutiquement acceptables selon une ou plusieurs des revendications 1 à 9 seuls ou en combinaison avec d'autres médicaments ou ingrédients actifs pour la fabrication d'un médicament pour le traitement ou la prophylaxie de troubles de la fonction intestinale.

17. Préparation pharmaceutique pour usage humain, vétérinaire ou phytoprotecteur, comprenant une quantité effective d'un composé de formule I et/ou d'un sel pharmaceutiquement acceptable de ce composé selon une ou plusieurs des revendications 1 à 9.

18. Préparation pharmaceutique pour usage humain, vétérinaire ou phytoprotecteur, comprenant une quantité effective d'un composé de formule I et/ou d'un sel pharmaceutiquement acceptable de ce composé selon une ou plusieurs des revendications 1 à 9, en combinaison avec d'autres médicaments ou ingrédients pharmacologiquement actifs.
